(19) Europäisches Patentamt
European Patent Office
Office européen des brevets

(11) EP 2 940 010 A1

(12) **EUROPEAN PATENT APPLICATION**

(43) Date of publication:
**04.11.2015 Bulletin 2015/45**

(21) Application number: **14166399.7**

(22) Date of filing: **29.04.2014**

(51) Int Cl.:
*C07D 233/58* (2006.01) *C07D 207/06* (2006.01)
*C07F 9/54* (2006.01) *C07C 211/63* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **BASF SE**
**67056 Ludwigshafen (DE)**

(72) Inventors:
- **Massonne, Klemens**
  **67098 Bad Dürkheim (DE)**
- **Geyer, Karolin**
  **67061 Ludwigshafen (DE)**
- **Schierle-Arndt, Kerstin**
  **64673 Zwingenberg (DE)**
- **Seikel, Elisabeth**
  **64347 Griesheim (DE)**
- **Seeler, Fabian**
  **69469 Weinheim (DE)**
- **Sundermeyer, Joerg**
  **35041 Marburg (DE)**
- **Braam, Axel**
  **35037 Marburg (DE)**

(54) **Ionic liquids based on oxalic acid mono amides**

(57) The present invention relates to monosubstituted oxalic acid amides of the general formula (I)

$$[A]^+ [O\text{-}C(O)\text{-}C(O)\text{-}X]^- \qquad (I)$$

wherein the meaning is for
$[A]^+$ a cation made from an organic moiety A having a formally positively charged heteroatom selected from the group consisting of nitrogen, phosphorus and sulfur and
X is the group $-NR^aR^b$, wherein $R^a$ and $R^b$ are the same or different and are independently from each other hydrogen or a $C_1$ to $C_{30}$ organic residue with the proviso that at least one of Ra and $R^b$ is a $C_1$ to $C_{30}$ organic residue.

EP 2 940 010 A1

## Description

The present invention relates to monosubstituted oxalic acid amides of the general formula (I)

$$[A]^+ [O\text{-}C(O)\text{-}C(O)\text{-}X]^- \qquad (I)$$

wherein the meaning is for

$[A]^+$ a cation made from an organic moiety A having a formally positively charged heteroatom selected from the group consisting of nitrogen, phosphorus and sulfur, and

X is the group $-NR^aR^b$, wherein $R^a$ and $R^b$ are the same or different and are independently from each other hydrogen or a $C_1$ to $C_{30}$ organic residue with the proviso that at least one of $R^a$ and $R^b$ is a $C_1$ to $C_{30}$ organic residue.

The present invention further relates to a process for the preparation of monosubstituted oxalic acid derivatives of the fomula (I) as defined above and the use of the monosubstituted oxalic acid derivatives of the general formula (I) above for at least partially dissolving an inorganic or an organic material such as metal compounds, e.g. rare earth element compounds or actinide element compounds or the use of the monosubstituted oxalic acid derivatives of the general formula (I) above (without disclaimer) in a separation process for metals such as rare earth elements or actinide elements , e.g. in a liquid-liquid extraction process for the separation of rare earth elements.

The use of imidazolium based organic liquids having anions different from oxalic acid derivatives in e.g. lanthanide speciation is described in I Billard et al., Radiochim Acta (2009), 355-359, the use of imidazolium based organic liquids having anions different from oxalic acid derivatives in extraction of e.g. lanthanides is described in I. Billard et al., Anal Bioanal Chem. (2011) 1555-1566 and in X. Sun et al.,Talanta (2012), 132-137.

None of these state of the art does disclose the use of the monosubstituted oxalic acid derivatives of the general formula (I) above for at least partially dissolving an inorganic or an organic material such as metal compounds, e.g. rare earth element compounds or actinide element compounds or the use of the monosubstituted oxalic acid derivatives of the general formula (I) above in a separation process for metals such as rare earth elements or actinide elements , e.g. in a liquid-liquid extraction process for the separation of rare earth elements.

An object of the instant invention is to provide new ionic compounds and their use in dissolution and separation processes for inorganic or organic materials.

For the purpose of the present invention the expression $C_1$ to $C_{30}$ organic residue means an organic structural moiety containing one (1) to thirty (30) carbon atoms and in addition may contain at least one non-carbon atom.

The $C_1$ to $C_{30}$ organic residue is for example selected from the group consisting of the following organic residues (I) to (X).

(I) For the purposes of the present invention, the expression "alkyl" comprises straight-chain or branched alkyl. This is preferably straight-chain or branched $C_1$-$C_{30}$-alkyl, in particular $C_1$-$C_{18}$-alkyl and very particularly preferably $C_1$-$C_{12}$-alkyl. Examples of alkyl groups are, in particular, methyl, ethyl, n-propyl, isopropyl, n-butyl, isobutyl, sec-butyl, tert-butyl, n-pentyl, isopentyl, 1-methylbutyl, tert-pentyl, neopentyl, n-hexyl, 3-hexyl, 2-methyl-1-pentyl, 3-methyl-1-pentyl, 4-methyl-1-pentyl, 2-methyl-2-pentyl, 3-methyl-2-pentyl, 4-methyl-2-pentyl, 2-methyl-3-pentyl, 3-methyl-3-pentyl, 2,2-dimethyl-1-butyl, 2,3-dimethyl-1-butyl, 3,3-dimethyl-1-butyl, 2-ethyl-1-butyl, 2,3-dimethyl-2-butyl, 3,3-dimethyl-2-butyl, n-heptyl, n-octyl, 1-methylheptyl, 2-ethylhexyl, 2,4,4-trimethyl-pentyl, 1,1,3,3-tetramethylbutyl, n-nonyl, n-decyl, n-undecyl, n-dodecyl, n-tridecyl, n-tetradecyl, n-pentadecyl, n-hexadecyl, n-heptadecyl, n-octadecyl and n-eicosyl.

The expression alkyl also comprises alkyl radicals whose carbon chain may be interrupted by one or more nonadjacent heteroatoms or heteroatom-comprising groups which are preferably selected from among -O-, -S-, $-NR^z-$, $-PR^z-$, $-SiR^zR^{zz}$ and/or $-SO_2$. $R^z$ is preferably hydrogen, alkyl, cycloalkyl, heterocycloalkyl, aryl or hetaryl. $R^{zz}$ is preferably hydrogen, alkyl, cycloalkyl, heterocycloalkyl or aryl.

Examples of alkyl radicals whose carbon chains may be interrupted by one or two nonadjacent heteroatoms -O- are the following:

methoxymethyl, diethoxymethyl, 2-methoxyethyl, 2-ethoxyethyl, 2-propoxyethyl, diethoxyethyl, 2-butoxyethyl, 2-octyloxyethyl, 2-methoxypropyl, 3-methoxypropyl, 3-ethoxypropyl, 3-propoxypropyl, 2-isopropoxyethyl, 2-butoxypropyl, 3-butoxypropyl, 4-methoxybutyl, 4-ethoxybutyl, 4-propoxybutyl, 6-methoxyhexyl, 3,6-dioxaheptyl (5-methoxy-3-oxapentyl), 3,6-dioxaoctyl (7-methoxy-4-oxaheptyl), 4,8-dioxanonyl (7-methoxy-4-oxaheptyl), 3,7-dioxaoctyl, 3,7-dioxanonyl, 4,7-dioxaoctyl, 4,7-dioxanonyl, 2- and 4-butoxybutyl, 4,8-dioxadecyl, 9-ethoxy-5-oxanonyl.

Examples of alkyl radicals whose carbon chains may be interrupted by three or more than three nonadjacent heteroatoms -O- also include oligooxyalkylenes and polyoxyalkylenes, i.e. compounds having repeating units which are

preferably selected from among $(CH_2CH_2O)_{x1}$, $(CH(CH_3)CH_2O)_{x2}$ and $((CH_2)_4O)_{x3}$, where x1, x2 and x3 are each, independently of one another, an integer from 3 to 100, preferably from 3 to 80. The sum of x1, x2 and x3 is an integer from 3 to 300, in particular from 3 to 100. In polyoxyalkylenes having two or three different repeating units, the repeating units can be present in any order, i.e. they can be randomly distributed, alternating or block-like repeating units. Examples are 3,6,9-trioxadecyl, 3,6,9-trioxaundecyl, 3,6,9-trioxadodecyl, 4,8,12-trioxatridecyl (11-methoxy-4,8-dioxaundecyl), 4,8,12-trioxatetradecyl, 14-methoxy-5,10-dioxatetradecyl, 5,10,15-trioxaheptadecyl, 3,6,9,12-tetraoxatridecyl, 3,6,9,12-tetraoxatetradecyl, 4,8,12,16-tetraoxaheptadecyl (15-methoxy-4,8,12-trioxapentadecyl), 4,8,12,16-tetraoxaoctadecyl and the like.

Examples of alkyl radicals whose carbon chains may be interrupted by one or more, e.g. 1, 2, 3, 4 or more than 4, nonadjacent heteroatoms -S- are the following:

butylthiomethyl, 2-methylthioethyl, 2-ethylthioethyl, 2-propylthioethyl, 2-butylthioethyl, 2-dodecylthioethyl, 3-methylthiopropyl, 3-ethylthiopropyl, 3-propylthiopropyl, 3-butylthiopropyl, 4-methylthiobutyl, 4-ethylthiobutyl, 4-propylthiobutyl, 3,6-dithiaheptyl, 3,6-dithiaoctyl, 4,8-dithianonyl, 3,7-dithiaoctyl, 3,7-dithianonyl, 2- and 4-butylthiobutyl, 4,8-dithiadecyl, 3,6,9-trithiadecyl, 3,6,9-trithiaundecyl, 3,6,9-trithiadodecyl, 3,6,9,12-tetrathiatridecyl and 3,6,9,12-tetrathiatetradecyl.

Examples of alkyl radicals whose carbon chains are interrupted by one or two nonadjacent heteroatom-comprising groups $-NR^z-$ are the following:

2-monomethylaminoethyl and 2-monoethylaminoethyl, 2-dimethylaminoethyl, 3-methylaminopropyl, 2- and 3-dimethylaminopropyl, 3-monoisopropylaminopropyl, 2- and 4-monopropylaminobutyl, 2- and 4-dimethylaminobutyl, 6-methylaminohexyl, 6-dimethylaminohexyl, 6-methyl-3,6-diazaheptyl, 3,6-dimethyl-3,6-diazaheptyl, 3,6-diazaoctyl and 3,6-dimethyl-3,6-diazaoctyl.

Examples of alkyl radicals whose carbon chains may be interrupted by three or more than three nonadjacent heteroatom-comprising groups - $NR^z$- also include oligoalkylenimines and polyalkylenimines. What has been said above with regard to the polyoxyalkylenes applies analogously to polyalkylenimines, with the oxygen atom being in each case replaced by an $NR^z$ group, where $R^z$ is preferably hydrogen or $C_1$-$C_4$-alkyl. Examples are 9-methyl-3,6,9-triazadecyl, 3,6,9-trimethyl-3,6,9-triazadecyl, 3,6,9-triazaundecyl, 3,6,9-trimethyl-3,6,9-triazaundecyl, 12-methyl-3,6,9,12-tetraazatridecyl, 3,6,9,12-tetramethyl-3,6,9,12-tetraazatridecyl and the like.

Examples of alkyl radicals whose carbon chains are interrupted by one or more, e.g. 1 or 2, nonadjacent $-SO_2$-groups are 2-methylsulfonylethyl, 2-ethylsulfonylethyl, 2-propylsulfonylethyl, 2-isopropylsulfonylethyl, 2-butylsulfonylethyl, 2-methylsulfonylpropyl, 3-methylsulfonylpropyl, 2-ethylsulfonylpropyl, 3-ethylsulfonylpropyl, 2-propylsulfonylpropyl, 3-propylsulfonylpropyl, 2-butylsulfonylpropyl, 3-butylsulfonylpropyl, 2-methylsulfonylbutyl, 4-methylsulfonylbutyl, 2-ethylsulfonylbutyl, 4-ethylsulfonylbutyl, 2-propylsulfonylbutyl, 4-propylsulfonylbutyl and 4-butylsulfonylbutyl.

The expression alkyl also comprises substituted alkyl radicals. Substituted alkyl groups can, depending on the length of the alkyl chain, have one or more (e.g. 1, 2, 3, 4, 5 or more than 5) substituents. These are preferably selected independently from among cycloalkyl, cycloalkyloxy, polycyclyl, polycyclyloxy, heterocycloalkyl, aryl, aryloxy, arylthio, hetaryl, halogen, hydroxy, SH, =O =S, =NRa, COOH, carboxylate, $SO_3H$, sulfonate, NE1E2, nitro and cyano, where E1 and E2 are each, independently of one another, hydrogen, alkyl, cycloalkyl, heterocycloalkyl, aryl or hetaryl. Cycloalkyl, cycloalkyloxy, polycycloalkyl, polycycloalkyloxy, heterocycloalkyl, aryl and hetaryl substituents of the alkyl groups may in turn be unsubstituted or substituted; suitable substituents are those mentioned below for these groups.

What has been said above with regard to alkyl also applies in principle to the alkyl parts of alkoxy, alkylamino, dialkylamino, alkylthio (alkylsulfanyl), alkylsulfinyl, alkylsulfonyl, etc. Suitable substituted alkyl radicals are the following:

alkyl substituted by carboxy, e.g. carboxymethyl, 2-carboxyethyl, 3-carboxypropyl, 4-carboxybutyl, 5-carboxypentyl, 6-carboxyhexyl, 7-carboxyheptyl, 8-carboxyoctyl, 9-carboxynonyl, 10-carboxydecyl, 12-carboxydodecyl and 14-carboxytetradecyl; alkyl substituted by $SO_3H$, e.g. sulfomethyl, 2-sulfoethyl, 3-sulfopropyl, 4-sulfobutyl, 5-sulfopentyl, 6-sulfohexyl, 7-sulfoheptyl, 8-sulfooctyl, 9-sulfononyl, 10-sulfodecyl, 12-sulfododecyl and 14-sulfotetradecyl;

alkyl substituted by carboxylate, for example alkoxycarbonylalkyl, e.g. methoxycarbonylmethyl, ethoxycarbonylmethyl, n-butoxycarbonylmethyl, 2-methoxycarbonylethyl, 2-ethoxycarbonylethyl, 2-methoxycarbonylpropyl, 2-ethoxycarbonylpropyl, 2-(n-butoxycarbonyl)propyl, 2-(4-n-butoxycarbonyl)propyl, 3-methoxycarbonylpropyl, 3-ethoxycarbonylpropyl, 3-(n-butoxycarbonyl)propyl, 3-(4-n-butoxycarbonyl)propyl, aminocarbonylalkyl, e.g. aminocarbonylmethyl, aminocarbonylethyl, aminocarbonylpropyl and the like; alkylaminocarbonylalkyl such as methylaminocarbonylmethyl, methylaminocarbonylethyl, ethylcarbonylmethyl, ethylcarbonylethyl and the like, or dialkylaminocarbonylalkyl such as dimethylaminocarbonylmethyl, dimethylaminocarbonylethyl, dimethylcarbonylpropyl, diethylaminocarbonylmethyl, diethylaminocarbonylethyl, diethylcarbonylpropyl and the like;

alkyl substituted by hydroxy, e.g. 2-hydroxyethyl, 2-hydroxypropyl, 3-hydroxypropyl, 3-hydroxybutyl, 4-hydroxybutyl, 2-hydroxy-2,2-dimethylethyl, 5-hydroxy-3-oxapentyl, 6-hydroxyhexyl, 7-hydroxy-4-oxaheptyl, 8-hydroxy-4-oxaoctyl, 8-hydroxy-3,6-dioxaoctyl, 9-hydroxy-5-oxanonyl, 11-hydroxy-4,8-dioxaundecyl, 11-hydroxy-3,6,9-trioxaundecyl, 14-hydroxy-5,10-dioxatetradecyl, 15-hydroxy-4,8,12-trioxapentadecyl and the like;

alkyl substituted by amino, e.g. 2-aminoethyl, 2-aminopropyl, 3-aminopropyl, 4-aminobutyl, 6-aminohexyl and the like;

alkyl substituted by cyano, e.g. 2-cyanoethyl, 3-cyanopropyl, 3-cyanobutyl and 4-cyanobutyl; alkyl substituted by halogen as defined below, where the hydrogen atoms in the alkyl group may be partly or completely replaced by halogen atoms, e.g. $C_1$-$C_{18}$-fluoroalkyl, e.g. trifluoromethyl, difluoromethyl, fluoromethyl, pentafluoroethyl, heptafluoropropyl, heptafluoroisopropyl, nonafluorobutyl, nonafluoroisobutyl, undecylfluoropentyl, undecylfluoroisopentyl and the like, $C_1$-$C_{18}$-chloroalkyl, e.g. chloromethyl, dichloromethyl, trichloromethyl, 2-chloroethyl, 2- and 3-chloropropyl, 2-, 3- and 4-chlorobutyl, 1,1-dimethyl-2-chloroethyl and the like, $C_1$-$C_{18}$-bromoalkyl, e.g. bromoethyl, 2-bromoethyl, 2- and 3-bromopropyl and 2-, 3- and 4-bromobutyl and the like;

alkyl substituted by nitro, e.g. 2-nitroethyl, 2- and 3-nitropropyl and 2-, 3- and 4-nitrobutyl and the like;

alkyl substituted by cycloalkyl, e.g. cyclopentylmethyl, 2-cyclopentylethyl, 3-cyclopentylpropyl, cyclohexylmethyl, 2-cyclohexylethyl, 3-cyclohexylpropyl and the like;

alkyl substituted by =O (oxo group), e.g. 2-oxopropyl, 2-oxobutyl, 3-oxobutyl, 1-methyl-2-oxopropyl, 2-oxopentyl, 3-oxopentyl, 1-methyl-2-oxobutyl, 1-methyl-3-oxobutyl, 2-oxohexyl, 3-oxohexyl, 4-oxohexyl, 2-oxoheptyl, 3-oxoheptyl, 4-oxoheptyl, 4-oxoheptyl and the like;

alkyl substituted by =S (thioxo group) e.g. 2-thioxopropyl, 2-thioxobutyl, 3-thioxobutyl, 1-methyl-2-thioxopropyl, 2-thioxopentyl, 3-thioxopentyl, 1-methyl-2-thioxobutyl, 1-methyl-3-thioxobutyl, 2-thioxohexyl, 3-thioxohexyl, 4-thioxohexyl, 2-thioxoheptyl, 3-thioxoheptyl, 4-thioxoheptyl, 4-thioxoheptyl and the like;

alkyl substituted by $=NR^z$, preferably a group of the type in which $R^z$ is hydrogen or $C_1$-$C_4$-alkyl, e.g. 2-iminopropyl, 2-iminobutyl, 3-iminobutyl, 1-methyl-2-iminopropyl, 2-iminopentyl, 3-iminopentyl, 1-methyl-2-iminobutyl, 1-methyl-3-imino-butyl, 2-iminohexyl, 3-iminohexyl, 4-iminohexyl, 2-iminoheptyl, 3-iminoheptyl, 4-iminoheptyl, 4-iminoheptyl, 2-methyliminopropyl, 2-methyliminobutyl, 3-methyliminobutyl, 1-methyl-2-methyliminopropyl, 2-methyliminopentyl, 3-methyliminopentyl, 1-methyl-2-methyliminobutyl, 1-methyl-3-methyliminobutyl, 2-methyliminohexyl, 3-methyliminohexyl, 4-methyliminohexyl, 2-methyliminoheptyl, 3-methyliminoheptyl, 4-methyliminoheptyl, 4-methyliminoheptyl, 2-ethyliminopropyl, 2-ethyliminobutyl, 3-ethyliminobutyl, 1-methyl-2-ethyliminopropyl, 2-ethyliminopentyl, 3-ethyliminopentyl, 1-methyl-2-ethyliminobutyl, 1-methyl-3-ethyliminobutyl, 2-ethyliminohexyl, 3-ethyliminohexyl, 4-ethyliminohexyl, 2-ethyliminoheptyl, 3-ethyliminoheptyl, 4-ethyliminoheptyl, 4-ethyliminoheptyl, 2-propyliminopropyl, 2-propyliminobutyl, 3-propyliminobutyl, 1-methyl-2-propyliminopropyl, 2-propyliminopentyl, 3-propyliminopentyl, 1-methyl-2-propyliminobutyl, 1-methyl-3-propyliminobutyl, 2-propyliminohexyl, 3-propyliminohexyl, 4-propyliminohexyl, 2-propyliminoheptyl, 3-propyliminoheptyl, 4-propyliminoheptyl, 4-propyliminoheptyl and the like.

Alkoxy is an alkyl group bound via an oxygen atom. Examples of alkoxy are: methoxy, ethoxy, n-propoxy, 1-methylethoxy, butoxy, 1-methylpropoxy, 2-methylpropoxy, 1,1-dimethylethoxy, n-pentoxy, 1-methylbutoxy, 2-methylbutoxy, 3-methylbutoxy, 1,1-dimethylpropoxy, 1,2-dimethylpropoxy, 2,2-dimethylpropoxy, 1-ethylpropoxy, hexoxy, 1-methylpentoxy, 2-methylpentoxy, 3-methylpentoxy, 4-methylpentoxy, 1,1-dimethylbutoxy, 1,2-dimethylbutoxy, 1,3-dimethylbutoxy, 2,2-dimethylbutoxy, 2,3-dimethylbutoxy, 3,3-dimethylbutoxy, 1-ethylbutoxy, 2-ethylbutoxy, 1,1,2-trimethylpropoxy, 1,2,2-trimethylpropoxy, 1-ethyl-1-methylpropoxy or 1-ethyl-2-methylpropoxy, hexoxy and also $R^AO$-$(CH_2CH_2CH_2CH_2O)_n$-$CH_2CH_2CH_2CH_2O$- where $R^A$ is hydrogen or $C_1$-$C_4$-alkyl, preferably hydrogen, methyl or ethyl, and n is from 0 to 10, preferably from 0 to 3.

Alkylthio (alkylsulfanyl) is an alkyl group bound via a sulfur atom. Examples of alkylthio are methylthio, ethylthio, propylthio, butylthio, pentylthio and hexylthio.

Alkylsulfinyl is an alkyl group bound via an S(=O) group.

Alkylsulfonyl is an alkyl group bound via an $S(=O)_2$ group.

Aryl-substituted alkyl radicals ("arylalkyl") have at least one unsubstituted or substituted aryl group as defined below. Suitable substituents on the aryl group are those mentioned below. The alkyl group in "arylalkyl" can bear at least

one further substituent as defined above and/or be interrupted by one or more nonadjacent heteroatoms or heteroatom-comprising groups selected from among -O-, -S-, -NRz- and -SO2-. Arylalkyl is preferably phenyl-C1-C10-alkyl, particularly preferably phenyl-C1-C4-alkyl, e.g. benzyl, 1-phenethyl, 2-phenethyl, 1-phenprop-1-yl, 2-phenprop-1-yl, 3-phenprop-1-yl, 1-phenbut-1-yl, 2-phenbut-1-yl, 3-phenbut-1-yl, 4-phenbut-1-yl, 1-phenbut-2-yl, 2-phenbut-2-yl, 3-phenbut-2-yl, 4-phenbut-2-yl, 1-(phenmeth)-eth-1-yl, 1-(phenmethyl)-1-(methyl)-eth-1-yl or-(phenmethyl)-1-(methyl)-prop-1-yl; preferably benzyl and 2-phenethyl.

(II) For the purposes of the present invention, the expression "alkenyl" comprises straight-chain and branched alkenyl groups which can, depending on the length oft the chain, have one or more double bonds (e.g. 1, 2, 3, 4 or more than 4). Preference is given to $C_2$-$C_{18}$-alkenyl, particularly preferably $C_2$-$C_{12}$-alkenyl groups. The expression "alkenyl" also comprises substituted alkenyl groups which may bear one or more (e.g. 1, 2, 3, 4, 5 or more than 5) substituents. Suitable substituents are, for example, selected from among =O, =S, =$NR^z$, cycloalkyl, cycloalkyloxy, polycyclyl, polycyclyloxy, heterocycloalkyl, aryl, aryloxy, arylthio, hetaryl, halogen, hydroxy, SH, COOH, carboxylate, $SO_3H$, sulfonate, alkylsulfinyl, alkylsulfonyl, $NE^3E^4$, nitro and cyano, where $E^3$ and $E^4$ are each, independently of one another, hydrogen, alkyl, cycloalkyl, heterocycloalkyl, aryl or hetaryl.

The expression "alkenyl" also comprises alkenyl radicals whose carbon chain may be interrupted by one or more nonadjacent heteroatoms or heteroatom-comprising groups which are preferably selected from among -O-, -S-, -$NR^z$- and -$SO_2$-.

Alkenyl is then, for example, ethenyl (vinyl), 1-propenyl, 2-propenyl, 1-methylethenyl, 1-butenyl, 2-butenyl, 3-butenyl, 1-pentenyl, 2-pentenyl, 3-pentenyl, 4-pentenyl, 1-hexenyl, 2-hexenyl, 3-hexenyl, 4-hexenyl, 5-hexenyl, penta-1,3-dien-1-yl, hexa-1,4-dien-1-yl, hexa-1,4-dien-3-yl, hexa-1,4-dien-6-yl, hexa-1,5-dien-1-yl, hexa-1,5-dien-3-yl, hexa-1,5-dien-4-yl, hepta-1,4-dien-1-yl, hepta-1,4-dien-3-yl, hepta-1,4-dien-6-yl, hepta-1,4-dien-7-yl, hepta-1,5-dien-1-yl, hepta-1,5-dien-3-yl, hepta-1,5-dien-4-yl, hepta-1,5-dien-7-yl, hepta-1,6-dien-1-yl, hepta-1,6-dien-3-yl, hepta-1,6-dien-4-yl, hepta-1,6-dien-5-yl, hepta-1,6-dien-2-yl, octa-1,4-dien-1-yl, octa-1,4-dien-2-yl, octa-1,4-dien-3-yl, octa-1,4-dien-6-yl, octa-1,4-dien-7-yl, octa-1,5-dien-1-yl, octa-1,5-dien-3-yl, octa-1,5-dien-4-yl, octa-1,5-dien-7-yl, octa-1,6-dien-1-yl, octa-1,6-dien-3-yl, octa-1,6-dien-4-yl, octa-1,6-dien-5-yl, octa-1,6-dien-2-yl, deca-1,4-dienyl, deca-1,5-dienyl, deca-1,6-dienyl, deca-1,7-dienyl, deca-1,8-dienyl, deca-2,5-dienyl, deca-2,6-dienyl, deca-2,7-dienyl, deca-2,8-dienyl and the like.

(III) For the purposes of the present invention, the expression "cycloalkyl" comprises both unsubstituted and substituted monocyclic saturated hydrocarbon groups which generally have from 3 to 12 ring carbons, ($C_3$-$C_{12}$-cycloalkyl groups) such as cyclopropyl, cyclobutyl, cyclopentyl, cyclohexyl, cycloheptyl, cyclooctyl, cyclononyl, cyclodecyl, cycloundecyl or cyclododecyl, in particular $C_5$-$C_{12}$-cycloalkyl. Suitable substituents are generally selected from among the substituents mentioned above for alkyl groups, alkoxy and alkylthio. Substituted cycloalkyl groups can have one or more (e.g. 1, 2, 3, 4, 5 or more than 5) substituents, and in the case of halogen the cycloalkyl radical is partially or completely substituted by halogen.

Examples of cycloalkyl groups are cyclopentyl, 2- and 3-methylcyclopentyl, 2- and 3-ethylcyclopentyl, chloropentyl, dichloropentyl, dimethylcyclopentyl, cyclohexyl, 2-, 3- and 4-methylcyclohexyl, 2-, 3- and 4-ethylcyclohexyl, 3- and 4-propylcyclohexyl, 3- and 4-isopropylcyclohexyl, 3- and 4-butylcyclohexyl, 3- and 4-sec-butylcyclohexyl, 3- and 4-tert-butylcyclohexyl, chlorohexyl, dimethylcyclohexyl, diethylcyclohexyl, methoxycyclohexyl, dimethoxycyclohexyl, diethoxycyclohexyl, butoxycyclohexyl, methylthiocyclohexyl, chlorocyclohexyl, dichlorocyclohexyl, cycloheptyl, 2-, 3- and 4-methylcycloheptyl, 2-, 3- and 4-ethylcycloheptyl, 3- and 4-propylcycloheptyl, 3- and 4-isopropylcycloheptyl, 3- and 4-butylcycloheptyl, 3- and 4-sec-butylcycloheptyl, 3- and 4-tert-butylcycloheptyl, cyclooctyl, 2-, 3-, 4- and 5-methylcyclooctyl, 2-, 3-, 4- and 5-ethylcyclooctyl, 3-, 4- and 5-propylcyclooctyl, partially fluorinated cycloalkyl and perfluorinated cycloalkyl of the formula $C_nF_{2(n-a)-(1-b)}H_{2a-b}$ where n = 5 to 12, 0 <= a <= n and b = 0 or 1.

Cycloalkyloxy is a cycloalkyl group as defined above bound via oxygen.

(IV) The expression "cycloalkenyl" comprises unsubstituted and substituted, singly or doubly unsaturated hydrocarbon groups having from 3 to 5, up to 8, up to 12, preferably from 5 to 12, ring carbons, e.g. cyclopent-1-en-1-yl, cyclopent-2-en-1-yl, cyclopent-3-en-1-yl, cyclohex-1-en-1-yl, cyclohex-2-en-1-yl, cyclohex-3-en-1-yl, cyclohexa-2,5-dien-1-yl and the like. Suitable substituents are those mentioned above for cycloalkyl.

Cycloalkenyloxy is a cycloalkenyl group as defined above bound via oxygen.

(V) For the purposes of the present invention, the expression "polycyclyl" comprises in the broadest sense compounds which comprise at least two rings, regardless of how these rings are linked. These can be carbocyclic and/or heterocyclic rings. The rings can be saturated or unsaturated. The rings can be linked via a single or double bond ("multiring compounds") joined by fusion ("fused ring systems") or bridged ("bridged ring systems", "cage compounds"). Preferred polycyclic compounds are bridged ring systems and fused ring systems. Fused ring systems can be aromatic, hydroaromatic and cyclic compounds joined by fusion (fused compounds). Fused ring systems comprise two, three or more than three rings. Depending on the way in which the rings are joined in fused ring systems, a distinction is made between ortho-fusion, i.e. each ring shares an edge or two atoms with each adjacent ring, and peri-fusion in which a carbon atom belongs to more than two rings. Among fused ring systems, preference is given to ortho-fused ring systems. For the purposes of the present invention, bridged ring systems include systems which do not belong to the multiring

ring systems nor to the fused ring systems and in which at least two ring atoms belong to at least two different rings. Among the bridged ring systems, a distinction is made according to the number of ring opening reactions which are formally required to obtain an open-chain compound between bicyclo, tricyclo, tetracyclo compounds, etc., which comprise two, three, four, etc., rings. The expression "bicycloalkyl" comprises bicyclic hydrocarbon radicals which preferably have from 5 to 10 carbon atoms, e.g. bicyclo[2.2.1]hept-1-yl, bicyclo[2.2.1]hept-2-yl, bicyclo[2.2.1]hept-7-yl, bicyclo[2.2.2]oct-1-yl, bicyclo[2.2.2]oct-2-yl, bicyclo[3.3.0]octyl, bicyclo[4.4.0]decyl and the like. The expression "bicycloalkenyl" comprises monounsaturated, bicyclic hydrocarbon radicals which preferably have from 5 to 10 carbon atoms, e.g. bicyclo[2.2.1]hept-2-en-1-yl.

(VI) For the purposes of the present invention, the expression "aryl" comprises aromatic hydrocarbon radicals which have one or more rings and may be unsubstituted or substituted. The term aryl generally refers to hydrocarbon radicals having from 6 to 10, up to 14, up to 18, preferably from 6 to 10, ring carbons. Aryl is preferably unsubstituted or substituted phenyl, naphthyl, anthracenyl, phenanthrenyl, naphthacenyl, chrysenyl, pyrenyl, etc., and particularly preferably phenyl or naphthyl. Substituted aryls can, depending on the number and size of their ring systems, have one or more (e.g. 1, 2, 3, 4, 5 or more than 5) substituents. These are preferably selected independently from among alkyl, alkoxy, cycloalkyl, cycloalkyloxy, heterocycloalkyl, aryl, aryloxy, arylthio, hetaryl, halogen, hydroxy, SH, alkylthio, alkylsulfinyl, alkylsulfonyl, COOH, carboxylate, $SO_3H$, sulfonate, $NE^5E^6$, nitro and cyano, where $E^5$ and $E^6$ are each, independently of one another, hydrogen, alkyl, cycloalkyl, cycloalkyloxy, polycyclyl, polycyclyloxy, heterocycloalkyl, aryl, aryloxy or hetaryl. Aryl is particularly preferably phenyl which, if it is substituted, can generally bear 1, 2, 3, 4 or 5 substituents, preferably 1, 2 or 3 substituents.

Aryl which bears one or more radicals is, for example, 2-, 3- and 4-methylphenyl, 2,4-, 2,5-, 3,5- and 2,6-dimethylphenyl, 2,4,6-trimethylphenyl, 2-, 3- and 4-ethylphenyl, 2,4-, 2,5-, 3,5- and 2,6-diethylphenyl, 2,4,6-triethylphenyl, 2-, 3- and 4-propylphenyl, 2,4-, 2,5-, 3,5- and 2,6-dipropylphenyl, 2,4,6-tripropylphenyl, 2-, 3- and 4-isopropylphenyl, 2,4-, 2,5-, 3,5- and 2,6-diisopropylphenyl, 2,4,6-triisopropylphenyl, 2-, 3- and 4-butylphenyl, 2,4-, 2,5-, 3,5- and 2,6-dibutylphenyl, 2,4,6-tributylphenyl, 2-, 3- and 4-isobutylphenyl, 2,4-, 2,5-, 3,5- and 2,6-diisobutylphenyl, 2,4,6-triisobutylphenyl, 2-, 3- and 4-sec-butylphenyl, 2,4-, 2,5-, 3,5- and 2,6-di-sec-butylphenyl, 2,4,6-tri-sec-butylphenyl, 2-, 3- and 4-tert-butylphenyl, 2,4-, 2,5-, 3,5- and 2,6-di-tert-butylphenyl, 2,4,6-tri-tert-butylphenyl and 2-, 3-, 4-dodecylphenyl; 2-, 3- and 4-methoxyphenyl, 2,4-, 2,5-, 3,5- and 2,6-dimethoxyphenyl, 2,4,6-trimethoxyphenyl, 2-, 3- and 4-ethoxyphenyl, 2,4-, 2,5-, 3,5- and 2,6-diethoxyphenyl, 2,4,6-triethoxyphenyl, 2-, 3- and 4-propoxyphenyl, 2,4-, 2,5-, 3,5- and 2,6-dipropoxyphenyl, 2-, 3- and 4-isopropoxyphenyl, 2,4-, 2,5-, 3,5- and 2,6-diisopropoxyphenyl, 2-, 3- and 4-butoxyphenyl, 2-, 3-, 4-hexyloxyphenyl; 2-, 3-, 4-chlorophenyl, 2,4-, 2,5-, 3,5- and 2,6-dichlorophenyl, trichlorophenyl, 2-, 3-, 4-fluorophenyl, 2,4-, 2,5-, 3,5- and 2,6-difluorophenyl, trifluorophenyl, for example 2,4,6-trifluorophenyl, tetrafluorophenyl, pentafluorophenyl, 2-, 3- and 4-cyanophenyl; 2-nitrophenyl, 4-nitrophenyl, 2,4-dinitrophenyl, 2,6-dinitrophenyl; 4-dimethylaminophenyl; 4-acetylphenyl; methoxyethylphenyl, ethoxymethylphenyl; methylthiophenyl, isopropylthiophenyl or tert-butylthiophenyl; methylnaphthyl; isopropylnaphthyl or ethoxynaphthyl. Examples of substituted aryl in which two substituents bound to adjacent carbon atoms of the aryl ring form a fused ring or fused ring system are indenyl and fluorenyl.

For the purposes of the present invention, the expression "aryloxy" refers to aryl bound via an oxygen atom.

For the purposes of the present invention, the expression "arylthio" refers to aryl bound via a sulfur atom.

(VII) For the purposes of the present invention, the expression "heterocycloalkyl" comprises nonaromatic, unsaturated or fully saturated, cycloaliphatic groups which generally have from 5 to 8 ring atoms, for example 5 or 6 ring atoms, and in which 1, 2 or 3 of the ring carbons have been replaced by heteroatoms selected from among oxygen, nitrogen, sulfur and an $-NR^z$-group and which are unsubstituted or substituted by one or more, for example, 1, 2, 3, 4, 5 or 6, $C_1$-$C_6$-alkyl groups. Examples of such heterocycloaliphatic groups are pyrrolidinyl, piperidinyl, 2,2,6,6-tetramethylpiperidinyl, imidazolidinyl, pyrazolidinyl, oxazolidinyl, morpholidinyl, thiazolidinyl, isothiazolidinyl, isoxazolidinyl, piperazinyl, tetrahydrothienyl, dihydrothienyl, tetrahydrofuranyl, dihydrofuranyl, tetrahydropyranyl, 1,2-oxazolin-5-yl, 1,3-oxazolin-2-yl and dioxanyl. Nitrogen-comprising heterocycloalkyl can in principle be bound either via a carbon atom or via a nitrogen atom.

(VIII) For the purposes of the present invention, the expression "heteroaryl (hetaryl)" comprises unsubstituted or substituted, heteroaromatic groups which have one or more rings and generally have from 5 to 14 ring atoms, preferably 5 or 6 ring atoms, in which 1, 2 or 3 of the ring carbons have been replaced by one, two, three or four heteroatoms selected from among O, N, $-NR^a$- and S, e.g. furyl, thienyl, oxazolyl, isoxazolyl, thiazolyl, isothiazolyl, benzofuranyl, benzthiazolyl, benzimidazolyl, pyridyl, quinolinyl, acridinyl, pyridazinyl, pyrimidinyl, pyrazinyl, pyrrolyl, imidazolyl, pyrazolyl, indolyl, purinyl, indazolyl, benzotriazolyl, 1,2,3-triazolyl, 1,3,4-triazolyl and carbazolyl, where these heterocycloaromatic groups may, if they are substituted, generally bear 1, 2 or 3 substituents. The substituents are generally selected from among $C_1$-$C_6$-alkyl, $C_1$-$C_6$-alkoxy, hydroxy, carboxy, halogen and cyano.

5- to 7-membered nitrogen-comprising heterocycloalkyl or heteroaryl radicals which may optionally comprise further heteroatoms are, for example, pyrrolyl, pyrazolyl, imidazolyl, triazolyl, pyrrolidinyl, pyrazolinyl, pyrazolidinyl, imidazolinyl, imidazolidinyl, pyridinyl, pyridazinyl, pyrimidinyl, pyrazinyl, triazinyl, piperidinyl, piperazinyl, oxazolyl, isoxazolyl, thiazolyl, isothiazolyl, indolyl, quinolinyl, isoquinolinyl or quinaldinyl which may be unsubstituted or substituted as

mentioned above.

(IX) For the purposes of the present invention, carboxylate and sulfonate are preferably a derivative of a carboxylic acid function or a sulfonic acid function, in particular a metal carboxylate or sulfonate, a carboxylic ester or sulfonic ester function or a carboxamide or sulfonamide function. These include, for example, esters with $C_1$-$C_4$-alkanols such as methanol, ethanol, n-propanol, isopropanol, n-butanol, sec-butanol and tert-butanol.

(X) For the purposes of the present invention, the expression "acyl" refers to alkanoyl, hetaroyl or aroyl groups which generally have from 1 to 11, preferably from 2 to 8, carbon atoms, for example the formyl, acetyl, propanoyl, butanoyl, pentanoyl, hexanoyl, heptanoyl, 2-ethylhexanoyl, 2-propylheptanoyl, benzoyl or naphthoyl group.

Very suitable are $C_1$ to $C_{30}$ organic residues which are chosen amongst the above defined groups (I), (III), (V) and (VI).

Halogen is fluorine, chlorine, bromine or iodine herein.

Suitable monosubstituted oxalic acid derivatives of the general formula (I) have a molar mass of less than 1000 g/mol, preferably less than 800 g/mol and in particular in the range of 100 to 600 g/mol.

The meaning of the substituents and terms in formula (I) is the following:

> $[A]^+$ is a cation made from an organic moiety A having a formally positively charged heteroatom selected from the group consisting of nitrogen, phosphorus and sulfur, preferably from nitrogen and phosphorus. The organic moiety which contains the formally positively charged heteroatom can be linear or cyclic. The organic moiety which contains a nitrogen atom as formally positively charged heteroatom is preferably cyclic and does for example include the respective compounds mentioned under the groups (VII) and (VIII) above. The organic moiety which contains a nitrogen atom as formally positively charged heteroatom is more preferably a five-membered heterocycle having one formally positively charged nitrogen atom and may further contain one or more other heteroatoms which are not formally positively charged.

Preferably $[A]^+$ is selected from among the compounds of the formulae (IV.a) to (IV.z):

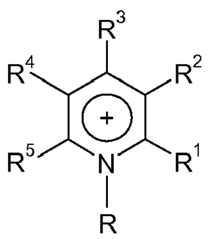

(IV.a)

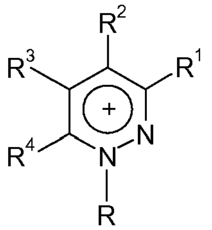

(IV.b)

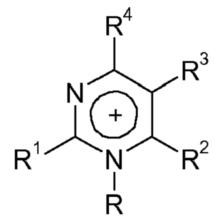

(IV.c)

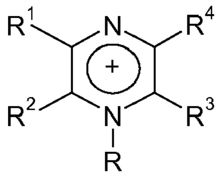

(IV.d)

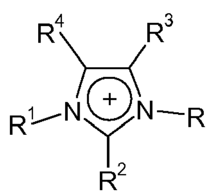

(IV.e)

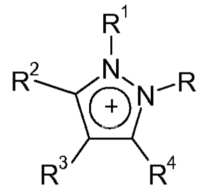

(IV.f)

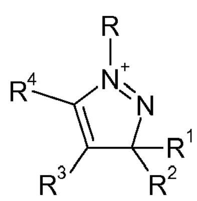

(IV.g)

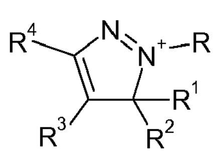

(IV.g')

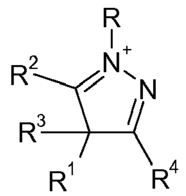

(IV.h)

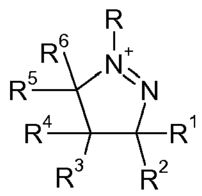
(IV.i)

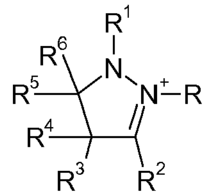
(IV.j)

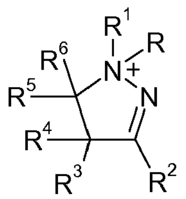
(IV.j')

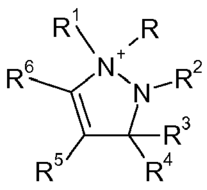
(IV.k)

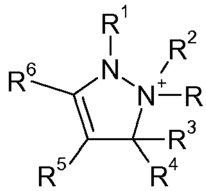
(IV.k')

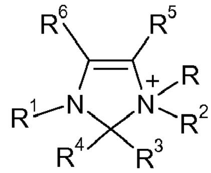
(IV.l)

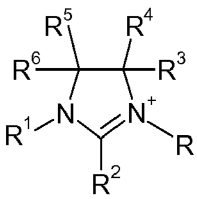
(IV.m)

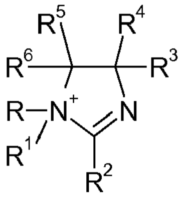
(IV.m')

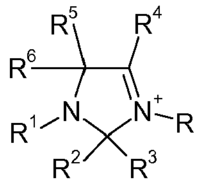
(IV.n)

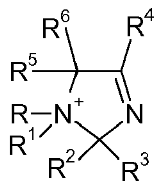
(IV.n')

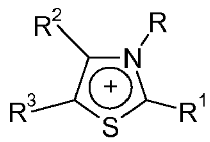
(IV.o)

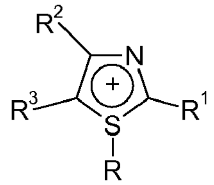
(IV.o')

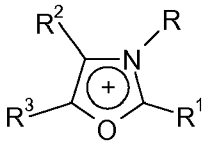
(IV.p)

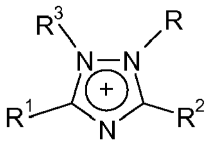
(IV.q)

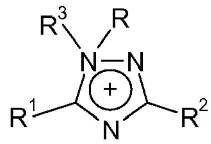
(IV.q')

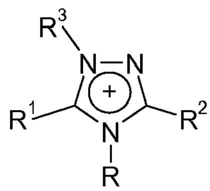
(IV.q'')

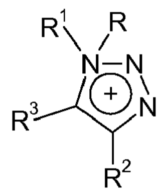
(IV.r)

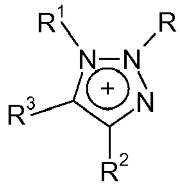
(IV.r')

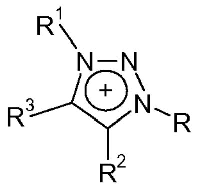

(IV.r'')

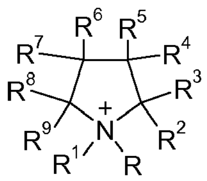

(IV.s)

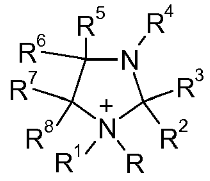

(IV.t)

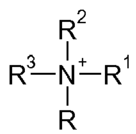

(IV.u)

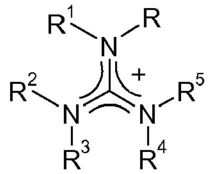

(IV.v)

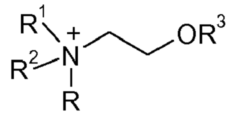

(IV.w)

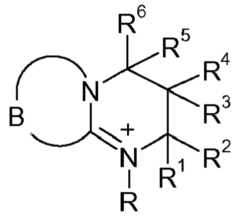

(IV.x.1)

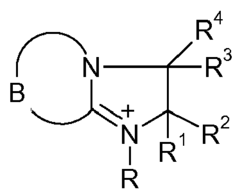

(IV.x.2)

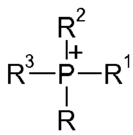

(IV.y)

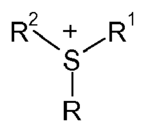

(IV.z)

and oligomers comprising these structures, where the residues R and $R^1$ to $R^9$ have the following meaning, with the proviso that at least one of R and $R^1$ is not hydrogen:

R is hydrogen or a $C_1$ to $C_{30}$ organic residue as defined above, which can be preferably selected from residues defined herein under groups (I) to (VIII) above, for example substituted or unsubstituted "alkyl", "arylalkyl", "alkenyl", "cycloalkyl", "cycloalkenyl", "polycyclyl", "aryl", "aryloxy", "arylthio", "heterocycloalkyl","heteroarayl", very preferably a straight chain or branched C1 to C30 alkyl and most preferably unsubstituted straight chain or branched $C_1$ to $C_{30}$ "alkyl" residues as defined in group (I) above, for example methyl, ethyl, 1-propyl, 2-propyl (iso-propyl), 1-butyl (n-butyl), 2-butyl (sec-butyl), 2-methyl-1-propyl (isobutyl), 2-methyl-2-propyl (tert-butyl), 1-pentyl, 2-pentyl, 3-pentyl, 2-methyl-1-butyl, 3-methyl-1-butyl, 2-methyl-2-butyl, 3-methyl-2-butyl, 2,2-dimethyl-1-propyl, 1-hexyl, 2-hexyl, 3-hexyl, 2-methyl-1-pentyl, 3-methyl-1-pentyl, 4-methyl-1-pentyl, 2-methyl-2-pentyl, 3-methyl-2-pentyl, 4-methyl-2-pentyl, 2-methyl-3-pentyl, 3-methyl-3-pentyl, 2,2-dimethyl-1-butyl, 2,3-dimethyl-1-butyl, 3,3-dimethyl-1-butyl, 2-ethyl-1-butyl, 2,3-dimethyl-2-butyl, 3,3-dimethyl-2-butyl, 1-heptyl, 1-octyl, 1-methylheptyl, 2-ethylhexyl, 2,4,4-trimethyl-pentyl, 1,1,3,3-tetramethylbutyl, 1-nonyl, 1-decyl, 1-undecyl, 1-dodecyl, 1-tridecyl, 1-tetradecyl, 1-pentadecyl, 1-hexadecyl, 1-heptadecyl, 1-octadecyl and n-eicosyl;

radicals $R^1$, $R^2$, $R^3$, $R^4$, $R^5$, $R^6$, $R^7$, $R^8$ and $R^9$ which are bound to a ring carbon are each, independently of one another, hydrogen, a sulfo group, COOH, carboxylate, sulfonate, acyl, alkoxycarbonyl, cyano, halogen, hydroxyl, SH, nitro, $NE^1E^2$, alkyl, alkoxy, alkylthio, alkylsulfinyl, alkylsulfonyl, alkenyl, cycloalkyl, cycloalkyloxy, cycloalkenyl, cycloalkenyloxy, polycyclyl, polycyclyloxy, heterocycloalkyl, aryl, aryloxy or heteroaryl, where $E^1$ and $E^2$ are each, independently of one another, hydrogen, alkyl, cycloalkyl, heterocycloalkyl, aryl or hetaryl,

radicals $R^1$, $R^2$, $R^3$, $R^4$, $R^5$, $R^6$, $R^7$, $R^8$ and $R^9$ which are bound to a ring heteroatom are each hydrogen, $SO_3H$, $NE^1E^2$, alkyl, alkoxy, alkenyl, cycloalkyl, cycloalkenyl, polycyclyl, heterocycloalkyl, aryl or heteroaryl, where $E^1$ and $E^2$ are each, independently of one another, hydrogen, alkyl, cycloalkyl, heterocycloalkyl, aryl or hetaryl, or
two adjacent radicals $R^1$ to $R^9$ together with the ring atoms to which they are bound may also form at least one fused-on, saturated, unsaturated or aromatic ring or a ring system having from 1 to 30 carbon atoms, where the ring or the ring system may have from 1 to 5 nonadjacent heteroatoms or heteroatom-comprising groups and the ring or the ring system may be unsubstituted or substituted,
two geminal radicals $R^1$ to $R^9$ may also together be =O =S or $=NR^b$, where $R^b$ is hydrogen, alkyl, cycloalkyl, aryl or heteroaryl,
and $R^1$ and $R^3$ or $R^3$ and $R^5$ in the compounds of the formula (IV.x.1) may together also represent the second part of a double bond between the ring atoms bearing these radicals,

B in the compounds of the formulae (IV.x.1) and (IV.x.2) together with the C-N group to which it is bound forms a 4- to 8-membered, saturated or unsaturated or aromatic ring which may optionally be substituted and/or may optionally have further heteroatoms or heteroatom-comprising groups and/or may comprise further fused-on, saturated, unsaturated or aromatic carbocycles or heterocycles.

As regards the general meaning of the abovementioned radicals carboxylate, sulfonate, acyl, alkoxycarbonyl, halogen, $NE^1E^2$, alkyl, alkoxy, alkylthio, alkylsulfinyl, alkylsulfonyl, alkenyl, cycloalkyl, cycloalkyloxy, cycloalkenyl, cycloalkenyloxy, polycyclyl, polycyclyloxy, heterocycloalkyl, aryl, aryloxy or heteroaryl, what has been said above applies in full. Radicals $R^1$ to $R^9$ in the abovementioned formulae (IV) which are bound to a carbon atom and have a heteroatom or a heteroatom-comprising group can also be bound directly via a heteroatom to the carbon atom.

If two adjacent radicals $R^1$ to $R^9$ together with the ring atoms to which they are bound form at least one fused-on, saturated, unsaturated or aromatic ring or a ring system having from 1 to 30 carbon atoms, where the ring or ring system can have from 1 to 5 nonadjacent heteroatoms or heteroatom-comprising groups and the ring or the ring system may be unsubstituted or substituted, these radicals can together as fused-on building blocks preferably be 1,3-propylene, 1,4-butylene, 1,5-pentylene, 2-oxa-1,3-propylene, 1-oxa-1,3-propylene, 2-oxa-1,3-propylene, 1-oxa-1,3-propenylene, 3-oxa-1,5-pentylene, 1-aza-1,3-propenylene, 1-$C_1$-$C_4$-alkyl-1-aza-1,3-propenylene, 1,4-buta-1,3-dienylene, 1-aza-1,4-buta-1,3-dienylene or 2-aza-1,4-buta-1,3-dienylene.

The radical R is for example

- hydrogen or unsubstituted $C_1$-$C_{18}$-alkyl such as methyl, ethyl, 1-propyl, 2-propyl (isopropyl), 1-butyl (n-butyl), 2-butyl (sec-butyl), 2-methyl-1-propyl (isobutyl), 2-methyl-2-propyl (tert-butyl), 1-pentyl, 2-pentyl, 3-pentyl, 2-methyl-1-butyl, 3-methyl-1-butyl, 2-methyl-2-butyl, 3-methyl-2-butyl, 2,2-dimethyl-1-propyl, 1-hexyl, 2-hexyl, 3-hexyl, 2-methyl-1-pentyl, 3-methyl-1-pentyl, 4-methyl-1-pentyl, 2-methyl-2-pentyl, 3-methyl-2-pentyl, 4-methyl-2-pentyl, 2-methyl-3-pentyl, 3-methyl-3-pentyl, 2,2-dimethyl-1-butyl, 2,3-dimethyl-1-butyl, 3,3-dimethyl-1-butyl, 2-ethyl-1-butyl, 2,3-dimethyl-2-butyl, 3,3-dimethyl-2-butyl, 1-heptyl, 1-octyl, 1-methylheptyl, 2-ethylhexyl, 2,4,4-trimethyl-pentyl, 1,1,3,3-tetramethylbutyl, 1-nonyl, 1-decyl, 1-undecyl, 1-dodecyl, 1-tridecyl, 1-tetradecyl, 1-pentadecyl, 1-hexadecyl, 1-heptadecyl and 1-octadecyl;

- $C_1$-$C_{18}$-alkyl substituted by one or more hydroxy, halogen, phenyl, cyano, $C_1$-$C_6$-alkoxycarbonyl and/or $SO_3H$ groups, especially hydroxy-$C_1$-$C_{18}$-alkyl such as 2-hydroxyethyl or 6-hydroxyhexyl; phenyl-$C_1$-$C_{18}$-alkyl, such as benzyl, 3-phenylpropyl; cyano-$C_1$-$C_{18}$-alkyl, such as 2-cyanoethyl; $C_1$-$C_6$-alkoxy-$C_1$-$C_{18}$-alkyl, such as 2-(methoxycarbonyl)ethyl, 2-(ethoxycarbonyl)ethyl or 2-(n-butoxycarbonyl)ethyl; $C_1$-$C_{18}$-fluoroalkyl such as trifluoromethyl, difluoromethyl, fluoromethyl, pentafluoroethyl, heptafluoropropyl, heptafluoroisopropyl, nonafluorobutyl, nonafluoroisobutyl, undecylfluoropentyl, undecylfluoroisopentyl; sulfo-$C_1$-$C_{18}$-alkyl such as 3-sulfopropyl;
- hydroxyethyloxyalkyl, radicals of oligoalkylene and polyalkylene glycols such as polyethylene glycols and polypropylene glycols and their oligomers having 2 to 100 units and a hydrogen or a $C_1$-$C_8$-alkyl as end group, for example $R^AO-(CHR^B-CH_2-O)_n-CHR^B-CH_2-$ where $R^A$ and $R^B$ are preferably each hydrogen, methyl or ethyl and n is preferably from 0 to 3, in particular 3-oxabutyl, 3-oxapentyl, 3,6-dioxaheptyl, 3,6-dioxaoctyl, 3,6,9-trioxadecyl, 3,6,9-trioxaundecyl, 3,6,9,12-tetraoxatridecyl and 3,6,9,12-tetraoxatetradecyl; and
- $C_2$-$C_6$-alkenyl such as vinyl or propenyl.

Preference is given to the radicals $R^1$ to $R^9$ each being, independently of one another,

- hydrogen;
- halogen;
- a functional group selected from among hydroxy, alkoxy, alkylthio, carboxyl, -COOH, sulfonate, cyano, acyl, alkoxycarbonyl, $NE^1E^2$ and nitro, where $E^1$ and $E^2$ are as defined above;
- $C_1$-$C_{18}$-alkyl which is unsubstituted or substituted as defined above and/or may, as defined above, be interrupted by at least one heteroatom or heteroatom-comprising group;
- $C_2$-$C_{18}$-alkenyl which is unsubstituted or substituted as defined above and/or may, as defined above, be interrupted by at least one heteroatom;
- $C_6$-$C_{10}$-aryl which is unsubstituted or substituted as defined above;
- $C_5$-$C_{12}$-cycloalkyl which is unsubstituted or substituted as defined above;
- polycyclyl which is unsubstituted or substituted as defined above;
- $C_5$-$C_{12}$-cycloalkenyl which is unsubstituted or substituted as defined above;
- heterocycloalkyl having 5 or 6 ring atoms, where the ring comprises, apart from ring carbons, 1, 2 or 3 heteroatoms or heteroatom-comprising groups selected from among oxygen, nitrogen, sulfur and $NR^a$ and is unsubstituted or substituted as defined above;
- heteroaryl having from 5 to 10 ring atoms, where the ring has, apart from ring carbons, 1, 2 or 3 heteroatoms or heteroatom-comprising groups selected from among oxygen, nitrogen, sulfur and $NR^a$ and is unsubstituted or substituted as defined above.

Preference is likewise given to two adjacent radicals $R^1$ to $R^9$ together with the ring atoms to which they are bound forming at least one fused-on, saturated, unsaturated or aromatic ring or ring system having from 1 to 12 carbon atoms, where the ring or ring system may have from 1 to 5 nonadjacent heteroatoms or heteroatom-comprising groups which are preferably selected from among oxygen, nitrogen, sulfur and $NR^z$ and the ring or the ring system may be unsubstituted or substituted, where the substituents are preferably selected independently from among alkoxy, cycloalkyl, cycloalkoxy, polycyclyl, polycyclyloxy, heterocycloalkyl, aryl, aryloxy, arylthio, heteroaryl halogen, hydroxy, SH, =O =S, $=NR^z$, COOH, carboxylate, $-SO_3H$, sulfonate, $NE^1E^2$, nitro and cyano, where $E^1$ and $E^2$ are each, independently of one another, hydrogen, alkyl, cycloalkyl, heterocycloalkyl, aryl or hetaryl.

If $R^1$ to $R^9$ are alkoxy, then $R^1$ to $R^9$ are preferably methoxy or ethoxy or $R^AO-(CH_2CH_2CH_2CH_2O)_n-CH_2CH_2CH_2CH_2O-$ where $R^A$ and $R^B$ are preferably each hydrogen, methyl or ethyl and n is preferably from 0 to 3.

If $R^1$ to $R^9$ are acyl, then $R^1$ to $R^9$ are preferably formyl or $C_1$-$C_4$-alkylcarbonyl, in particular formyl or acetyl.

If $R^1$ to $R^9$ are $C_1$-$C_{18}$-alkyl, then $R^1$ to $R^9$ are preferably:

unsubstituted $C_1$-$C_{18}$-alkyl such as , for example methyl, ethyl, 1-propyl, 2-propyl (iso-propyl), 1-butyl (n-butyl), 2-butyl (sec-butyl), 2-methyl-1-propyl (isobutyl), 2-methyl-2-propyl (tert-butyl), 1-pentyl, 2-pentyl, 3-pentyl, 2-methyl-1-butyl, 3-methyl-1-butyl, 2-methyl-2-butyl, 3-methyl-2-butyl, 2,2-dimethyl-1-propyl, 1-hexyl, 2-hexyl, 3-hexyl, 2-methyl-1-pentyl, 3-methyl-1-pentyl, 4-methyl-1-pentyl, 2-methyl-2-pentyl, 3-methyl-2-pentyl, 4-methyl-2-pentyl, 2-methyl-3-pentyl, 3-methyl-3-pentyl, 2,2-dimethyl-1-butyl, 2,3-dimethyl-1-butyl, 3,3-dimethyl-1-butyl, 2-ethyl-1-butyl, 2,3-dimethyl-2-butyl, 3,3-dimethyl-2-butyl, 1-heptyl, 1-octyl, 1-methylheptyl, 2-ethylhexyl, 2,4,4-trimethyl-pentyl, 1,1,3,3-tetramethylbutyl, 1-nonyl, 1-decyl, 1-undecyl, 1-dodecyl, 1-tridecyl, 1-tetradecyl, 1-pentadecyl, 1-hexadecyl, 1-heptadecyl and 1-octadecyl;

$C_1$-$C_{18}$-haloalkyl, especially $C_1$-$C_{18}$-fluoroalkyl, for example trifluoromethyl, difluoromethyl, fluoromethyl, pentafluoroethyl, heptafluoropropyl, heptafluoroisopropyl, nonafluorobutyl, nonafluoroisobutyl, undecylfluoropentyl, undecylisopentyl, $C_6F_{13}$, $C_8F_{17}$, $C_{10}F_{21}$, $C_{12}F_{25}$, especially $C_1$-$C_{18}$-chloroalkyl such as chloromethyl, 2-chloroethyl, trichloromethyl, 1,1-dimethyl-2-chloroethyl;

amino-$C_1$-$C_{18}$-alkyl such as 2-aminoethyl, 2-aminopropyl, 3-aminopropyl, 4-aminobutyl, 6-aminohexyl,

$C_1$-$C_6$-alkylamino-$C_1$-$C_{18}$-alkyl such as 2-methylaminoethyl, 2-methylaminopropyl, 3-methylaminopropyl, 4-methylaminobutyl, 6-methylaminohexyl;

di($C_1$-$C_6$-alkyl)-$C_1$-$C_{18}$-alkyl such as 2-dimethylaminoethyl, 2-dimethylaminopropyl, 3-dimethylaminopropyl, 4-dimethylaminobutyl, 6-dimethylaminohexyl,

cyano-$C_1$-$C_{18}$-alkyl such as 2-cyanoethyl, 2-cyanopropyl,

$C_1$-$C_{10}$-alkoxy-$C_1$-$C_{18}$-alkyl such as methoxymethyl, 2-methoxyethyl, 2-methoxypropyl, 3-methoxypropyl, 2-methoxyisopropyl, 4-methoxybutyl, 6-methoxyhexyl, 2-ethoxyethyl, 2-ethoxypropyl, 3-ethoxypropyl, 4-ethoxybutyl, 6-ethoxyhexyl, 2-isopropoxyethyl, 2-butoxyethyl, 2-butoxypropyl, 2-octyloxyethyl, 5-methoxy-3-oxa-pentyl, 8-methoxy-3,6-dioxaoctyl, 7-methoxy-4-oxaheptyl, 11-methoxy-4,8-dioxaundecyl, 9-methoxy-5-oxanonyl, 9-methoxy-5-oxanonyl, 14-methoxy-5,10-dioxatetradecyl, 5-ethoxy-3-oxapentyl, 8-ethoxy-3,6-dioxaoctyl, 7-ethoxy-4-oxaheptyl, 11-ethoxy-4,8-dioxaundecyl, 9-ethoxy-5-oxanonyl or 14-ethoxy-5,10-oxatetradecyl, 15-methoxy-4,8,12-trioxapentadecyl, 11-methoxy-3,6,9-trioxaundecyl, 11-ethoxy-3,6,9-trioxaundecyl, 15-ethoxy-4,8,12-trioxapentadecyl;

di($C_1$-$C_{10}$-alkoxy-$C_1$-$C_{18}$-alkyl) such as diethoxymethyl or diethoxyethyl,

$C_1$-$C_6$-alkoxycarbonyl-$C_1$-$C_{18}$-alkyl such as 2-(methoxycarbonyl)ethyl, 2-(ethoxycarbonyl)ethyl, 2-(n-butoxycarbonyl)ethyl,

di($C_1$-$C_6$-alkoxycarbonyl)-$C_1$-$C_{18}$-alkyl such as 1,2-di(methoxycarbonyl)ethyl,

hydroxy-$C_1$-$C_{18}$-alkyl such as 2-hydroxyethyl, 2-hydroxypropyl, 3-hydroxypropyl, 4-hydroxybutyl, 6-hydroxyhexyl, 2-hydroxy-2,2-dimethylethyl, 5-hydroxy-3-oxapentyl, 8-hydroxy-3,6-dioxaoctyl, 11-hydroxy-3,6,9-trioxaundecyl, 7-hydroxy-4-oxaheptyl, 11-hydroxy-4,8-dioxaundecyl, 15-hydroxy-4,8,12-trioxapentadecyl, 9-hydroxy-5-oxanonyl, 14-hydroxy-5,10-dioxatetradecyl;

$C_1$-$C_{12}$-alkylsulfanyl-$C_1$-$C_{18}$-alkyl such as butylthiomethyl, 2-dodecylthioethyl,

$C_5$-$C_{12}$-cycloalkyl-$C_1$-$C_{18}$-alkyl such as cyclopentylmethyl, 2-cyclopentylethyl, 3-cyclopentylpropyl, cyclohexylmethyl, 2-cyclohexylethyl, 3-cyclohexylpropyl, phenyl-$C_1$-$C_{18}$-alkyl, where the phenyl part of phenyl-$C_1$-$C_{18}$-alkyl is unsubstituted or substituted by one, two, three or four substituents selected independently from among $C_1$-$C_6$-alkyl, halogen, $C_1$-$C_6$-alkoxy and nitro, e.g. benzyl (phenylmethyl), 1-phenylethyl, 2-phenylethyl, 3-phenylpropyl, p-tolylmethyl, 1-(p-butylphenyl)ethyl, p-chlorobenzyl, 2,4-dichlorobenzyl, p-methoxybenzyl, m-ethoxybenzyl, phenyl-$C(CH_3)_2$-, 2,6-dimethylphenylmethyl, diphenyl-$C_1$-$C_{18}$-alkyl such as diphenylmethyl (benzhydryl); triphenyl-$C_1$-$C_{18}$-alkyl such as triphenylmethyl;

phenoxy-$C_1$-$C_{18}$-alkyl such as 2-phenoxyethyl, 2-phenoxypropyl, 3-phenoxypropyl, 4-phenoxybutyl, 6-phenoxyhexyl;

phenylthio-$C_1$-$C_{18}$-alkyl such as 2-phenylthioethyl.

If $R^1$ to $R^9$ are $C_2$-$C_{18}$-alkenyl, then $R^1$ to $R^9$ are preferably each $C_2$-$C_6$-alkenyl such as vinyl, 2-propenyl, 3-butenyl, cis-2-butenyl, trans-2-butenyl or $C_2$-$C_{18}$-alkenyl which may be partially or completely substituted by fluorine.

If $R^1$ to $R^9$ are $C_6$-$C_{10}$-aryl, then $R^1$ to $R^9$ are each preferably phenyl or naphthyl, where phenyl or naphthyl is unsubstituted or substituted by one, two, three or four substituents selected independently from among halogen, $C_1$-$C_{15}$-alkyl, $C_1$-$C_6$-alkoxy, $C_1$-$C_6$-alkylsulfanyl, $C_1$-$C_6$-alkoxy-$C_1$-$C_6$-alkyl, $C_1$-$C_6$-alkylcarbonyl, amino, $C_1$-$C_6$-alkylamino, di($C_1$-$C_6$-dialkyl)amino and nitro, e.g. phenyl, methylphenyl (tolyl), dimethylphenyl (xylyl) such as 2,6-dimethylphenyl, trimethylphenyl such as 2,4,6-trimethylphenyl, ethylphenyl, diethylphenyl, isopropylphenyl, tert-butylphenyl, dodecylphenyl, chlorophenyl, dichlorophenyl, trichlorophenyl, fluorophenyl, difluorophenyl, trifluorophenyl, tetrafluorophenyl,

pentafluorophenyl, 2,6-dichlorophenyl, 4-bromophenyl, methoxyphenyl, dimethoxyphenyl, ethoxyphenyl, hexyloxyphenyl, 2,6-dimethoxyphenyl, 2-nitrophenyl, 4-nitrophenyl, 2,4-dinitrophenyl, 2,6-dinitrophenyl, 4-dimethylaminophenyl, 4-acetylphenyl, methoxyethylphenyl, ethoxymethylphenyl, methylthiophenyl, isopropylthiophenyl, tert-butylthiophenyl, $\alpha$-naphthyl, $\beta$-naphthyl, methylnaphthyl, isopropylnaphthyl, chloronaphthyl, ethoxynaphthyl or partially fluorinated phenyl or perfluorinated phenyl.

If $R^1$ to $R^9$ are $C_5$-$C_{12}$-cycloalkyl, then $R^1$ to $R^9$ are preferably each unsubstituted cycloalkyl such as cyclopentyl or cyclohexyl;

$C_5$-$C_{12}$-cycloalkyl which is substituted by one or two substituents selected independently from among $C_1$-$C_6$-alkyl, $C_1$-$C_6$-alkoxy, $C_1$-$C_6$-alkylsulfanyl and chlorine, e.g. butylcyclohexyl, methoxycyclohexyl, dimethoxycyclohexyl, diethoxycyclohexyl, butylthiocyclohexyl, chlorocyclohexyl, dichlorocyclohexyl, dichlorocyclopentyl;

$C_5$-$C_{12}$-cycloalkyl which is completely or fully fluorinated.

If $R^1$ to $R^9$ are polycyclyl, then $R^1$ to $R^9$ are each preferably $C_5$-$C_{12}$-bicycloalkyl such as norbornyl or $C_5$-$C_{12}$-bicycloalkenyl such as norbornenyl.

If $R^1$ to $R^9$ are $C_5$-$C_{12}$-cycloalkenyl, then $R^1$ to $R^9$ are each preferably unsubstituted cycloalkenyl such as cyclopent-2-en-1-yl, cyclopent-3-en-1-yl, cyclohex-2-en-1-yl, cyclohex-1-en-1-yl, cyclohexa-2,5-dien-1-yl or partially or completely fluorinated cycloalkenyl.

If $R^1$ to $R^9$ are heterocycloalkyl having 5 or 6 ring atoms, then $R^1$ to $R^9$ are each preferably 1,3-dioxolan-2-yl, 1,3-dioxan-2-yl, 2-methyl-1,3-dioxolan-2-yl, 4-methyl-1,3-dioxolan-2-yl.

If $R^1$ to $R^9$ are heteroaryl, then $R^1$ to $R^9$ are each preferably furyl, thienyl, pyrryl, pyridyl, indolyl, benzoxazolyl, benzimidazolyl, benzothiazolyl. If hetaryl is substituted, it bears 1, 2 or 3 substituents selected independently from among $C_1$-$C_6$-alkyl, $C_1$-$C_6$-alkoxy and halogen, for example dimethylpyridyl, methylquinolyl, dimethylpyrryl, methoxyfuryl, dimethoxypyridyl or difluoropyridyl.

In the following described very particularly preferred [$A^+$] cations (IVa) to (IVz) the substituent R has the following meaning:

R is hydrogen or preferably a $C_1$ to $C_{30}$ organic residue as defined above, which can be preferably selected from residues defined herein under groups (I) to (VIII) above, for example substituted or unsubstituted "alkyl", "arylalkyl", "alkenyl", "cycloalkyl", "cycloalkenyl", "polycyclyl", "aryl", "aryloxy", "arylthio", "heterocycloalkyl","heteroaryl", very preferably a straight chain or branched $C_1$ to $C_{30}$ alkyl and most preferably unsubstituted straight chain or branched $C_1$ to $C_{30}$ "alkyl" residues as defined in group (I) above, for example methyl, ethyl, 1-propyl, 2-propyl (iso-propyl), 1-butyl (n-butyl), 2-butyl (sec-butyl), 2-methyl-1-propyl (isobutyl), 2-methyl-2-propyl (tert-butyl), 1-pentyl, 2-pentyl, 3-pentyl, 2-methyl-1-butyl, 3-methyl-1-butyl, 2-methyl-2-butyl, 3-methyl-2-butyl, 2,2-dimethyl-1-propyl, 1-hexyl, 2-hexyl, 3-hexyl, 2-methyl-1-pentyl, 3-methyl-1-pentyl, 4-methyl-1-pentyl, 2-methyl-2-pentyl, 3-methyl-2-pentyl, 4-methyl-2-pentyl, 2-methyl-3-pentyl, 3-methyl-3-pentyl, 2,2-dimethyl-1-butyl, 2,3-dimethyl-1-butyl, 3,3-dimethyl-1-butyl, 2-ethyl-1-butyl, 2,3-dimethyl-2-butyl, 3,3-dimethyl-2-butyl, 1-heptyl, 1-octyl, 1-methylheptyl, 2-ethylhexyl, 2,4,4-trimethyl-pentyl, 1,1,3,3-tetramethylbutyl, 1-nonyl, 1-decyl, 1-undecyl, 1-dodecyl, 1-tridecyl, 1-tetradecyl, 1-pentadecyl, 1-hexadecyl, 1-heptadecyl, 1-octadecyl and n-eicosyl.

Very particularly preferred pyridinium ions (IVa) are those in which

- one of the radicals $R^1$ to $R^5$ is methyl, ethyl or chlorine and the remaining radicals $R^1$ to $R^5$ are each hydrogen;
- $R^3$ is dimethylamino and the remaining radicals $R^1$, $R^2$, $R^4$ and $R^5$ are each hydrogen;
- all radicals $R^1$ to $R^5$ are hydrogen;
- $R^2$ is carboxy or carboxamide and the remaining radicals $R^1$, $R^2$, $R^4$ and $R^5$ are each hydrogen; or
- $R^1$ and $R^2$ or $R^2$ and $R^3$ are 1,4-buta-1,3-dienylene and the remaining radicals $R^1$, $R^3$, $R^4$ and $R^5$ are each hydrogen; $R^4$ and

and in particular those in which

- $R^1$ to $R^5$ are each hydrogen; or
- one of the radicals $R^1$ to $R^5$ is methyl or ethyl and the remaining radicals $R^1$ to $R^5$ are each hydrogen.

Particularly preferred pyridinium ions (IVa) are pyridinium, 2-methylpyridinium, 2-ethylpyridinium, 5-ethyl-2-methylpyridinium and 2-methyl-3-ethylpyridinium and also 1-methylpyridinium, 1-ethylpyridinium, 1-(1-butyl)pyridinium, 1-(1-hexyl)pyridinium, 1-(1-octyl)pyridinium, 1-(1-hexyl)pyridinium, 1-(1-octyl)pyridinium, 1-(1-dodecyl)pyridinium, 1-(1-tetradecyl)pyridinium, t-(1-hexadecyl)pyridinium, 1,2-dimethylpyridinium, 1-ethyl-2-methylpyridinium, 1-(1-butyl)-2-methylpyridinium, 1-(1-hexyl)-2-methylpyridinium, 1-(1-octyl)-2-methylpyridinium, 1-(1-dodecyl)-2-methylpyridinium, 1-(1-tetradecyl)-2-methylpyridinium, 1-(1-hexadecyl)-2-methylpyridinium, 1-methyl-2-ethylpyridinium, 1,2-diethylpyridinium, 1-(1-butyl)-2-ethylpyridinium, 1-(1-hexyl)-2-ethylpyridinium, 1-(1-octyl)-2-ethylpyridinium, 1-(1-dodecyl)-2-ethyl-

pyridinium, 9-(1-tetradecyl)-2-ethylpyridinium, 1-(1-hexadecyl)-2-ethylpyridinium, 1,2-dimethyl-5-ethylpyridinium, 1,5-diethyl-2-methylpyridinium, 1-(1-butyl)-2-methyl-3-ethyl-pyridinium, 1-(1-hexyl)-2-methyl-3-ethylpyridinium and 1-(1-octyl)-2-methyl-3-ethylpyridinium, 1-(1-dodecyl)-2-methyl-3-ethylpyridinium, 1-(1-tetradecyl)-2-methyl-3-ethylpyridinium and 1-(1-hexadecyl)-2-methyl-3-ethylpyridinium.

Particularly preferred pyridazinium ions (IVb) are those in which
the radicals $R^1$ to $R^4$ are each hydrogen or
one of the radicals $R^1$ to $R^4$ is methyl or ethyl and the remaining radicals $R^1$ to $R^4$ are each hydrogen.

Particularly preferred pyrimidinium ions (IVc) are those in which
$R^1$ is hydrogen, methyl or ethyl and $R^2$ to $R^4$ are each, independently of one another, hydrogen or methyl, or
$R^1$ is hydrogen, methyl or ethyl and $R^2$ and $R^4$ are each methyl and $R^3$ is hydrogen.

Particularly preferred pyrazinium ions (IVd) are those in which
$R^1$ is hydrogen, methyl or ethyl and $R^2$ to $R^4$ are each, independently of one another, hydrogen or methyl, or
$R^1$ is hydrogen, methyl or ethyl and $R^2$ and $R^4$ are each methyl and $R^3$ is hydrogen, or
$R^1$ to $R^4$ are each methyl or
$R^1$ to $R^4$ are each hydrogen.

Particularly preferred imidazolium ions (IVe) are those in which
$R^1$ is hydrogen, methyl, ethyl, 1-propyl, 2-propyl (iso-propyl), 1-butyl (n-butyl), 2-butyl (sec-butyl), 2-methyl-1-propyl (isobutyl), 2-methyl-2-propyl (tert-butyl), 1-pentyl, 2-pentyl, 3-pentyl, 2-methyl-1-butyl, 3-methyl-1-butyl, 2-methyl-2-butyl, 3-methyl-2-butyl, 2,2-dimethyl-1-propyl, 1-hexyl, 2-hexyl, 3-hexyl, 2-methyl-1-pentyl, 3-methyl-1-pentyl, 4-methyl-1-pentyl, 2-methyl-2-pentyl, 3-methyl-2-pentyl, 4-methyl-2-pentyl, 2-methyl-3-pentyl, 3-methyl-3-pentyl, 2,2-dimethyl-1-butyl, 2,3-dimethyl-1-butyl, 3,3-dimethyl-1-butyl, 2-ethyl-1-butyl, 2,3-dimethyl-2-butyl, 3,3-dimethyl-2-butyl, 1-heptyl, 1-octyl, 1-methylheptyl, 2-ethylhexyl, 2,4,4-trimethyl-pentyl, 1,1,3,3-tetramethylbutyl, 1-nonyl, 1-decyl, 1-undecyl, 1-dodecyl, 1-tridecyl, 1-tetradecyl, 1-pentadecyl, 1-hexadecyl, 1-heptadecyl, 1-octadecyl and n-eicosyl, 2-hydroxyethyl or 2-cyanoethyl and $R^2$ to $R^4$ are each, independently of one another, hydrogen, methyl or ethyl.

Particularly useful imidazolium ions (IVe) are 1-methylimidazolium, 1-ethylimidazolium, 1-(1-propyl)imidazolium, 1-(1-allyl)imidazolium, 1-(1-butyl)imidazolium, 1-(1-octyl)imidazolium, 1-(1-dodecyl)imidazolium, 1-(1-tetradecyl)imidazolium, 1-(1-hexadecyl)imidazolium, 1,3-dimethylimidazolium, 1,3-diethylimidazolium, 1-ethyl-3-methylimidazolium, 1-(1-butyl)-3-methylimidazolium, 1-(1-butyl)-3-ethylimidazolium, 1-(1-hexyl)-3-methylimidazolium, 1-(1-hexyl)-3-ethylimidazolium, 1-(1-hexyl)-3-butylimidazolium, 1-(1-octyl)-3-methylimidazolium, 1-(1-octyl)-3-ethylimidazolium, 1-(1-octyl)-3-butylimidazolium, 1-(2-ethylhexyl)-3-methylimidazolium, 1-(1-decyl)-3-methylimidazolium, 1-(1-dodecyl)-3-methylimidazolium, 1-(1-dodecyl)-3-ethylimidazolium, 1-(1-dodecyl)-3-butylimidazolium, 1-(1-dodecyl)-3-octylimidazolium, 1-(1-tetradecyl)-3-methylimidazolium, 1-(1-tetradecyl)-3-ethylimidazolium, 1-(1-tetradecyl)-3-butylimidazolium, 1-(1-tetradecyl)-3-octylimidazolium, 1-(1-hexadecyl)-3-methylimidazolium, 1-(1-hexadecyl)-3-ethylimidazolium, 1-(1-hexadecyl)-3-butylimidazolium, 1-(1-hexadecyl)-3-octylimidazolium, 1,2-dimethylimidazolium, 1,2,3-trimethylimidazolium, 1-ethyl-2,3-dimethylimidazolium, 1-(1-butyl)-2,3-dimethylimidazolium, 1-(1-hexyl)-2,3-dimethylimidazolium, 1-(1-octyl)-2,3-dimethylimidazolium, 1,4-dimethylimidazolium, 1,3,4-trimethylimidazolium, 1,4-dimethyl-3-ethylimidazolium, 3-methylimidazolium, 3-ethylimidazolium, 3-n-propylimidazolium, 3-n-butylimidazolium, 1,4-dimethyl-3-octylimidazolium, 1,4,5-trimethylimidazolium, 1,3,4,5-tetramethylimidazolium, 1,4,5-trimethyl-3-ethylimidazolium, 1,4,5-trimethyl-3-butylimidazolium, 1,4,5-trimethyl-3-octylimidazolium, 1-(prop-1-en-3-yl)-3-methylimidazolium and 1-(prop-1-en-3-yl)-3-butylimidazolium. Especially suitable imidazolium ions (IVe) are 1,3-dimethylimidazolium, 1,3-diethylimidazolium, 1-ethyl-3-methylimidazolium, 1-(1-butyl)-3-methylimidazolium, 1-(2-ethylhexyl)-3-methylimidazolium, 1-(1-decyl)-3-methylimidazolium.

Particularly preferred pyrazolium ions (IVf), (IVg) and (IVg') are those in which
$R^1$ is hydrogen, methyl or ethyl and $R^2$ to $R^4$ are each, independently of one another, hydrogen or methyl.

Particularly preferred pyrazolium ions (IVh) are those in which
$R^1$ to $R^4$ are each, independently of one another, hydrogen or methyl.

As particularly preferred pyrazolium ions, mention may be made of pyrazolium and 1,4-dimethylpyrazolium.

1-Pyrazolinium ions (IVi) used in the process of the invention are particularly preferably those in which
$R^1$ to $R^6$ are each, independently of one another, hydrogen or methyl.

Particularly preferred 2-pyrazolinium ions (IVj) and (IVj') are those in which
$R^1$ is hydrogen, methyl, ethyl or phenyl and $R^2$ to $R^6$ are each, independently of one another, hydrogen or methyl.

Particularly preferred 3-pyrazolinium ions (IVk) and (IVk') are those in which
$R^1$ and $R^2$ are each, independently of one another, hydrogen, methyl, ethyl or phenyl and $R^3$ to $R^6$ are each, independently of one another, hydrogen or methyl.

Particularly preferred imidazolinium ions (IV I) are those in which
$R^1$ and $R^2$ are each, independently of one another, hydrogen, methyl, ethyl, 1-butyl or phenyl and $R^3$ and $R^4$ are each, independently of one another, hydrogen, methyl or ethyl and $R^5$ and $R^6$ are each, independently of one another, hydrogen or methyl.

Particularly preferred imidazolinium ions (IVm) and (IVm') are those in which
$R^1$ and $R^2$ are each, independently of one another, hydrogen, methyl or ethyl and $R^3$ to $R^6$ are each, independently of one another, hydrogen or methyl.

Particularly preferred imidazolinium ions (IVn) and (IVn') are those in which
$R^1$ to $R^3$ are each, independently of one another, hydrogen, methyl or ethyl and $R^4$ to $R^6$ are each, independently of one another, hydrogen or methyl.

Particularly preferred thiazolium ions (IVo) and (IVo') and oxazolium ions (IVp) are those in which
$R^1$ is hydrogen, methyl, ethyl or phenyl and $R^2$ and $R^3$ are each, independently of one another, hydrogen or methyl.

1,2,4-Triazolium ions (IVq), (IVq') and (IVq") used in the process of the invention are particularly preferably those in which
$R^1$ and $R^2$ are each, independently of one another, hydrogen, methyl, ethyl or phenyl and $R^3$ is hydrogen, methyl or phenyl.

Particularly preferred 1,2,3-triazolium ions (IVr), (IVr') and (IVr") are those in which
$R^1$ is hydrogen, methyl or ethyl, $R^2$ and $R^3$ are each, independently of one another, hydrogen or methyl or $R^2$ and $R^3$ are together 1,4-buta-1,3-dienylene.

Particularly preferred pyrrolidinium ions (IVs) are those in which
$R^1$ is hydrogen, methyl, ethyl, 1-propyl, 2-propyl (iso-propyl), 1-butyl (n-butyl), 2-butyl (sec-butyl), 2-methyl-1-propyl (isobutyl), 2-methyl-2-propyl (tert-butyl), 1-pentyl, 2-pentyl, 3-pentyl, 2-methyl-1-butyl, 3-methyl-1-butyl, 2-methyl-2-butyl, 3-methyl-2-butyl, 2,2-dimethyl-1-propyl, 1-hexyl, 2-hexyl, 3-hexyl, 2-methyl-1-pentyl, 3-methyl-1-pentyl, 4-methyl-1-pentyl, 2-methyl-2-pentyl, 3-methyl-2-pentyl, 4-methyl-2-pentyl, 2-methyl-3-pentyl, 3-methyl-3-pentyl, 2,2-dimethyl-1-butyl, 2,3-dimethyl-1-butyl, 3,3-dimethyl-1-butyl, 2-ethyl-1-butyl, 2,3-dimethyl-2-butyl, 3,3-dimethyl-2-butyl, 1-heptyl, 1-octyl, 1-methylheptyl, 2-ethylhexyl, 2,4,4-trimethyl-pentyl, 1,1,3,3-tetramethylbutyl, 1-nonyl, 1-decyl, 1-undecyl, 1-dodecyl, 1-tridecyl, 1-tetradecyl, 1-pentadecyl, 1-hexadecyl, 1-heptadecyl, 1-octadecyl and n-eicosyl, in particular methyl, ethyl, 1-butyl, 2-ethylhexyl or phenyl and $R^2$ to $R^9$ are each, independently of one another, hydrogen or methyl. Examples for preferred pyrrolidinium ions (IVs) are the ones in which $R^1$ and R are independently from each other methyl, ethyl, 1-propyl, 2-propyl (iso-propyl), 1-butyl (n-butyl), 2-butyl (sec-butyl), 2-methyl-1-propyl (isobutyl), 2-methyl-2-propyl (tert-butyl), and $R^2$ to $R^9$ are each hydrogen, for example $R^1$ = 1-butyl, R = methyl and $R^2$ to $R^9$ are each hydrogen.

Particularly preferred imidazolidinium ions (IVt) are those in which
$R^1$ and $R^4$ are each, independently of one another, hydrogen, methyl, ethyl or phenyl and $R^2$, $R^3$ and $R^5$ to $R^8$ are each, independently of one another, hydrogen or methyl.

Particularly preferred guanidinium ions (IVv) are those in which
$R^1$ to $R^5$ are methyl, ethyl, 1-propyl, 2-propyl (iso-propyl), 1-butyl (n-butyl), 2-butyl (sec-butyl), 2-methyl-1-propyl (isobutyl), 2-methyl-2-propyl (tert-butyl), 1-pentyl, 2-pentyl, 3-pentyl, 2-methyl-1-butyl, 3-methyl-1-butyl, 2-methyl-2-butyl, 3-methyl-2-butyl, 2,2-dimethyl-1-propyl, 1-hexyl, 2-hexyl, 3-hexyl, 2-methyl-1-pentyl, 3-methyl-1-pentyl, 4-methyl-1-pentyl, 2-methyl-2-pentyl, 3-methyl-2-pentyl, 4-methyl-2-pentyl, 2-methyl-3-pentyl, 3-methyl-3-pentyl, 2,2-dimethyl-1-butyl, 2,3-dimethyl-1-butyl, 3,3-dimethyl-1-butyl, 2-ethyl-1-butyl, 2,3-dimethyl-2-butyl, 3,3-dimethyl-2-butyl, 1-heptyl, 1-octyl, 1-methylheptyl, 2-ethylhexyl, 2,4,4-trimethyl-pentyl, 1,1,3,3-tetramethylbutyl, 1-nonyl, 1-decyl, 1-undecyl, 1-dodecyl, 1-tridecyl, 1-tetradecyl, 1-pentadecyl, 1-hexadecyl, 1-heptadecyl, 1-octadecyl and n-eicosyl, in particular methyl, ethyl or 1-butyl. As very particularly preferred guanidinium ions (IVv), mention may be made of N,N,N',N' -tetrabutyl-N",N'-'dimethylguanidinium, N,N,N',N',N''-Pentabutyl-N''-methylguanidinium, N,N,N',N'-Tetrabutyl-N "-ethyl-N ''-methylguanidinium.

Particularly preferred cholinium ions (IVw) are those in which
$R^1$ and $R^2$ are each, independently of one another, methyl, ethyl, 1-butyl or 1-octyl and $R^3$ is hydrogen, methyl, ethyl, acetyl, $-SO_2OH$ or $-PO(OH)_2$, or
$R^1$ is methyl, ethyl, 1-butyl or 1-octyl, $R^2$ is a $-CH_2-CH_2-OR^4$ group and $R^3$ and $R^4$ are each, independently of one another, hydrogen, methyl, ethyl, acetyl, $-SO_2OH$ or $-PO(OH)_2$, or $R^1$ is a $-CH_2-CH_2-OR^4$ group, $R^2$ is a $-CH_2-CH_2-OR^5$ group and $R^3$ to $R^5$ are each, independently of one another, hydrogen, methyl, ethyl, acetyl, $-SO_2OH$ or $-PO(OH)_2$.

As cholinium ions (IVw), particular preference is given to those in which $R^3$ is selected from among hydrogen, methyl, ethyl, acetyl, 5-methoxy-3-oxapentyl, 8-methoxy-3,6-dioxaoctyl, 11-methoxy-3,6,9-trioxaundecyl, 7-methoxy-4-oxaheptyl, 11-methoxy-4,8-dioxaundecyl, 15-methoxy-4,8,12-trioxapentadecyl, 9-methoxy-5-oxanonyl, 14-methoxy-5,10-oxatetradecyl, 5-ethoxy-3-oxapentyl, 8-ethoxy-3,6-dioxaoctyl, 11-ethoxy-3,6,9-trioxaundecyl, 7-ethoxy-4-oxaheptyl, 11-ethoxy-4,8-dioxaundecyl, 15-ethoxy-4,8,12-trioxapentadecyl, 9-ethoxy-5-oxanonyl or 14-ethoxy-5,10-oxatetradecyl.

The cations (IV.x.1) are particularly preferably selected from among cations of 1,5-diazabicyclo[4.3.0]non-5-ene (DBN) and 1,8-diazabicyclo[5.4.0]undec-7-ene (DBU).

Particularly preferred phosphonium ions (IVy) are those in which
$R^1$ to $R^3$ are each, independently of one another aryl, $C_1$-$C_{18}$-alkyl for example methyl, ethyl, 1-propyl, 2-propyl (iso-propyl), 1-butyl (n-butyl), 2-butyl (sec-butyl), 2-methyl-1-propyl (isobutyl), 2-methyl-2-propyl (tert-butyl), 1-pentyl, 2-pentyl, 3-pentyl, 2-methyl-1-butyl, 3-methyl-1-butyl, 2-methyl-2-butyl, 3-methyl-2-butyl, 2,2-dimethyl-1-propyl, 1-hexyl, 2-hexyl, 3-hexyl, 2-methyl-1-pentyl, 3-methyl-1-pentyl, 4-methyl-1-pentyl, 2-methyl-2-pentyl, 3-methyl-2-pentyl, 4-methyl-2-pentyl, 2-methyl-3-pentyl, 3-methyl-3-pentyl, 2,2-dimethyl-1-butyl, 2,3-dimethyl-1-butyl, 3,3-dimethyl-1-butyl, 2-ethyl-1-butyl, 2,3-dimethyl-2-butyl, 3,3-dimethyl-2-butyl, 1-heptyl, 1-octyl, 1-methylheptyl, 2-ethylhexyl, 2,4,4-trimethyl-pentyl, 1,1,3,3-tetramethylbutyl, 1-nonyl, 1-decyl, 1-undecyl, 1-dodecyl, 1-tridecyl, 1-tetradecyl, 1-pentadecyl, 1-hexadecyl, 1-heptadecyl, 1-octadecyl, in particular methyl, ethyl, 1-propyl, 2-propyl (iso-propyl), 1-butyl (n-butyl), 2-butyl (sec-butyl), 2-methyl-1-propyl (isobutyl), 2-methyl-2-propyl (tert-butyl), , 1-hexyl, 2-ethylhexyl or 1-octyl, or phenyl which is unsubstituted or bears 1, 2, 3, 4 or 5 substituents selected independently from among $C_1$-$C_{18}$-alkyl, carboxylate, sulfonate, COOH and $SO_3H$. Examples for preferred phosphonium ions (IVy) are the ones in which R and $R^1$ to $R^3$ are independently from each other methyl, ethyl, 1-propyl, 2-propyl (iso-propyl), 1-butyl (n-butyl), 2-butyl (sec-butyl), 2-methyl-1-propyl (isobutyl), 2-methyl-2-propyl (tert-butyl), and very preferably phosphonium ions (IVy) are the ones in which three of the before mentioned radicals R and $R^1$ to $R^3$ are the same and the forth of said radicals is methyl, for example R = methyl and $R^1$ to $R^3$ = 1-butyl.

Particularly preferred sulfonium ions (IVz) are those in which
$R^1$ and $R^2$ are each, independently of one another, $C_1$-$C_{18}$-alkyl, in particular butyl, isobutyl, 1-hexyl or 1-octyl.

Among the abovementioned heterocyclic cations, the imidazolium ions, imidazolinium ions, pyrrolidinium ions, guanidinium ions and phosphonium ions are preferred.
Particular preference is given to the imidazolium ions.

Very preferably $[A]^+$cations are selected amongst the following groups (i) to (iv):

In the following described very preferred $[A^+]$ cations (i) to (iv) the substituent R has the following meaning:

R is hydrogen or preferably a $C_1$ to $C_{30}$ organic residue as defined above, which can be preferably selected from residues defined herein under groups (I) to (VIII) above, for example substituted or unsubstituted "alkyl", "arylalkyl", "alkenyl", "cycloalkyl", "cycloalkenyl", "polycyclyl", "aryl", "aryloxy", "arylthio", "heterocycloalkyl", "heteroarayl", very preferably a straight chain or branched $C_1$ to $C_{30}$ alkyl and most preferably unsubstituted straight chain or branched $C_1$ to $C_{30}$ "alkyl" residues as defined in group (I) above, for example methyl, ethyl, 1-propyl, 2-propyl (iso-propyl), 1-butyl (n-butyl), 2-butyl (sec-butyl), 2-methyl-1-propyl (isobutyl), 2-methyl-2-propyl (tert-butyl), 1-pentyl, 2-pentyl, 3-pentyl, 2-methyl-1-butyl, 3-methyl-1-butyl, 2-methyl-2-butyl, 3-methyl-2-butyl, 2,2-dimethyl-1-propyl, 1-hexyl, 2-hexyl, 3-hexyl, 2-methyl-1-pentyl, 3-methyl-1-pentyl, 4-methyl-1-pentyl, 2-methyl-2-pentyl, 3-methyl-2-pentyl, 4-methyl-2-pentyl, 2-methyl-3-pentyl, 3-methyl-3-pentyl, 2,2-dimethyl-1-butyl, 2,3-dimethyl-1-butyl, 3,3-dimethyl-1-butyl, 2-ethyl-1-butyl, 2,3-dimethyl-2-butyl, 3,3-dimethyl-2-butyl, 1-heptyl, 1-octyl, 1-methylheptyl, 2-ethylhexyl, 2,4,4-trimethyl-pentyl, 1,1,3,3-tetramethylbutyl, 1-nonyl, 1-decyl, 1-undecyl, 1-dodecyl, 1-tridecyl, 1-tetradecyl, 1-pentadecyl, 1-hexadecyl, 1-heptadecyl, 1-octadecyl and n-eicosyl.

(i) imidazolium ions (IVe) in which
$R^1$ is hydrogen, methyl, ethyl, 1-propyl, 1-butyl, 1-pentyl, 1-hexyl, 1-octyl, 2-ethylhexyl, 1-nonyl, 1-decyl, 1-dodecyl, 2-hydroxyethyl or 2-cyanoethyl and $R^2$ to $R^4$ are each, independently of one another, hydrogen, methyl or ethyl. Particularly useful imidazolium ions (IVe) are 1-methylimidazolium, 1-ethylimidazolium, 1-(1-propyl)imidazolium, 1-(1-allyl)imidazolium, 1-(1-butyl)imidazolium, 1-(1-octyl)imidazolium, 1-(1-dodecyl)imidazolium, 1-(1-tetradecyl)imidazolium, 1-(1-hexadecyl)imidazolium, 1,3-dimethylimidazolium, 1,3-diethylimidazolium, 1-ethyl-3-methylimidazolium, 1-(1-butyl)-3-methylimidazolium, 1-(1-butyl)-3-ethylimidazolium, 1-(1-hexyl)-3-methylimidazolium, 1-(1-hexyl)-3-ethylimidazolium, 1-(1-hexyl)-3-butylimidazolium, 1-(1-octyl)-3-methylimidazolium, 1-(1-octyl)-3-ethylimidazolium, 1-(1-octyl)-3-butylimidazolium, 1-(2-ethylhexyl)-3-methylimidazolium, 1-(1-decyl)-3-methylimidazolium, 1-(1-dodecyl)-3-methylimidazolium, 1-(1-dodecyl)-3-ethylimidazolium, 1-(1-dodecyl)-3-butylimidazolium, 1-(1-dodecyl)-3-octylimidazolium, 1-(1-tetradecyl)-3-methylimidazolium, 1-(1-tetradecyl)-3-ethylimidazolium, 1-(1-tetradecyl)-3-butylimidazolium, 1-(1-tetradecyl)-3-octylimidazolium, 1-(1-hexadecyl)-3-methylimidazolium, 1-(1-hexadecyl)-3-ethylimidazolium, 1-(1-hexadecyl)-3-butylimidazolium, 1-(1-hexadecyl)-3-octylimidazolium, 1,2-dimethylimidazolium, 1,2,3-trimethylimidazolium, 1-ethyl-2,3-dimethylimidazolium, 1-(1-butyl)-2,3-dimethylimidazolium, 1-(1-hexyl)-2,3-dimethylimidazolium, 1-(1-octyl)-2,3-dimethylimidazolium, 1,4-dimethylimidazolium, 1,3,4-trimethylimidazolium, 1,4-dimethyl-3-ethylimidazolium, 3-methylimidazolium, 3-ethylimidazolium, 3-n-propylimidazolium, 3-n-butylimidazolium, 1,4-dimethyl-3-octylimidazolium, 1,4,5-trimethylimidazolium, 1,3,4,5-tetramethylimidazolium, 1,4,5-trimethyl-3-ethylimidazolium, 1,4,5-trimethyl-3-butylimidazolium, 1,4,5-trimethyl-3-octylimidazolium, 1-(prop-1-en-3-yl)-3-methylimidazolium and 1-(prop-1-en-3-yl)-3-butylimidazolium. Especially suitable imidazolium ions (IVe) are 1,3-dimethylimidazolium, 1,3-diethylimidazolium, 1-ethyl-3-methylimidazolium, 1-(1-butyl)-3-methylimidazolium, 1-(2-ethylhexyl)-3-methylimidazolium, 1-(1-decyl)-3-methylimidazolium, 1-(1-dodecyl)-3-methylimidazolium.

(ii) pyrrolidinium ions (IVs) in which
$R^1$ is hydrogen, methyl, ethyl, propyl, butyl or phenyl and $R^2$ to $R^9$ are each, independently of one another, hydrogen or methyl. Examples for preferred pyrrolidinium ions (IVs) are the ones in which $R^1$ and R are independently from each other methyl, ethyl, 1-propyl, 2-propyl (iso-propyl), 1-butyl (n-butyl), 2-butyl (sec-butyl), 2-methyl-1-propyl (isobutyl), 2-methyl-2-propyl (tert-butyl), and $R^2$ to $R^9$ are each hydrogen, for example $R^1$ = 1-butyl, R = methyl and $R^2$ to $R^9$ are each hydrogen.

(iii) guanidinium ions (IVv) in which $R^1$ to $R^5$ are methyl, ethyl or butyl. As very particularly preferred guanidinium ions (IVv), mention may be made of N,N,N',N' -tetrabutyl-N",N'-'dimethylguanidinium, N,N,N',N',N"-Pentabutyl-N"-methylguanidinium, N,N,N',N'-Tetrabutyl-N"-ethyl-N"-methylguanidinium

(iv) phosphonium ions (IVy) in which $R^1$ to $R^3$ are each, independently of one another aryl, $C_1$-$C_{18}$-alkyl, for example methyl, ethyl, 1-propyl, 2-propyl (iso-propyl), 1-butyl (n-butyl), 2-butyl (sec-butyl), 2-methyl-1-propyl (isobutyl), 2-methyl-2-propyl (tert-butyl), 1-pentyl, 2-pentyl, 3-pentyl, 2-methyl-1-butyl, 3-methyl-1-butyl, 2-methyl-2-butyl, 3-methyl-2-butyl, 2,2-dimethyl-1-propyl, 1-hexyl, 2-hexyl, 3-hexyl, 2-methyl-1-pentyl, 3-methyl-1-pentyl, 4-methyl-1-pentyl, 2-methyl-2-pentyl, 3-methyl-2-pentyl, 4-methyl-2-pentyl, 2-methyl-3-pentyl, 3-methyl-3-pentyl, 2,2-dimethyl-1-butyl, 2,3-dimethyl-1-butyl, 3,3-dimethyl-1-butyl, 2-ethyl-1-butyl, 2,3-dimethyl-2-butyl, 3,3-dimethyl-2-butyl, 1-heptyl, 1-octyl, 1-methylheptyl, 2-ethylhexyl, 2,4,4-trimethyl-pentyl, 1,1,3,3-tetramethylbutyl, 1-nonyl, 1-decyl, 1-undecyl, 1-dodecyl, 1-tridecyl, 1-tetradecyl, 1-pentadecyl, 1-hexadecyl, 1-heptadecyl, 1-octadecyl, in particular methyl, ethyl, 1-propyl, 2-propyl (iso-propyl), 1-butyl (n-butyl), 2-butyl (sec-butyl), 2-methyl-1-propyl (isobutyl), 2-methyl-2-propyl (tert-butyl), , 1-hexyl, 2-ethylhexyl or 1-octyl, or phenyl which is unsubstituted or bears 1, 2, 3, 4 or 5 substituents selected independently from among $C_1$-$C_{18}$-alkyl, carboxylate, sulfonate, COOH and $SO_3H$. Examples for preferred phosphonium ions (IVy) are the ones in which R and $R^1$ to $R^3$ are independently from each other methyl, ethyl, 1-propyl, 2-propyl (iso-propyl), 1-butyl (n-butyl), 2-butyl (sec-butyl), 2-methyl-1-propyl (isobutyl), 2-methyl-2-propyl (tert-butyl), and very preferably phosphonium ions (IVy) are the ones in which three of the before mentioned radicals R and $R^1$ and $R^3$ are the same and the forth of said radicals is methyl, for example R = methyl and $R^1$ to $R^3$ = 1-butyl.

X in general formula (I) is the group $-NR^aR^b$, wherein $R^a$ and $R^b$ the same or different are independently from each other hydrogen and the following organic residues with the proviso that at least one of $R^a$ and $R^b$ and preferably both $R^a$ and $R^b$ of the following organic residues are a $C_1$ to $C_{30}$ organic residue as defined above, which can be preferably selected from residues defined herein under (I) to (X) above, preferably substituted or unsubstituted residues of the following: "alkyl", "", "arylalkyl", "alkenyl", "cycloalkyl", "cycloalkenyl", "polycyclyl", "aryl", "aryloxy", "arylthio", "heterocycloalkyl","heteroarayl", all as defined herein.

In general it is advisable that each $R^a$ and $R^b$ in the group $-NR^aR^b$ of X in general formula (I), independently from each other, or preferably both the same, are a $C_4$ to $C_{16}$ organic residue, preferably a $C_7$ to $C_{16}$ organic residue, very preferably a $C_8$ to $C_{12}$ organic residue wherein said organic residues are for example the respective carbon-number selection in the following groups: "alkyl", arylalkyl", "cycloalkyl", "aryl", "aryloxy", all as defined herein or mentioned in the following paragraphs under "first mode" or "second mode".

In a "first mode" the radical $-NR^aR^b$ has the residues $R^a$ = H and $R^b$ = substituted or unsubstituted residues of the following: "alkyl", arylalkyl", "cycloalkyl", "aryl", "aryloxy", all as defined herein, most preferably $R^b$ is a straight chain or branched $C_1$ to $C_{30}$ alkyl and most preferably unsubstituted "aryl" or straight chain or branched $C_1$ to $C_{30}$ "alkyl" residues as defined in group (I) above, for example phenyl, alkylsubstituted phenyl such as mono- di- or trimethylsubstituted phenyl such as mesityl, further the following straight chain or branched $C_1$ to $C_{30}$ "alkyl" residues methyl, ethyl, 1-propyl, 2-propyl (iso-propyl), 1-butyl (n-butyl), 2-butyl (sec-butyl), 2-methyl-1-propyl (isobutyl), 2-methyl-2-propyl (tert-butyl), 1-pentyl, 2-pentyl, 3-pentyl, 2-methyl-1-butyl, 3-methyl-1-butyl, 2-methyl-2-butyl, 3-methyl-2-butyl, 2,2-dimethyl-1-propyl, 1-hexyl, 2-hexyl, 3-hexyl, 2-methyl-1-pentyl, 3-methyl-1-pentyl, 4-methyl-1-pentyl, 2-methyl-2-pentyl, 3-methyl-2-pentyl, 4-methyl-2-pentyl, 2-methyl-3-pentyl, 3-methyl-3-pentyl, 2,2-dimethyl-1-butyl, 2,3-dimethyl-1-butyl, 3,3-dimethyl-1-butyl, 2-ethyl-1-butyl, 2,3-dimethyl-2-butyl, 3,3-dimethyl-2-butyl, 1-heptyl, 1-octyl, 1-methylheptyl, 2-ethylhexyl, 2,4,4-trimethyl-pentyl, 1,1,3,3-tetramethylbutyl, 1-nonyl, 1-decyl, 1-undecyl, 1-dodecyl, 1-tridecyl, 1-tetradecyl, 1-pentadecyl, 1-hexadecyl, 1-heptadecyl, 1-octadecyl and n-eicosyl.

Examples of the radical $-NR^aR^b$ are -N(H) $R^b$ with $R^b$= methyl, ethyl, 1-propyl, 2-propyl (iso-propyl), 1-butyl (n-butyl), 2-butyl (sec-butyl), 2-methyl-1-propyl (isobutyl), 2-methyl-2-propyl (tert-butyl), 1-pentyl, 1-hexyl, 1-heptyl, 1-octyl, , 2-ethylhexyl, phenyl, mono- di- or trimethylsubstituted phenyl such as mesityl.

In a preferable "second mode" in the radical $-NR^aR^b$ both residues $R^a$ and $R^b$ independently from each other are substituted or unsubstituted residues of the following: "alkyl", arylalkyl", "cycloalkyl", "aryl", "aryloxy", all as defined herein, most preferably Rb is a straight chain or branched $C_1$ to $C_{30}$ alkyl and most preferably unsubstituted "aryl" or straight chain or branched $C_1$ to $C_{30}$ "alkyl" residues as defined in group (I) above, for example phenyl, alkylsubstituted phenyl such as mono- di- or trimethylsubstituted phenyl such as mesityl, further the following straight chain or branched $C_1$ to $C_{30}$ "alkyl" residues methyl, ethyl, 1-propyl, 2-propyl (iso-propyl), 1-butyl (n-butyl), 2-butyl (sec-butyl), 2-methyl-1-propyl (isobutyl), 2-methyl-2-propyl (tert-butyl), 1-pentyl, 2-pentyl, 3-pentyl, 2-methyl-1-butyl, 3-methyl-1-butyl, 2-methyl-2-butyl, 3-methyl-2-butyl, 2,2-dimethyl-1-propyl, 1-hexyl, 2-hexyl, 3-hexyl, 2-methyl-1-pentyl, 3-methyl-1-pentyl, 4-methyl-1-pentyl, 2-methyl-2-pentyl, 3-methyl-2-pentyl, 4-methyl-2-pentyl, 2-methyl-3-pentyl, 3-methyl-3-pentyl, 2,2-dimethyl-1-butyl, 2,3-dimethyl-1-butyl, 3,3-dimethyl-1-butyl, 2-ethyl-1-butyl, 2,3-dimethyl-2-butyl, 3,3-dimethyl-2-butyl, 1-heptyl, 1-octyl, 1-methylheptyl, 2-ethylhexyl, 2,4,4-trimethyl-pentyl, 1,1,3,3-tetramethylbutyl, 1-nonyl, 1-decyl, 1-undecyl, 1-dodecyl, 1-tridecyl, 1-tetradecyl, 1-pentadecyl, 1-hexadecyl, 1-heptadecyl, 1-octadecyl and n-eicosyl.
Examples of the radical $-NR^aR^b$ wherein $R^a$ and $R^b$ being different or preferably the same are methyl, ethyl, 1-propyl, 2-propyl (iso-propyl), 1-butyl (n-butyl), 2-butyl (sec-butyl), 2-methyl-1-propyl (isobutyl), 2-methyl-2-propyl (tert-butyl), 1-pentyl, 1-hexyl, 1-heptyl, 1-octyl, 2-ethylhexyl, phenyl, mono- di- or trimethylsubstituted phenyl such as mesityl.

Examples for suitable monosubstituted oxalic acid derivatives of the general formula (I) are those wherein A is selcted from the group consisting of the above defined compounds IV.e (imidazolium), IV.s (pyrrolidinium), IV.v (guanidinium), IV.y (phosphonium) and X is selected from the above defined first mode and second mode.

Examples for very suitable monosubstituted oxalic acid derivatives of the general formula (I) are those wherein A is selcted from the group consisting of the above defined compounds IV.e (imidazolium), IV.s (pyrrolidinium), and IV.y (phosphonium) and X is selected from the above defined first mode and preferable second mode, in particular respective $R^a$ and $R^b$ having $C_4$ to $C_{16}$ organic residues, preferably $C_7$ to $C_{16}$ organic residues, very preferably $C_8$ to $C_{12}$ organic residues.

Examples for preferred monosubstituted oxalic acid derivatives of the general formula (I) are the following:

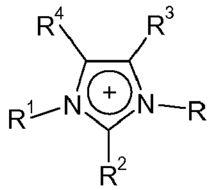

(IV.e) + $[O\text{-}C(O)\text{-}C(O)\text{-}NR^aR^b]^-$

having the following residues:

| R | $R^1$ | $R^2$ to $R^4$ | $R^a$ | $R^b$ |
|---|---|---|---|---|
| methyl | 1-butyl | hydrogen | hydrogen | tert-butyl |
| methyl | 1-butyl | hydrogen | hydrogen | phenyl |
| methyl | 1-butyl | hydrogen | hydrogen | mesityl |
| methyl | 1-butyl | hydrogen | 1-hexyl | 1-hexyl |
| methyl | 1-butyl | hydrogen | 1-octyl | 1-octyl |
| methyl | 1-butyl | hydrogen | 2-ethylhexyl | 2-ethylhexyl |
| methyl | methyl | hydrogen | methyl | methyl |
| methyl | methyl | hydrogen | ethyl | ethyl |
| methyl | methyl | hydrogen | ethyl | 1-butyl |
| methyl | methyl | hydrogen | 1-butyl | 1-butyl |
| methyl | methyl | hydrogen | 1-octyl | 1-octyl |
| methyl | methyl | hydrogen | 2-ethylhexyl | 2-ethylhexyl |
| methyl | 1-decyl | hydrogen | 1-octyl | 1-octyl |
| methyl | 2-ethylhexyl | hydrogen | 2-ethylhexyl | 2-ethylhexyl |
| methyl | 1-decyl | hydrogen | 2-ethylhexyl | 2-ethylhexyl |
| methyl | 1-dodecyl | hydrogen | 2-ethylhexyl | 2-ethylhexyl |

and/or

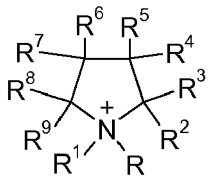

(IV.s) + [O-C(O)-C(O)-$NR^aR^b$]$^-$

having the following residues:

| R | $R^1$ | $R^2$ to $R^9$ | $R^a$ | $R^b$ |
|---|---|---|---|---|
| methyl | 1-butyl | hydrogen | hydrogen | phenyl |
| methyl | 1-butyl | hydrogen | 2-propyl | 2-propyl |
| methyl | 1-butyl | hydrogen | ethyl | 1-butyl |
| methyl | 1-butyl | hydrogen | 1-hexyl | 1-hexyl |
| methyl | 1-butyl | hydrogen | 1-octyl | 1-octyl |
| methyl | 1-butyl | hydrogen | 2-ethylhexyl | 2-ethylhexyl |

and/or

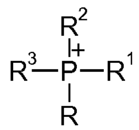

(IV.y) + [O-C(O)-C(O)-$NR^aR^b$]

having the following residues:

| R | $R^1$ | $R^2$ to $R^3$ | $R^a$ | $R^b$ |
|---|---|---|---|---|
| methyl | 1-butyl | 1-butyl | Hydrogen | 2-ethylhexyl |
| methyl | 1-butyl | 1-butyl | 2-ethylhexyl | 2-ethylhexyl |

Monosubstituted oxalic acid derivatives of the general formula (I) are preferably in the state of an ionic liquid.

For the purpose of the present patent application ionic liquids are organic salts which are liquid at temperatures below 180 °C, preferably below 130 °C. In general the melting points of the oxalic acid derivatives of the general formula (I) are in a range of from - 50 °C to 180 °C, more preferably in the range of from - 20 °C to 130 °C.

The oxalic acid derivatives of the general formula (I) are prepared by the reaction of an oxalic acid compound of the general formula (II)

$R^1$O-C(O)-C(O)-X (II)

with the organic moiety A as defined above, however wherein the heteroatom in the precursor of A is not yet formally positively charged by binding to the residue $R^1$.

$R^1$ in formula (II) has the meaning of R as defined above, including all modes, with the proviso that $R^1$ in formula (II) is not hydrogen. X has the meaning as defined above, including all modes. Preferably $R^1$ in formula (II) is a straight chain or branched $C_1$ to $C_{30}$ alkyl and most preferably unsubstituted straight chain or branched $C_1$ to $C_{30}$ "alkyl" residues as defined in group (I) above, for example methyl, ethyl, propyl, butyl, pentyl, hexyl, heptyl, octyl, nonyl, decyl, undecyl, dodecyl; very preferably X is methyl, ethyl, propyl, butyl, dodecyl; most preferably $R^1$ in formula (II) is methyl, ethyl,

propyl, in particular $R^1$ in formula (II) is methyl.

Usually the oxalic acid compound of the general formula (II) is obtained by reaction of oxalic acid dichloride (Cl-C(O)-C(O)-Cl) with about one molar equivalent of the respective alcohol $R^1OH$, wherein $R^1$ has the meaning as defined for general formula (II), including all modes. This reaction usually leads to the $R^1O$-monosubstituted oxalic acid monochloride ($R^1O$-C(O)-C(O)-Cl) which is then usually reacted with about one molar equivalent of an organic amine compound of the general formula $HNR^aR^b$ wherein $R^aR^b$ have the same meaning as defined in general formula (I), including all modes.

Usually the above mentioned reactions for forming the compound (II) or its precursor are conducted under usual conditions of organic preparative chemistry, for example by using solvents such as ether compounds, e.g. diethyl ether, and at temperatures for example in the range of - 30 to 100 °C, or -15 to 50 °C.

Examples for oxalic acid derivatives of the general formula (II) as well as their related precursors are described in the section "Examples" of this application.

The monosubstituted oxalic acid derivatives of the general formula (I), preferably those which are in the state of an ionic liquid, can be used in the presence or absence of further solvents, preferably organic aprotic solvents, prefereably those as defined below, to at least partially dissolve inorganic or organic material, for example metal compounds such as compounds of rare earth elements or actinide elements.

Suitable solvents are all solvents, preferably organic solvents, in which the compound (I) is soluble at least at room temperature (about 20 °C) and normal pressure (1 bar (abs.)), which solvents when mixed with water form a two phase system by phase separation water/solvent and which solvents do basically not react chemically with the following compound or compounds by decomposing it: compound (I) and/or the inorganic or organic material to be dissolved by compound (I) and/or any composition of compound (I) with the inorganic or organic material. Said solvents may be the pure solvent or any mixture of those.

Suitable solvents are for example: (i) Aliphatic straight chain or branched $C_1$ to $C_{30}$-hydrocarbons such as alkanes for example methane, ethane, propane, butane, 1-pentane and its isomers, 1-hexane and its isomers, 1-heptane and its isomers, 1-octane and its isomers, 1-nonane and its isomers, 1-decane and its isomers, petrol ether, kerosin (ii) substituted and non-substituted $C_4$ to $C_{10}$ cyclic hydrocarbons such as cycloalkanes for example cyclobutane, cyclopentane, cyclohexane, cycloheptane, (iii) substituted and non-substituted $C_6$ to $C_{20}$ aromatic hydro carbons such as aliphatic straight chain or branched $C_1$ to $C_{30}$-alkyl mono substituted or multiply substituted aromatic hydrocarbons, for example benzene, naphthalene, mono-, di or tri-$C_1$ to $C_6$-alkyl substituted benzene, like toluene, xylene, ethyl benzene, (iv) halogenanted hydrocarbons such as halogented aliphatic straight chain or branched $C_1$ to $C_{30}$-hydrocarbons or halogenated $C_4$ to $C_{10}$ cyclic hydrocarbons, for example monochlor methane, dichlor methane, trichlor methane, terachlor methane, monochlor ethane 1,2-dichlor ethane and its isomers, 1,1,1-trichlor ethane and its isomers; (v) aliphatic straight chain or branched $C_1$ to $C_{30}$, $C_6$ to $C_{20}$ aromatic or $C_4$ to $C_{10}$ cycloaliphatic symmetrically and non-symmetrically ethers, such as diethylether, tert-butyl-methyl ether, tetrahydrofuran. Mixtures of those solvents are explicitely included.

Suitable inorganic material are metal compunds, such as transition metal compounds, for example Zn, Sn, Au, Ag, compounds of the rare earth elements or of the actinide elements.

Very suitable inorganic compounds to be dissolved by the compounds of formula (I) are compounds of the rare earth elements of the periodic table of elements, namely Sc, Y, La, Ce, Pr, Nd, Pm, Sm, Eu, Gd, Tb, Dy, Ho, Er, Tm Yb, Lu, or compounds of the actinide elements of the periodic table of elements, in particular Ac, Th, Pa, U, Np, Pu, Am, Bk, Cf. Suitable compounds of the rare earth elements, preferably in their formal oxidation state +III or actinide elements, preferably in their formal oxidation state +III or +IV, are oxides, sulfates or any other salts of inorganic or organic acids, preferably nitrates or halogenides, in particular chlorides.

Usually the compound of the rare earth element or actinide element as described above is combined - with or without the above mentioned organic solvent - with the monosubstituted oxalic acid derivatives of the general formula (I) usually at a temperature in the range of -20 °C to 150 °C in order to dissolve the compounds of the rare earth element or actinide element.

The monosubstituted oxalic acid derivatives of the general formula (I), for example those which are in the state of an ionic liquid, can be used in the absence or preferably in the presence of further solvents, preferably organic aprotic solvents, very preferably those as defined above, in a separation process for metals, preferably in a separation process for rare earth elements of the periodic table of elements or in a separation process for actinide elements of the periodic table of elements. Said separation process for metals, preferably for rare earth elements of the periodic table of elements or for actinide elements of the periodic table of elements is for example a liquid-liquid extraction processes, preferably used for the separation of metals, for example the separation of rare earth elements from each other or the separation of rare earth elements from actinide elements or the separation of actinide elements from each other or the separation of rare earth elements and/or actinide elements from other metals.

The separation process for metals, preferably for rare earth elements of the periodic table of the elements or for actinide elements of the periodic table of the elements, preferably the liquid-liquid extraction process is usually conducted at temperatures in the range of -20 °C to 150 °C, preferably 10°C to 80 °C, for example 20 °C to 60 °C.

The separation process for metals, preferably for rare earth elements of the periodic table of the elements or for actinide elements of the periodic table of the elements, preferably the liquid-liquid extraction process may in general be conducted batchwise or continously.

Usually for the separation process for metals, preferably for rare earth elements of the periodic table of the elements or for actinide elements of the periodic table of the elements, preferably the liquid-liquid extraction process, compounds of the rare earth elements, preferably those as defined above, for example the chlorides or compounds of the actinide elements, preferably those as defined above, for example the chlorides are dissolved or dispersed in a water containing solvent, preferably pure water. Preferably the pH value at a temperature of about 20 to 60 °C of said solution or dispersion should not be lower than 1,0, preferably said pH value should be in the range of 1 to 6, very preferably said pH value should be in the range of 1 to 4 and most preferably said pH value should be in the range of 1,0 to 1,2.

A preferred separation process for rare earth elements of the periodic table of elements and actinide elements of the periodic table of elements, is the liquid-liquid extraction, for example the one as described as follows and to which the parameters of the above described general separation process parameters apply as well.

Usually compounds of the rare earth elements, preferably those as defined above, for example the chlorides or compounds of the actinide elements, preferably those as defined above, for example the chlorides are dissolved or dispersed in a water containing solvent, preferably pure water. Preferably the pH value at a temperature of about 20 to 60 °C of said solution or dispersion should not be lower than 1,0, preferably said pH value should be in the range of 1 to 6, very preferably said pH value should be in the range of 1 to 4 and most preferably said pH value should be in the range of 1,0 to 1,2. This may be achieved by adding a suitable acid, prefereably the complementary inorganic acid, such as hydrochloric acid for chlorides or nitric acid for nitates and so on.

The monosubstituted oxalic acid derivatives of the general formula (I) as defined above including all modes, preferably the ones of formulae (IV.e), (IV.s), (IV.v) or (IV.y), in particular the ones bearing the anion [O-C(O)-C(O)-N$R^aR^b$] with $R^a$ and $R^b$ both being 2-Ethylhexyl, are dissolved or dispersed in an organic solvent as defined above which by mixing with the solution or dispersion of the rare earth element compounds or actinide element compounds, preferably water, forms a liquid-liquid two phase system, for example two liquids in separate layers. Said organic solvents are preferably chlorinated hydrocarbons, for example dichloromethane or even more preferred substituted or unsubstituted aromatic hydrocarbons, for example toluene. The two solutions are mixed and then the two liquid phases are usually allowed to separate, This process may be repeated once or more. The liquid-liquid separation process may be conducted batchwise or continously.

The described separation process is very suitable to separate compounds of the rare earth elements of the periodic table of elements, namely Sc, Y, La, Ce, Pr, Nd, Pm, Sm, Eu, Gd, Tb, Dy, Ho, Er, Tm Yb, Lu, from each other or to separate compounds of the actinide elements of the periodic table of elements, in particular Ac, Th, Pa, U, Np, Pu, Am, Bk, Cf from each other or from said rare earth metals. Suitable compounds of the rare earth elements, preferably in their formal oxidation state +III or actinides, preferably in their formal oxidation state +III or +IV, are oxides, sulfates or any other salts of inorganic or organic acids, preferably nitrates or halogenides, in particular chlorides.

Examples

Abbreviations and definitions and general remarks

"ICP-MS" means "inductively coupled plasma mass spectrometry".

This is a very suitable method for quantitative and qualitative detection of e.g. the amounts of rare earth elements in the respective aqueous or non-aqueous phase.

"IL" means a compound as defined in formula (I).

"Extraction efficiency" means the mol-percentage fraction (calculation based on the original molar amount of inorganic compound) of respective inorganic compound, for example rare earth compound, which was transferred from one phase, for example the aqueous phase, to the other phase, for example organic solvent phase.

"Separation Factor" is shown in tables I to III and is an indicator for the separation of e.g. a rare earth (RE) metal pair into its rare earth metal components and is the ratio of the two respective distribution coeffcients (D) of the respective rare earth metal ion in the organic phase versus aqueous phase. It is calculated as follows:

$$\text{Separation Factor} = D(RE_{\text{horizontal}}) / D(RE_{\text{vertical}})$$

$$D = D(RE_{\text{organic phase}}) / D(RE_{\text{aqueous}})$$

wherein $D(RE_{\text{organique phase}})$ is calculated as set forth for the extraction efficiency above

For example: In Example 26, Table I the Separation Factor for the pair Ce (horizontal) and La (vertical) is 1,45. This means that the 1,45 fold molar amount of Ce in comparison to La is extracted from the aqueous phase to the organic (non-aqueous) phase.

$REE^{3+}$ = rare earth element in its formal oxidation state 3+

Percentage values are mol-% if not expressly stated otherwise.

A) Synthesis of precursors for the ionic monosubstituted oxalic acid derivatives (I)

Example 1

Synthesis of monomethyl oxalyl chloride

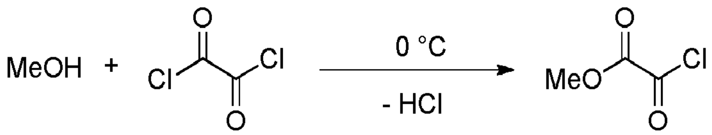


Oxalyl chloride (79.64 g, 627.5 mmol, 1.05 eq) was dissolved in diethylether (40 mL) and carefully mixed at 0°C with a solution of methanol (19.15 g, 597.6 mmol, 1.00 eq) in diethylether (50 mL). The reaction mixture was stirred at 0°C for 2h and the crude product purified by distillation (bp 116°C). Monomethyl oxalyl chloride was isolated in 93% yield (68.05 g, 555.5 mmol). $^{1}$H-NMR (300 MHz, MeCN-$d^3$): $\delta$ / ppm = 3.93 (s, 3H, COOC*H*$_3$). $^{13}$C-NMR (75 MHz, MeCN-$d^3$): $\delta$/ ppm = 162.4 (COCl), 156.8 (*C*OOCH$_3$), 56.0 (COOCH$_3$).

Example 2

Synthesis of methyl butylamino oxamate

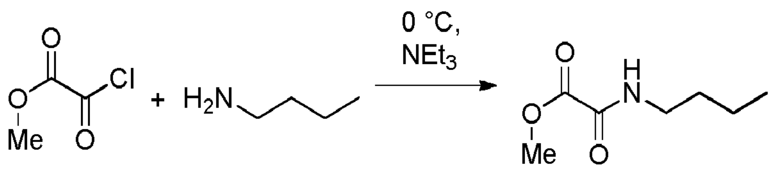


1.04 g (8.50 mmol, 1.00 eq) monomethyl oxalyl chloride was dissolved in diethyl ether (10 mL) and the solution was cooled to 0 °C. A solution of 1.55 g (21.20 mmol, 2.50 eq) n-butyl amine in diethyl ether (10 mL) was added slowly and the reaction mixture ws stirred at room temperature for 24 h. The reaction mixture was washed with sulphuric acid (5% in water, 50 mL), aqueous $Na_2CO_3$ solution (50 mL) and water (50 mL), the organic layer was dried over $Na_2SO_4$ and the solvent was removed in vacuo. The product was isolated as colourless solid (0.84 g, 5.28 mmol, 62 %).
1 H-NMR (300 MHz, MeCN-d3): $\delta$ / ppm = 7.59 (s, 1 H, CONH), 3.79 (s, 3H, OCH3), 3.22 (dd, 2H, 3JHH = 13.6 Hz, 6.7 Hz, CONCH2CH2CH2CH3), 1.59 - 1.41 (m, 2H, CONCH2CH2CH2CH3), 1.40 1.21 (m, 2H, CONCH2CH2CH2CH3), 0.90 (t, 3H, 3JHH = 7.3 Hz, CONCH2CH2CH2CH3).
13C-NMR (75 MHz, MeCN-d3): $\delta$ / ppm = 162.3 (COOCH3), 157.7 (CONH), 53.7 (COOCH3), 40.0 (CONCH2(CH2)2CH3), 31.9 (CONCH2CH2CH2CH3), 20.7 (CONCH2CH2CH2CH3), 14.0 (CONCH2CH2CH2CH3).
IR: $\tilde{v}$ / cm 1 = 3294 (m), 2956 (m), 2931 (m), 2872 (w), 1739 (m), 1685 (s), 1648 (s), 1525 (s), 1457 (s), 1437 (s), 1368 (m), 1295 (s), 1260 (s), 1231 (s), 1214 (s), 1180 (m), 1146 (m), 1093 (m), 1021 (m), 985 (w), 940 (w), 872 (s), 796 (s), 757 (s), 738 (s), 553 (m), 481 (w), 464 (w), 437 (w).
ESI-MS (MeOH): pos.: m/z (%) = 182.1 (100) [C7H13NO3Na]+.

HR-MS (MeOH): pos.: m/z = gef. 182.0787, ber. 182.0788 [C7H13NO3Na]+.

Example 3

Synthesis of methyl phenylamino oxamate

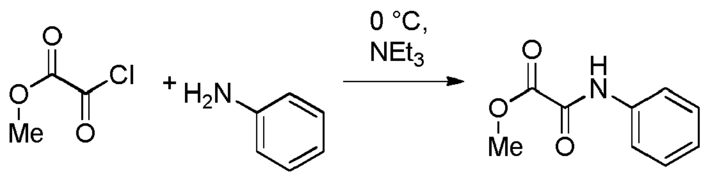


A solution of aniline (0.91 g, 9.70 mmol, 1.00 eq) and $NEt_3$ (0.99 g, 9.70 mmol, 1.00 eq) in diethyl ether (10 mL) was added to a solution of monomethyl oxalyl chloride (1.52 g, 10.20 mmol, 1.05 eq) in diethyl ether (20 mL) at 0 °C. The reaction mixture was stirred for 72 h at room temperature and subsequently washed with sulphuric acid (5% in water, 50 mL), aqueous $Na_2CO_3$ solution (50 mL) and water (50 mL). The organic layer was dried over $Na_2SO_4$ and the solvent was removed in vacuo. The product was isolated as colourless solid (1.08 g, 6.03 mmol, 62 %). 1 H-NMR (300 MHz, MeCN-d3): $\delta$ / ppm = 9.32 (s, 1 H, CONH), 7.68 (d, 2H, 3JHH = 8.1 Hz, Hortho), 7.38 (t, 2H, 3JHH = 7.8 Hz, Hmeta), 7.19 (t, 1H, 3JHH = 7.9 Hz, Hpara), 3.87 (s, 3H, COOCH3).
13C-NMR (75 MHz, MeCN-d3): $\delta$ / ppm = 162.0 (COOCH3), 155.5 (CON), 138.1 (CONCquart), 129.9 (Cortho) 126.2 (Cpara), 121.4 (Cmeta), 54.2 (COOCH3).
IR: $\tilde{v}$ / cm 1 = 3338 (w), 3303 (w), 2961 (w), 1695 (s), 1596 (m), 1537 (m), 1496 (m), 1440 (m), 1290 (s), 1260 (m), 1236 (m), 1197 (m), 1169 (m), 1076 (m), 1016 (m), 980 (m), 915 (w), 900 (w), 841 (w), 798 (m), 786 (m), 757 (s), 707 (s), 687 (s), 578 (w), 546 (m), 517 (m), 484 (s). ESI-MS (MeOH): pos.: m/z (%) = 202.0 (100) [C9H9NO3Na]+.
HR-MS (MeOH): pos.: m/z = gef. 202.0475, ber. 202.0475 [C9H9NO3Na]+.

Example 4

Synthesis of methyl 2-ethylhexylamino oxamate

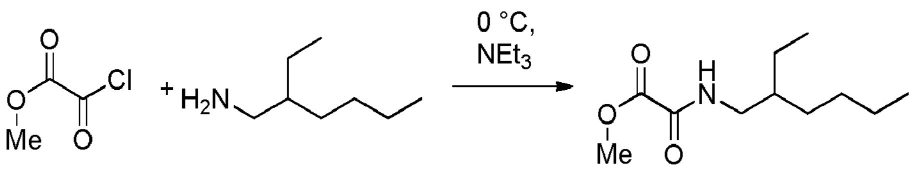


67.04 g (547.1 mmol, 1.07 eq) monomethyl oxalyl chloride were dissolved in diethylether (250 mL), cooled to 0°C and a solution of 2-ethyl hexyl amine (66.00 g, 511.0 mmol, 1.00 eq) and $NEt_3$ (62.86 g, 620.9 mmol, 1.20 eq) in diethyl ether (150 mL) was slowly added over a period of 3 h. The reaction mixture was stirred for 2 h at room temperature. The precipitated solid was removed by filtration and washed with diethyl ether. The solid ($NEt_3HCl$) was dissolved in water, extracted with diethyl ether (3x) and the combined organic layers were dried over $Na_2SO_4$. After filtration and removal of the solvent in vacuo, the product was isolated by distillation (5 • 10-1 mbar, 105 °C) as colourless resin (31.46 g, 145.9 mmol, 29 %).
1 H-NMR (300 MHz, MeCN-d3): $\delta$ / ppm = 7.56 (s, 1 H, CONH CH2CH(CH2CH3)(CH2)3CH3), 3.79 (s, 3H, COOCH3), 3.20 - 3.13 (m, 2H, CONHCH2CH(CH2CH3)(CH2)3CH3), 1.57 - 1.45 (m, 1 H, CONHCH2CH(CH2CH3)(CH2)3CH3), 1.36 - 1.19 (m, 8H, CONCH2CH(CH2CH3)-(CH2)3CH3), 0.94 - 0.81 (m, 6H, CONCH2CH(CH2CH3)(CH2)3CH3).
13C-NMR (75 MHz, MeCN-d3): $\delta$ / ppm = 162.2 (COOCH3), 157.7 (CON), 53.6 (COOCH3), 43.3 (CONHCH2CH(CH2CH3)(CH2)3CH3), 40.0 (CONHCH2CH(CH2CH3)(CH2)3CH3), 31.5 (CONHCH2CH(CH2CH3)(CH2)3CH3), 29.4 (CONHCH2CH(CH2CH3)CH2(CH2)2CH3), 24.7 (CONHCH2CH(CH2CH3)CH2CH2CH2CH3), 23.6 (CONHCH2CH(CH2CH3)(CH2)2CH2CH3), 14.3 (CONHCH2CH(CH2CH3)(CH2)3CH3), 11.0 (CONCH2CH(CH2CH3)(CH2)3CH3).

Example 5

Synthesis of methyl dimethylamino oxamate

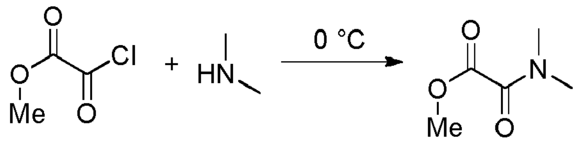


Dimethyl amine (103.9 g, 2.30 mol, 2.5 eq) was dissolved in diethyl ether (150 mL) and the solution was cooled to 0 °C. A solution of monomethyl oxalyl chloride (114.7 g, 0.94 mol, 1.0 eq) in diethylether (200 mL) was added slowly. The precipitate was filtered off and the filtrate was concentrated in vacuo. The product was isolated as yellowish oil (65.6 g, 0.50 mmol, 53 %) upon distillation (bp.: 110 °C, 4.3 mbar).
1 H-NMR (300 MHz, MeCN-d3): δ / ppm = 3.81 (s, 3H, COOCH3), 2.93, 2.88 (s, 2H, CON(CH3)2).
13C-NMR (75 MHz, MeCN-d3): δ / ppm = 165.0 (COOCH3), 162.6 (CON), 53.2 (COOCH3),37.3, 33.9 (CON(CH3)2).
ESI-MS (MeOH): pos.: m/z (%) = 154.6 (60) [C5H9NO3Na]+,
pos.: m/z (%) = 285.1 (15) [C10H18N2O6Na]+.
HR-MS (MeOH): pos.: m/z = gef. 154.0476, ber. 154.0475 [C5H9NO3Na]+,
pos.: m/z = gef. 285.1056, ber. 285.1057 [C10H18N2O6Na]+.

Example 6

Synthesis of methyl dihexylamino oxamate

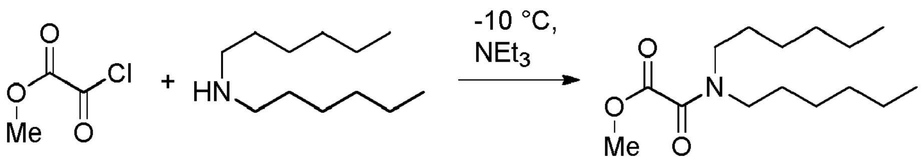


Dihexyl amine (2.88 g, 15.51 mmol, 1.00 eq) and $NEt_3$ (1.98 g, 19.59 mmol, 1.25 eq) were dissolved in diethyl ether (50 mL) and cooled to -10 °C. Monomethyl oxalyl chloride (2.00 g, 16.33 mmol, 1.05 eq), dissolved in diethyl ether (50 mL), was added slowly. The reaction mixture was stirred for additional 2 h at room temperature, the formed precipitate was filtered off and the resulting crude product was purified by distillation (131 °C, 6.4 • 10-2 mbar). The product was isolated as yellowish pale oil (3.77 g, 13.89 mmol, 90%).
1 H-NMR (300 MHz, MeCN-d3): δ / ppm = 3.81 (s, 3H, COOCH3), 3.35 - 3.26, 3.22 - 3.12 (m, 4H, CONCH2(CH2)4CH3), 1.63 - 1.46 (m, 4H, CONCH2CH2(CH2)3CH3), 1.39 - 1.18 (m, 12H, CON(CH2)2(CH2)3CH3), 0.89 (t, 6H,3JHH = 6.8 Hz, CON(CH2)5CH3).
13C-NMR (75 MHz, MeCN-d3): δ / ppm = 165.1 (COOCH3), 162.7 (CON), 52.9 (COOCH3),48.6, 44.7 (CONCH2(CH2)4CH3), 32.2, 32.1 (CONCH2CH2(CH2)3CH3), 29.2, 27.8 (CON(CH2)2CH2-(CH2)2CH3), 27.2, 26.8 (CON(CH2)3CH2CH2CH3), 23.3, 23.2 (CON(CH2)4CH2CH3), 14.3, 14.3 (CON(CH2)5CH3).
ESI-MS (MeOH): pos.: m/z (%) = 294.3 (15) [C15H29NO3Na]+.
HR-MS (MeOH): pos.: m/z = gef. 294.2042, ber. 294.2040 [C15H29NO3Na]+,
pos.: m/z = gef. 565.4195, ber. 565.4187 [C30H58N2O6Na]+.

Example 7

Synthesis of methyl di-2-ethylhexylamino oxamate

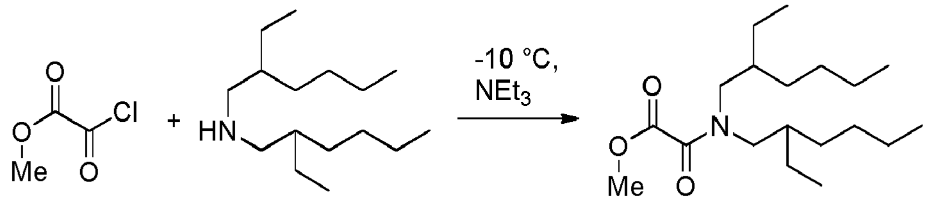


Di-2-ethylhexyl amine (33.63 g, 139.3 mmol, 1.00 eq) and $NEt_3$ (27.76 g, 274.4 mmol, 1.97 eq) were dissolved in diethyl ether (120 mL) at -10 °C. Monomethyl oxalyl chloride (17.87 g, 145.9 mmol, 1.05 eq), dissolved in diethyl ether (180 mL), was added slowly. The reaction mixture was stirred for another hour at room temperature, the formed precipitate was filtered off and the resulting crude product was washed with water and concentrated in vacuo. The product was isolated as yellowish pale oil (40.14 g, 122.6 mmol, 88%) upon distillation (110 °C, 3*10-2 mbar).
1 H-NMR (300 MHz, MeCN-d3): δ / ppm = 3.80 (s, 3H, COOCH3), 3.38 - 3.17, 3.15 - 3.05 (m, 4H, CONCH2CH(CH2CH3)(CH2)3CH3), 1.79 - 1.54 (m, 2H, CONCH2CH(CH2CH3)-(CH2)3CH3), 1.41 - 1.13 (m, 16H, CONCH2CH(CH2CH3)(CH2)3CH3), 0.98 - 0.79 (m, 12H, CONCH2CH(CH2CH3)(CH2)3CH3).
13C-NMR (75 MHz, MeCN-d3): δ / ppm = 165.1 (COOCH3), 163.6 (CON), 52.9 (COOCH3), 51.8, 47.1 (CONCH2CH(CH2CH3)(CH2)3CH3), 38.0, 37.2 (CONCH2CH(CH2CH3)-(CH2)3CH3), 31.1, 31.0 (CONCH2CH(CH2CH3)(CH2)3CH3), 29.4, 29.3 (CONCH2CH-(CH2CH3)CH2(CH2)2CH3), 24.5, 24.3 (CONCH2CH(CH2CH3)CH2CH2CH2CH3), 23.7, 23.7 (CONCH2CH(CH2CH3)(CH2)2CH2CH3), 14.4, 14.3 (CONCH2CH(CH2CH3)(CH2)3CH3), 11.1, 11.0, 10.9, 10.9 (CONCH2CH(CH2CH3)(CH2)3CH3).
ESI-MS (MeOH): pos.: m/z (%) = 350.1 (7) [C19H37NO3Na]+.
HR-MS (MeOH): pos.: m/z = gef. 350.2665, ber. 350.2666 [C19H37NO3Na]+.

Example 8

Synthesis of methyl di-iso-propylamino oxamate

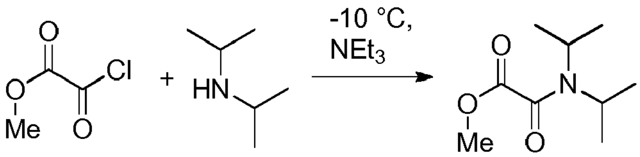


$NEt_3$ (2.48 g, 24.60 mmol, 1.20 eq) was added to a solution of monomethyl oxalyl chloride (2.52 g, 20.60 mmol, 1.00 eq) in diethyl ether (40 mL) at 10 °C. To the reaction mixture, di-iso-propyl amine (2.50 g, 24.70 mmol, 1.20 eq) was added at 20 °C and the mixture was stirred at room temperature for 72 h. Subsequently, the mixture was washed with sulphuric acid (5% in water, 50 mL), aqueous $Na_2CO_3$ solution (50 mL) and water (50 mL), the combined organic layers dried over $Na_2SO_4$ and the solvent removed in vacuo. The resulting solid was washed with a mixture of hexanes / diethyl ether (4:1) and dried in vacuo, yielding the product as pale yellow solid (3.45 g, 18.43 mmol, 89 %).
1 H-NMR (300 MHz, MeCN-d3): δ / ppm = 3.79 (s, 3H, COOCH3), 3.71 - 3.48 (m, 2H, CONCH(CH3)2), 1.37, 1.18 (d, 3H, 3JHH = 6.7 Hz, CONCH(CH3)2).
13C-NMR (75 MHz, MeCN-d3): δ / ppm = 165.2 (COOCH3), 162.5 (CON), 53.0 (COOCH3), 51.7, 45.4 (CONCH(CH3)2), 20.3, 20.8 (CONCH(CH3)2).
IR: $\tilde{v}$ / cm 1 = 2972 (w), 2937 (w), 1727 (s), 1645 (s), 1458 (m), 1434 (m), 1368 (m), 1352 (m), 1301 (w), 1229 (s), 1205 (s), 1184 (m), 1152 (m), 1138 (m), 1119 (m), 1041 (s), 969 (s), 917 (w), 880 (w), 860 (w), 804 (m), 753 (m), 615 (m), 531 (m), 514 (w), 499 (w).
ESI-MS (MeOH): pos.: m/z (%) = 188.1 (100) [C9H19NO3]+.
HR-MS (MeOH): pos.: m/z = gef. 188.1280, ber. 188.1281 [C9H19NO3]+.

Example 9

Synthesis of methyl dioctylamino oxamate

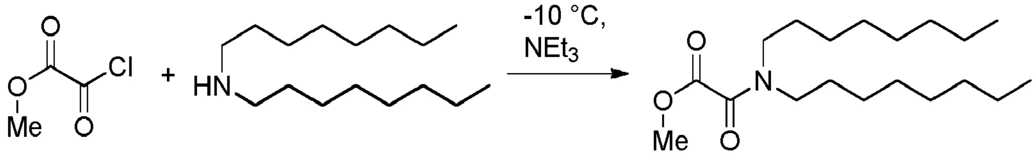


Dioctylamine (5.05 g, 20.91 mmol, 1.00 eq) and $NEt_3$ (2.12 g, 20.91 mmol, 1.00 eq) were dissolved in diethyl ether (40 mL) at -10 °C.
Monomethyl oxalyl chloride (4.25 g, 19.01 mmol, 0.91 eq), dissolved in diethyl ether (60 mL), was added slowly. The reaction mixture was stirred for another hour at room temperature, the formed precipitate was filtered off and the resulting crude product was washed with water and concentrated in vacuo. The product was isolated as yellowish pale oil (3.15 g, 9.63 mmol, 51 %). 1 H-NMR (300 MHz, MeCN-d3): δ / ppm = 3.80 (s, 3H, COOCH3), 3.35 - 3.26, 3.21 - 3.11 (m, 4H, CONCH2(CH2)6CH3), 1.65 - 1.45 (m, 4H, CONCH2CH2(CH2)5CH3), 1.41 - 1.16 (m, 20H, CON(CH2)2(CH2)5CH3), 0.89 (t, 6H, 3JHH = 6.7 Hz, CON(CH2)7CH3).
13C-NMR (75 MHz, MeCN-d3): δ / ppm = 164.9 (COOCH3), 162.7 (CON), 52.9 (COOCH3),48.6, 44.8 (CONCH2(CH2)6CH3), 32.6, 32.5 (CONCH2CH2(CH2)5CH3), 30.0, 29.9 (CON(CH2)2CH2-(CH2)4CH3), 29.9, 29.9 (CON(CH2)3CH2(CH2)3CH3), 29.3, 27.8 (CON(CH2)4CH2(CH2)2-CH3),27.5, 27.2 (CON(CH2)5CH2CH2CH3), 23.4 (CON(CH2)6CH2CH3), 14.4 (CON(CH2)7-CH3).
ESI-MS (MeOH): pos.: m/z (%) = 350.5 (35) [C19H37NO3Na]+.
HR-MS (MeOH): pos.: m/z = gef. 350.2664, ber. 350.2666 [C19H37NO3Na]+.

B) Synthesis of compounds (I)

Example 10

Synthesis of *N*-butyl-*N*-methyl imidazolium *tert.* butylamino oxamate

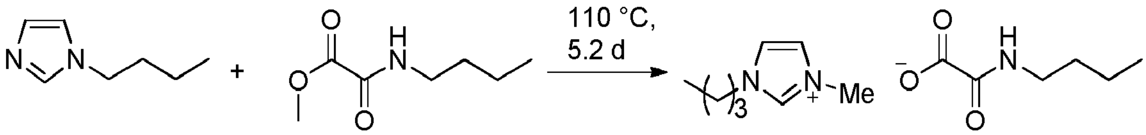


0.48 g (3.84 mmol, 3.00 eq) *N*-Butylimidazole was stirred with 0.20 g (1.28 mmol, 1.00 eq) methyl *tert.* butylamino oxamate at 110 °C for 5.2 days. The recation mixture was washed with hexanes and dried at 100°C in vacuo. The product was isolated as colourless solid (0.22 g, 0.79 mmol, 62 %).
$^1$H-NMR (300 MHz, MeCN-d$^3$):δ / ppm = 10.37 (s, 1 H, NCHN), 7.66 (s, 1H, CON*H*), 7.57 - 7.48 (m, 2H, N(C*H*)$_2$N), 4.23 (t, 2H, $^3J_{HH}$ = 7.2 Hz, NCHNC$H_2$(CH$_2$)$_2$CH$_2$), 3.90 (s, 3H, NCHNC$H_3$), 1.84 - 1.72 (m, 2H, NCHNCH$_2$C$H_2$CH$_2$CH$_3$), 1.35 - 1.20 (m, 9H, NCHN(CH$_2$)$_2$C$H_2$CH$_3$, CONHCH(C$H_3$)$_3$), 0.80 (t, 3H, $^3J_{HH}$ = 7.3 Hz, NCHN(CH$_2$)$_3$C$H_3$).
$^{13}$C-NMR (75 MHz, MeCN-d$^3$):δ / ppm = 166.1 (COO-), 165.3 (CON), 140.0 (NCHN), 124.3, 122.9 (N(*C*H)$_2$N), 50.4 (CONH*C*H(CH$_3$)$_3$), 49.7 (NCHNCH$_2$(CH$_2$)$_2$CH$_3$), 36.4 (NCHNCH$_3$), 32.8 (NCHNCH$_2$CH$_2$CH$_2$CH$_3$), 28.8 (CONH-CH(*C*H$_3$)$_3$), 19.9 (NCHN(CH$_2$)$_2$*C*H$_2$CH$_3$), 13.7 (NCHN(CH$_2$)$_3$CH$_3$).
ESI-MS (MeCN): pos.: *m*/*z* (%) = 139.1 (100) $[C_8H_{15}N_2]^+$,
neg.: *m*/*z* (%) = 144.4 (100) $[C_6H_{10}NO_3]^-$.
HR-MS (MeCN): pos.: *m*/*z*= gef. 139.1229, ber. 139.1230 $[C_8H_{15}N_2]^+$,
neg.: *m*/*z* = gef. 144.0668, ber. 144.0666 $[C_6H_{10}NO_3]^-$.

Example 11

Synthesis of tributyl methyl phosphonium 2-ethylhexylamino-oxamate

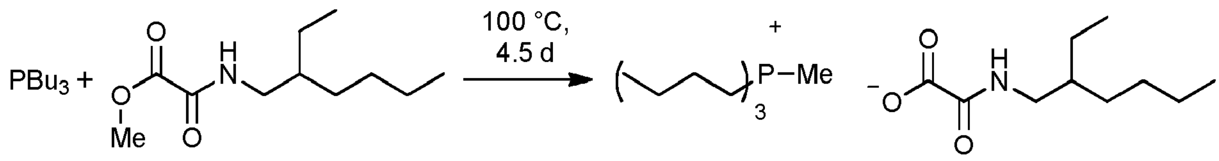


4.92 g (24.32 mmol, 1.90 eq) tributylphosphine and 2.75 g (12.78 mmol, 1.00 eq) methyl-2 ethylhexylamino oxamate were stirred for 4.5 days at 100°C. The reaction mixture was dried for 5h at 100°C and 2 x 10-2 mbar. The product was isolated as yellow-brown resin (5.22 g, 12.49 mmol, 98%).

1H-NMR (300 MHz, MeCN-d3):δ / ppm = 3.09 - 2.96 (m, 2H, HCIII), 2.34 - 2.16 (m, 6H, HC1), 1.85 (d, 3H, 2JPH = 14.0 Hz, Me), 1.57 - 1.12 (m, 21 H, HC2,3,IV-VII,IX), 0.97 - 0.74 (m, 15H, HC4,VIII,X).

13C-NMR (75 MHz, MeCN-d3): δ / ppm = 166.7 (CI), 164.4 (CII), 42.4 (CIII), 40.4 (CIV), 31.8 (CIX), 29.6 (CV), 25.0 (CVI), 24.5 (d, 3JCP = 16.0 Hz, C3), 24.0 (d, 2JCP = 4.5 Hz, C2), 23.7 (CVII), 20.2 (d, 1JCP = 49.4 Hz, C1),14.4 (CVIII), 13.7 (C4), 11.3 (CX), 4.1 (d, 1JCP = 52.1 Hz, Me).

31 P-NMR (122 MHz, MeCN-d3): δ / ppm = 33.39 (s).

IR: ṽ / cm 1 = 2957 (w), 2928 (w), 2872 (w), 1690 (m), 1670 (s), 1638 (s), 1494 (w), 1464 (w), 1351 (m), 1310 (w), 1235 (w), 1191 (w), 1098 (w), 1005 (w), 970 (w), 942 (m), 818 (w), 749 (w), 726 (w).

ESI-MS (MeOH): pos.: m/z (%) = 217.3 (100) [C13H30P]+,
neg.: m/z (%) = 200.2 (100) [C10H18NO3]-.
neg.: m/z (%) = 423.4 (95) [(C10H18NO3)2Na]-.

HR-MS (MeOH): pos.: m/z = gef. 217.2077, ber. 217.2080 [C13H30P]+,
neg.: m/z = gef. 200.1293, ber. 200.1292 [(C10H18NO3)2Na]-.

Example 12

Synthesis of *N*-butyl-*N*-methyl pyrrolidinium phenylamino oxamate

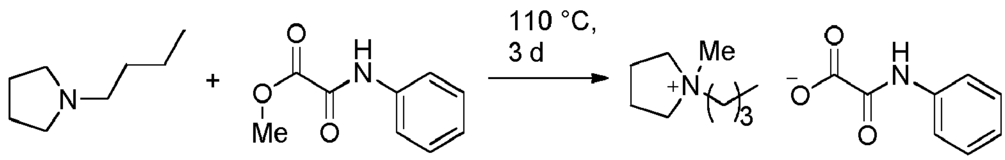


0.05 g (0.39 mmol, 1.05 eq) *N*-butylpyrrolidine was stirred with 0.07 g (0.37 mmol, 1.00 eq) methyl phenylamino oxamate and 2 mL of acetonitrile at 90 °C for 3 d. The reaction mixture was washed with hexanes and dried in vacuo. The product was isolated as yellowish solid (0.11 g (0.36 mmol, 92 %).

1 H-NMR (300 MHz, MeCN-d3): δ / ppm = 9.89 (s, 1 H, CONH), 7.71 - 7.62 (m, 2H, Hortho), 7.35 - 7.26 (m, 2H, Hmeta), 7.08 - 6.96 (m, 1 H, Hpara), 3.48 3.37 (m, 4H, NCH2(CH2)2CH2N), 3.29 - 3.22 (m, 2H, NCH2(CH2)2CH3), 2.95 (s, 3H, NCH3), 2.19 - 2.09 (m, 4H, NCH2(CH2)2CH2N), 1.77 - 1.65 (m, 2H, NCH2CH2CH2CH3), 1.42 1.29 (m, 2H, N(CH2)2CH2CH3), 0.95 (t, 3H, 3JHH = 7.3 Hz, N(CH2)3CH3).

13C-NMR (75 MHz, MeCN-d3): δ / ppm = 165.4 (COO-), 164.0 (CON), 140.2 (CONCquart), 129.7 (Cortho), 124.0 (Cpara), 120.0 (Cmeta), 65.1 (NCH2(CH2)2CH2N), 64.8 (NCH2(CH2)2CH3), 49.0 (NCH3), 26.3 (NCH2CH2CH2CH3), 22.3 (NCH2(CH2)2CH2)N, 20.4 (N(CH2)2CH2CH3), 13.9 (N(CH2)3CH3).

IR: ṽ / cm 1 = 2961 (w), 2874 (w), 1741 (s), 1668 (s), 1628 (s), 1587 (s), 1514 (s), 1491 (s), 1469 (s), 1438 (s), 1359 (s), 1300 (s), 1239 (s), 1175 (s), 1079 (w), 1028 (w), 1007 (w), 963 (w), 929 (w), 800 (w), 761 (s), 695 (s), 552 (w), 519 (w), 480 (w), 415 (w).

| Elemental analysis: | C17H26N2O3 (306.40 g/mol) in | | |
|---|---|---|---|
| | % | calculated | found |
| | C | 66.64 | 66.22 |
| | H | 8.55 | 8.68 |
| | N | 9.14 | 9.36 |

ESI-MS (MeOH): pos.: m/z (%) = 142.2 (100) [C9H20N]+,
neg.: m/z (%) = 351.0 (100) [(C8H6NO3)2Na]-.

HR-MS (MeOH): pos.: m/z = gef. 142.1590, ber. 142.1590 [C9H20N]+,
neg.: m/z = gef. 351.0601, ber. 351.0599 [(C8H6NO3)2Na]-.

Example 13

Synthesis of N,N'-dimethyl imidazolium dimethylamino oxamate

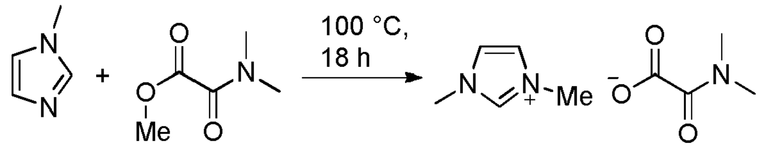


5.49 g (41.87 mmol, 1.00 eq) methyl dimethylamino oxamate and 10.35 g (126.1 mmol, 3.01 eq) N-methyl imidazole were heated for 18 hours at 100 °C. The reaction mixture was dried in vacuo for 1.5 h (100 °C, 2 x 10-5 mbar). The product was isolated as yellow oil (8.47 g, 39.72 mmol, 95 %).
Melting point: 59.4 °C
1 H-NMR (300 MHz, MeCN-d3): δ / ppm = 9.72 (s, 1 H, NCHN), 7.62 (s, 2H, N(CH)2N), 3.84 (s, 6H, CH3NCHNCH3), 2.88, 2.70 (s, 6H, CON(CH3)2).
13C-NMR (75 MHz, MeCN-d3): δ / ppm = 172.6 (COO-), 169.6 (CON), 139.4 (NCHN), 124.4 (N(CH)2N), 37.2, 32.6 (CON(CH3)2), 36.4 (CH3NCHNCH3).

| Elemental analysis: | C9H15N3O3 (213.23 g/mol) in | | |
|---|---|---|---|
| | % | calculated | found |
| | C | 50.69 | 50.59 |
| | H | 7.09 | 7.21 |
| | N | 19.71 | 20.15 |

ESI-MS (MeOH): pos.: m/z (%) = 97.1 (100) [C5H9N2]+,
neg.: m/z (%) = 116.1 (30) [C4H6NO3]-.
HR-MS (MeOH): pos.: m/z = gef. 97.0760, ber. 97.0760 [C5H9N2]+,
neg.: m/z = gef. 116.0354, ber. 116.0353 [C4H6NO3]-.

Example 14

Synthesis of N,N'-Dimethyl imidazolium dihexylamino oxamate

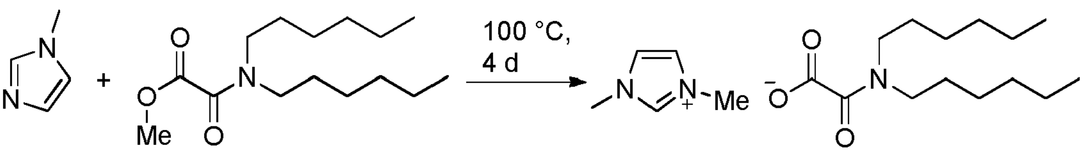


0.65 g (7.94 mmol, 2.00 eq) N-methyl imidazole and 1.08 g (3.97 mmol, 1.00 eq) methyl dihexylamino oxamate were heated for 6 days at 100 °C. Remaining starting material was removed in vacuo (95 °C, 4 h, 3*10-5 mbar) and the product was isolated as yellow oil (0.83 g, 2.34 mmol, 59%).
1 H-NMR (300 MHz, MeCN-d3): δ / ppm = 9.90 (s, 1 H, NCHN), 7.64 - 7.61 (m, 2H, N(CH)2N), 3.87 (s, 6H, NCHNCH3), 3.26 - 3.11 (m, 4H, CONCH2(CH2)4CH3), 1.59 - 1.33 (m, 4H, CONCH2CH2(CH2)3CH3), 1.30 - 1.11 (m, 12H, CON(CH2)2(CH2)3CH3), 0.87 - 0.74 (m, 6H, CON(CH2)5CH3).
13C-NMR (75 MHz, MeCN-d3): δ / ppm = 172.6 (COO-), 169.8 (CON), 139.6 (NCHN), 124.4 (N(CH)2N), 48.3, 43.4 (CONCH2(CH2)4CH3), 36.3 (NCHNCH3), 32.2, 32.2 (CONCH2CH2(CH2)3CH3), 29.6, 28.3 (CON(CH2)2CH2(CH2)2CH3), 27.4, 27.2 (CON(CH2)3CH2CH2CH3), 23.2 (CON(CH2)4CH2CH3), 14.3, 14.3 (CON(CH2)5CH3).

IR: $\tilde{\nu}$ / cm 1 = 3071 (w), 2954 (m), 2926 (m), 2856 (m), 1604 (s), 1573 (s), 1461 (m), 1427 (m), 1365 (m), 1292 (w), 1250 (w), 1180 (m), 1141 (w), 887 (w), 836 (w), 758 (w), 727 (w), 696 (w), 629 (w).

| Elemental analysis: | C19H35N3O3 (353.30 g/mol) in | | |
|---|---|---|---|
| | % | calculated | found |
| | C | 64.56 | 64.10 |
| | H | 9.98 | 10.34 |
| | N | 11.89 | 11.73 |

ESI-MS (MeCN): pos.: m/z (%) = 97.1 (80) [C5H9N2]+,
neg.: m/z (%) = 256.3(100) [C14H26NO3]-.
HR-MS (MeCN): pos.: m/z = gef. 97.0760, ber. 97.0760 [C5H9N2]+,
neg.: m/z = gef. 256.1918, ber. 256.1918 [C14H26NO3]-.

Example 15

Synthesis of N,N'-Dimethyl imidazolium di-2-ethylhexylamino oxamate

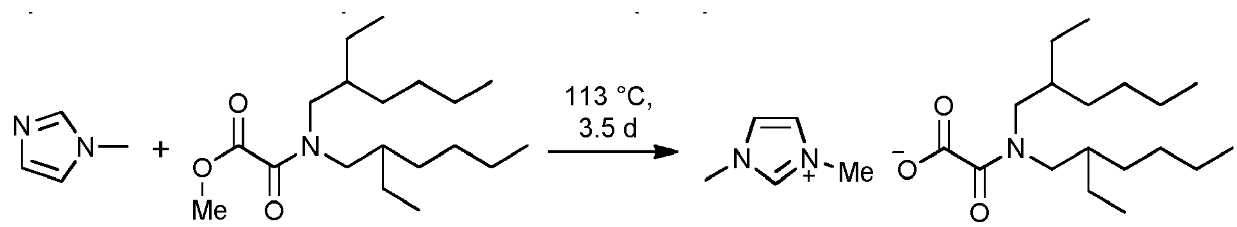


11.64 g (114.7 mmol, 4.05 eq) N-methyl imidazole and 9.28 g (28.3 mmol, 1.00 eq) methyl-di-2 ethyl hexylamino oxamate were stirred for 3.5 d at 113 °C. Remaining starting material was removed in vacuo (110 °C, 4 h, 3*10-5 mbar). The product was isolated as yellow-red resin (11.51 g, 28.09 mmol, 99%).

1 H-NMR (300 MHz, MeCN-d$^3$):$\delta$ / ppm = 9.70 (s, 1 H,NCHN), 7.50 - 7.35 (m, 2H, N(CH)2N), 3.88 (s, 6H, H3CNCHNCH3), 3.35 - 2.99 (m, 4H, CONCH2CH(CH2CH3)(CH2)3CH3), 1.71 - 1.55 (m, 2H,CONCH2CH(CH2CH3)(CH2)3CH3), 1.36-1.16 (m, 16H, CONCH2CH(CH2CH3)(CH2)3CH3), 0.93 - 0.78 (m, 12H, CONCH2CH(CH2CH3)(CH2)3CH3). 13C-NMR (75 MHz, MeCN-d3): $\delta$ / ppm = 173.7 (COO-), 169.7 (CON), 139.5 (NCHN), 124.3 (N(CH)2N), 51.4, 51.3, 45.3, 45.2 (CONCH2CH(CH2CH3)(CH2)3CH3), 37.8, 37.3, 37.3 (CONCH2CH(CH2CH3)(CH2)3CH3), 36.5 (H3CNCHNCH3), 31.4, 31.3, 31.3 (CONCH2CH-(CH2CH3)(CH2)3CH3), 29.6, 29.6, 29.5, 29.5 (CONCH2CH(CH2CH3)CH2(CH2)2CH3), 24.7, 24.6, 24.5, 24.4 (CONCH2CH(CH2CH3)CH2CH2CH2CH3), 23.8, 23.7 (CONCH2CH(CH2CH3)-(CH2)2CH2CH3), 14.3 (CONCH2CH(CH2CH3)(CH2)3CH3), 11.2, 11.2, 11.0, 11.9 (CONCH2-CH(CH2CH3)(CH2)3CH3).

IR: $\tilde{\nu}$ / cm 1 = 2956 (m), 2926 (m), 2871 (w), 1654 (w), 1604 (s), 1572 (m), 1456 (m), 1358 (m), 1284 (w), 1205 (m), 1178 (m), 1139 (w), 835 (m), 788 (w), 707 (w), 628 (w).

| Elemental analysis: | C23H43N3O3 (409.60 g/mol) in | | |
|---|---|---|---|
| | % | calculated | found |
| | C | 67.44 | 66.56 |
| | H | 10.58 | 10.70 |
| | N | 10.26 | 10.12 |

ESI-MS (MeCN): pos.: m/z (%) = 97.1 (100) [C5H9N2]+,
neg.: m/z (%) = 312.3 (100) [C18H34NO3]-.
HR-MS (MeCN): pos.: m/z = gef. 97.0760 , ber. 97.0760 [C5H9N2]+,
neg.: m/z = gef. 312.2546 , ber. 312.2544 [C18H34NO3]-.

Example 16

Synthesis of N-Decyl-N'-methylimidazolium-di-2-ethylhexylamino-oxamat

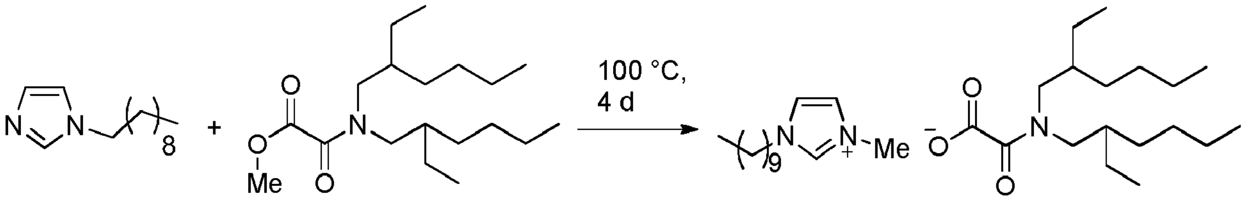


2.87 g (13.77 mmol, 1.60 eq) N-decyl imidazole and 2.83 g (8.63 mmol, 1.00 eq) methyl di-2 ethylhexylamino oxamate were heated for 4 days at 100 °C. Remaining starting material was removed in vacuo (90 °C, 7 h, 2*10-4 mbar) and the product was isolated as yellow oil (3.41 g, 6.36 mmol, 74%).

1 H-NMR (300 MHz, MeCN-$d^3$):$\delta$ / ppm = 10.14 (s, 1 H,NCHN), 7.73 - 7.60 (m, 2H, N(CH)2N), 4.22 (t, 2H, 3JHH = 7.3 Hz, NCHNCH2(CH2)8CH3), 3.90 (s, 3H, NCHNCH3), 3.38 - 2.96 (m, 4H, CONCH2CH(CH2CH3)(CH2)3CH3), 1.89 - 1.72 (m, 2H, NCHNCH2CH2(CH2)7CH3), 1.70 - 1.53 (m, 2H, CONCH2CH(CH2CH3)(CH2)3CH3), 1.39 - 1.09 (m, 30H, NCHN(CH2)2(CH2)7CH3, CONCH2CH(CH2CH3)(CH2)3CH3), 0.96 - 0.74 (m, 15H, NCHN(CH2)9CH3, CONCH2CH(CH2CH3)(CH2)3CH3).

13C-NMR (75 MHz, MeCN-$d^3$):$\delta$ / ppm = 173.6 (COO-), 169.7 (CON), 139.3 (NCHN), 124.4, 123.2 (N(CH)2N), 51.3, 51.2, 45.3, 45.2 (CONCH2CH(CH2CH3)(CH2)3CH3), 50.0 (NCHNCH2(CH2)8CH3), 37.8, 37.3, 37.2 (CONCH2CH(CH2CH3)(CH2)3CH3), 36.3 (NCHNCH3), 32.6 (NCHNCH2CH2(CH2)7CH3), 31.4, 31.3, 31.3 (CONCH2CH(CH2CH3)-(CH2)3CH3), 30.9 (NCHN(CH2)2CH2(CH2)6CH3), 30.2 (NCHN(CH2)3CH2(CH2)5CH3), 30.2 (NCHN(CH2)4CH2(CH2)4CH3), 30.0 (NCHN(CH2)5CH2(CH2)3CH3), 29.8 (NCHN(CH2)6-CH2(CH2)2CH3), 29.6, 29.5, 29.5, 29.4 (CONCH2CH(CH2CH3)CH2(CH2)2CH3), 26.8 (NCHN(CH2)7CH2CH2CH3), 24.6, 24.6, 24.5, 24.4 (CONCH2CH(CH2CH3)CH2CH2CH2CH3), 23.8, 23.7 (CONCH2CH(CH2CH3)(CH2)2CH2CH3), 23.3 (NCHN(CH2)8CH2CH3), 14.4 (CONCH2CH(CH2CH3)(CH2)3CH3,NCHN(CH2)9CH3), 11.3, 11.2, 11.1, 11.0 (CONCH2CH(CH2CH3)(CH2)3CH3).

IR: $\tilde{v}$ / cm 1 = 2956 (s), 2923 (s), 2855 (s), 1607 (s), 1570 (m), 1459 (m), 1377 (m), 1268 (w), 1222 (m), 1205 (m), 1178 (m), 890 (w), 836 (w), 768 (w), 708 (w), 657 (w), 627 (w).

| Elemental analysis: | C32H61N3O3 (535.85 g/mol) in | | |
|---|---|---|---|
| | % | calculated | found |
| | C | 71.73 | 71.42 |
| | H | 11.47 | 11.98 |
| | N | 7.84 | 7.92 |

ESI-MS (MeCN): pos.: m/z (%) = 223.3 (5) [C14H27N2]+,
neg.: m/z (%) = 312.3 (100) [C18H34NO3]-.
HR-MS (MeCN): pos.: m/z = gef. 223.2166 , ber. 223.2169 [C14H27N2]+,
neg.: m/z = gef. 312.2544 , ber. 312.2544 [C18H34NO3]-.

Example 17

Synthesis of N-butyl-N'-methyl imidazolium-di-2-ethylhexylamino oxamate

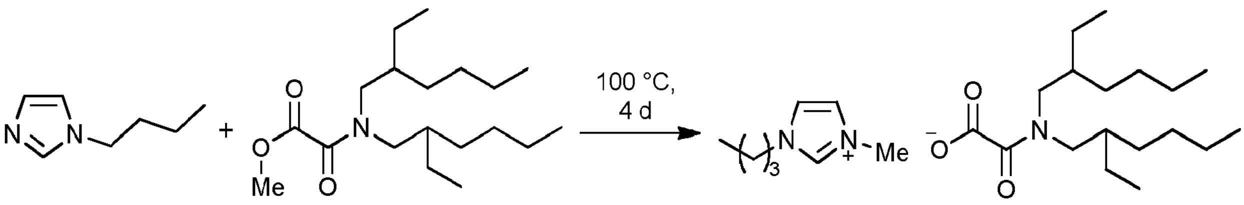


1.41 g (11.36 mmol, 1.50 eq) N-butyl imidazole and 2.48 g (7.58 mmol, 1.00 eq) methyl di-2 ethylhexylamino oxamate were heated for 4 days at 100 °C. Remaining starting material was removed in vacuo (90 °C, 4 h, 3*10-4 mbar) and the product was isolated as yellow oil (3.05 g, 6.75 mmol, 89%).

1H-NMR (300 MHz, MeCN-d$^3$):$\delta$ / ppm = 10.19 (s, 1 H,NCHN), 7.87 - 7.54 (m, 2H, N(CH)2N), 4.24 (t, 2H, 3JHH = 7.2 Hz, NCHNCH2(CH2)2CH3), 3.90 (s, 3H, NCHNCH3), 3.37 - 2.94 (m, 4H, CONCH2CH(CH2CH3)(CH2)3CH3), 1.83 - 1.68 (m, 2H, NCHNCH2CH2CH2CH3), 1.66-1.51 (m, 2H,CONCH2CH(CH2CH3)(CH2)3CH3), 1.33 - 1.08 (m, 18H, NCHN(CH2)2CH2CH3, CONCH2CH(CH2CH3)(CH2)3CH3), 0.91 - 0.72 (m, 15H, NCHN(CH2)3CH3, CONCH2CH(CH2CH3)(CH2)3CH3).

13C-NMR (75 MHz, MeCN-d3): $\delta$ / ppm = 173.5 (COO-), 169.7 (CON), 139.2 (NCHN), 124.5, 123.3 (N(CH)2N), 51.3, 51.2, 45.3, 45.2 (CONCH2CH(CH2CH3)(CH2)3CH3), 49.6 (NCHNCH2(CH2)2CH3), 37.7, 37.2, 37.2 (CONCH2CH(CH2CH3)(CH2)3CH3), 36.2 (NCHNCH3), 32.9 (NCHNCH2CH2CH2CH3), 31.3, 31.3 (CONCH2CH(CH2CH3)-(CH2)3CH3),29.5, 29.5, 29.4, 29.4 (CONCH2CH(CH2CH3)CH2(CH2)2CH3), 24.5, 24.5, 24.5, 24.4 (CONCH2CH(CH2CH3)CH2CH2CH2CH3), 23.7, 23.6 (CONCH2CH(CH2CH3)-(CH2)2CH2CH3), 19.9 (NCHN(CH2)2CH2CH3), 14.4 (CONCH2CH(CH2CH3)(CH2)3CH3), 13.8 (NCHN(CH2)3CH3), 11.2, 11.2, 11.1, 11.0 (CONCH2CH(CH2CH3)(CH2)3CH3).

IR: $\tilde{v}$ / cm 1 = 2956 (s), 2926 (s), 2871 (m), 1606 (s), 1570 (m), 1459 (m), 1376 (m), 1268 (w), 1222 (m), 1205 (m), 1176 (m), 1018 (w), 934 (w), 836 (w), 766 (w), 708 (w), 657 (w), 627 (w).

| Elemental analysis: | C26H49N3O3 (451.69 g/mol) in | | |
|---|---|---|---|
| | % | calculated | found |
| | C | 69.14 | 69.11 |
| | H | 10.93 | 11.05 |
| | N | 9.30 | 9.77 |

ESI-MS (MeCN): pos.: m/z (%) = 139.1 (100) [C8H15N2]+,
neg.: m/z (%) = 312.3 (100) [C18H34NO3]-.
HR-MS (MeCN): pos.: m/z = gef. 139.1228 , ber. 139.1230 [C8H15N2]+,
neg.: m/z = gef. 312.2543 , ber. 312.2544 [C18H34NO3]-.

Example 18

Synthesis of tributyl methyl phosphonium di-2-ethylhexylamino oxamate

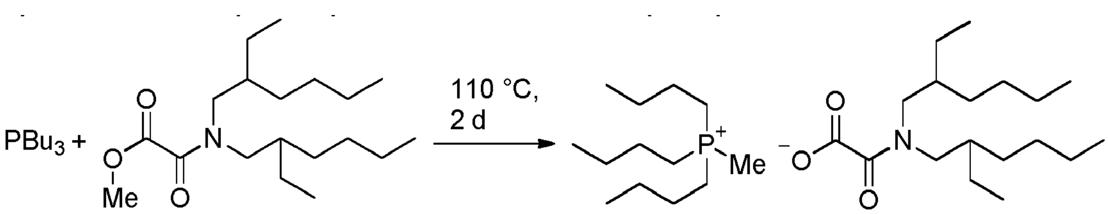


6.45 g (31.88 mmol, 1.20 eq) tributyl phosphine and 8.70 g (26.57 mmol, 1.00 eq) methyl di-2 ethylhexylamino oxamate were heated for 2 days at 110 °C. Remaining starting material was removed in vacuo (90 °C, 4 h, 3*10-5 mbar) and the product was isolated as yellow oil (11.56 g, 21.83 mmol, 82%).

1H-NMR (300 MHz, MeCN-d3): $\delta$ / ppm = 3.33 - 2.93 (m, 4H, HCIII), 2.30 - 2.13 (m, 6H, HC1), 1.80 (d, 3H, 2JPH = 13.9 Hz, Me), 1.67 - 1.13 (m, 30H, HC2,3,IV-VII,IX), 0.97 - 0.77 (m, 21 H, HC4,VIII,X).

13C-NMR (75 MHz, MeCN-d3): $\delta$ / ppm = 173.8 (CI), 169.2 (CII), 51.3, 51.2, 45.1, 45.0 (CIII), 37.7, 37.3, 37.2 (CIV),

31.4, 31.4, 31.3, 31.3 (CIX), 29.7, 29.6, 29.5, 29.5 (CV), 24.7, 24.6, 24.5, 24.4 (CVI), 24.5 (d, 3JCP = 16.0 Hz, C3), 23.9 (d, 2JCP = 4.5 Hz, C2), 23.8, 23.7 (CVII), 20.3 (d, 1JCP = 49.5 Hz, C1),14.4 (CVIII), 13.6 (C4), 11.3, 11.3, 11.0, 11.0 (CX), 4.2 (d, 1JCP = 52.4 Hz, Me).
IR: ṽ / cm 1 = 2957 (m), 2927 (m), 2872 (m), 1611 (s), 1460 (m), 1378 (m), 1391 (m), 1378 (m), 1352 (m), 1310 (w), 1222 (m), 1203 (m), 1130 (w), 1099 (w), 1005 (w), 948 (m), 834 (w), 779 (w), 708 (w).
ESI-MS (MeCN): pos.: m/z (%) = 217.3 (100) [C13H30P]+,
neg.: m/z (%) = 312.4 (100) [C18H34NO3]-.
HR-MS (MeCN): pos.: m/z = gef. 217.2078 , ber. 217.2080 [C13H30P]+,
neg.: m/z = gef. 312.2544 , ber. 312.2544 [C18H34NO3]-.

Example 19

Synthesis of N-butyl-N-methyl pyrrolidinium di-iso-propylamino oxamate

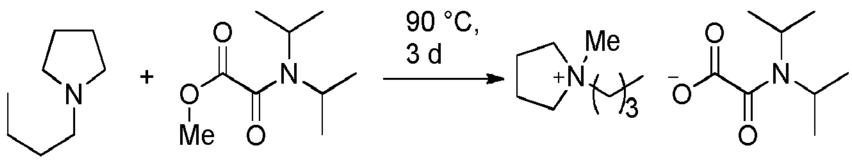


1.39 g (7.42 mmol, 1.00 eq) methyl di-iso-propylamino oxamate, 2.36 g (18.55 mmol, 2.50 eq) N- butyl pyrrolidine and 2 mL of acetonitril were stirred for 3 d at 90 °C. The reaction mixture was washed with a mixture of hexanes / diethylether (5:1) and dried in vacuo. The product was isolated as slightly yellowish solid (1.96 g, 6.23 mmol, 84 %).
Melting point: >100 °C
1 H-NMR (300 MHz, MeCN-d3): δ / ppm = 4.05 - 3.96 (m, 1 H, CONCH(CH3)2), 3.56 - 3.43 (m, 4H, NCH2(CH2)2CH2N), 3.42 - 3.26 (m, 3H, NCH2(CH2)2CH3, CONCH(CH3)2), 2.98 (s, 3H, NCH3), 2.21 - 2.05 (m, 4H, NCH2(CH2)2CH2N), 1.79 - 1.62 (m, 2H, NCH2CH2CH2CH3), 1.45 1.37 (m, 2H, N(CH2)2CH2CH3), 1.35, 1.11 (d, 12H, 3JHH = 6.7 Hz, CONCH(CH3)2), 0.95 (t, 3H, 3JHH = 7.3 Hz, N(CH2)3CH3).
13C-NMR (75 MHz, MeCN-d3): δ / ppm = 173.2 (COO-), 169.9 (CON), 65.1 (NCH2(CH2)2CH2N), 64.7 (NCH2(CH2)2CH3), 50.1 (CONCH(CH3)2), 49.0 (NCH3), 26.3 (NCH2CH2CH2CH3), 22.3 (NCH2(CH2)2CH2N), 21.2, 21.1 (NCH(CH3)2), 20.4 (N(CH2)2CH2CH3), 13.9 (N(CH2)3CH3).
IR: ṽ / cm 1 = 2955 (m), 2848 (m), 1707 (m), 1624 (s), 1559 (w), 1463 (w), 1398 (w), 1374 (w), 1181 (s), 960 (w), 881 (w), 767 (w), 746 (w), 735 (w), 660 (w), 632 (w), 614 (w), 449 (w), 425 (w).

| Elemental analysis | C17H34N2O3 (314.46 g/mol) in | | |
|---|---|---|---|
| | % | calculated | found |
| | C | 64.93 | 64.93 |
| | H | 10.90 | 10.96 |
| | N | 8.91 | 9.04 |

ESI-MS (MeOH): pos.: m/z (%) = 142.1 (100) [C9H20N]+,
neg.: m/z (%) = 367.2 (100) [(C8H14NO3)2Na]-, 172 (22) [C8H14NO3]-.
HR-MS (MeOH): pos.: m/z = gef. 142.1590, ber. 142.1590 [C9H20N]+,
neg.: m/z = gef. 367.1854, ber. 367.1851 [(C8H14NO3)2Na]-,
gef. 172.0980, ber. 172.0979 [C8H14NO3]-.

Example 20

Synthesis of N-butyl-N-methyl pyrrolidinium dioctylamino oxamate

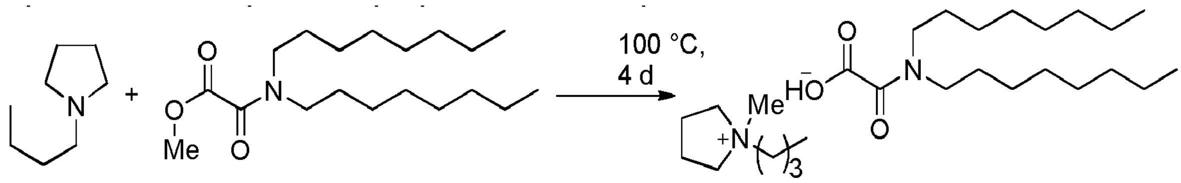


0.40 g (3.17 mmol, 1.25 eq) N-butyl pyrrolidine and 0.83 g (2.54 mmol, 1.00 eq) methyl dioctylamino oxamate were heated for 4 days at 100 °C. Remaining starting material was removed in vacuo (100 °C, 5 h, 2*10-5 mbar) and the product was isolated as brown resin (0.97 g, 2.13 mmol, 84%).

1 H-NMR (300 MHz, MeCN-d3): δ / ppm = 3.63 - 3.50 (m, 4H, NCH2(CH2)2CH2N), 3.45 - 3.36 (m, 2H, NCH2(CH2)2CH3), 3.22 - 3.08 (m, 4H, CONCH2(CH2)6CH3), 3.03 (s, 3H, NCH3), 2.16 - 2.05 (m, 4H, NCH2(CH2)2CH2N), 1.74 - 1.61 (m, 2H, NCH2CH2CH2CH3), 1.57 - 1.10 (m, 26H, CONCH2(CH2)6CH3, N(CH2)2CH2CH3), 0.91 (t, 3H, 3JHH = 7.4 Hz, N(CH2)3CH3), 0.87 - 0.79 (m, 6H, CON(CH2)5CH3).
13C-NMR (75 MHz, MeCN-d3): δ / ppm = 171.9 (COO-), 169.3 (CON), 64.5 (NCH2(CH2)2CH2N), 64.1 (NCH2(CH2)2CH3), 48.4 (NCH3), 48.3, 43.3 (CONCH2(CH2)6CH3), 32.5 (CONCH2CH2(CH2)5CH3), 30.1 (CON(CH2)2CH2(CH2)2CH3), 29.9 (CON(CH2)4CH2-(CH2)2CH3), 29.7, 28.4 (CON(CH2)4CH2(CH2)2CH3), 27.8, 27.6 (CON(CH2)5CH2CH2CH3), 26.3 (NCH2CH2CH2CH3), 23.3 (CON(CH2)6CH2CH3), 22.2 (NCH2(CH2)2CH2N), 20.4 (N(CH2)2CH2CH3), 14.4 (CON(CH2)7CH3), 14.0 (N(CH2)3CH3).
IR: $\tilde{v}$ / cm 1 = 2956 (m), 2923 (s), 2854 (m), 1609 (s), 1464 (m), 1360 (m), 1269 (w), 12301 (w), 1171 (m), 1137 (w), 1163 (w), 933 (w), 833 (w), 753 (w), 722 (w), 695 (w), 657 (w).

| Elemental analysis: | C27H54N2O3 (454.73 g/mol) | | | | |
|---|---|---|---|---|---|
| | in % | berechnet | gefunden | | |
| | C | 71.31 | 68.62 | 68.63 | 67.50 |
| | H | 11.97 | 11.95 | 11.93 | 12.02 |
| | N | 6.16 | 6.94 | 6.93 | 6.83 |

ESI-MS (MeCN): pos.: m/z (%) = 142.3 (100) [C9H20N2]+,
neg.: m/z (%) = 312.4 (100) [C18H34NO3]-.
HR-MS (MeCN): pos.: m/z = gef. 142.1591, ber. 142.1590 [C9H20N2]+,
neg.: m/z = gef. 312.2545 , ber. 312.2544 [C18H34NO3]-.

Example 21

Synthesis of N-Butyl-N-methyl pyrrolidinium di-2-ethylhexylamino oxamate

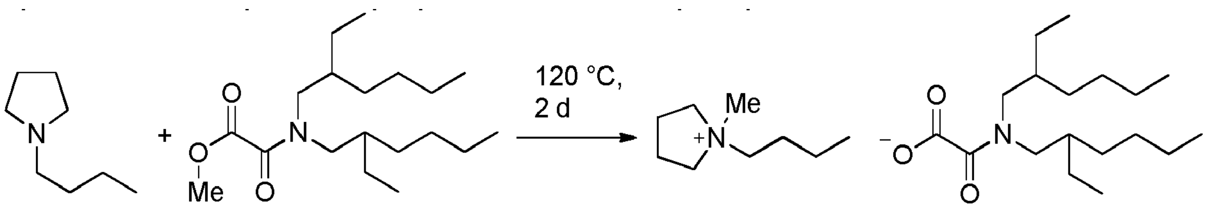


6.88 g (54.05 mmol, 1.15 eq) N-butyl pyrrolidine and 15.39 g (47.00 mmol, 1.00 eq) methyl di-2-ethylhexylamino oxamate were heated for 2 days at 120 °C. Remaining startinmaterial was removed in vacuo (130 °C, 5 h, 3•10-6 mbar) and the product was isolated as yellow solid (19.96 g, 43,92 mmol, 93%).
Melting point: 120 - 125 °C.
1 H-NMR (300 MHz, MeCN-d3): δ / ppm = 3.67 - 3.52 (m, 4H, HC1,1'), 3.51 - 3.40 (m, 2H, HC3), 3.29 - 2.86 (m, 7H, HCIII und Me), 2.16 - 2.00 (m, 4H, HC2,2'), 1.72 - 1.48 (m, 4H, HC4,IV), 1.37 - 1.07 (m, 18H, HC5,V-VII,IX), 0.95 - 0.68 (m, 15H, HC6,VIII,X).
13C-NMR (75 MHz, MeCN-d3): δ / ppm = 173.7 (CI), 169.2 (CII), 64.3 (C1,1'), 63.8 (C3), 51.2, 51.1, 45.1, 44.9 (CIII), 48.1 (Me), 37.7, 37.6, 37.2, 37.1 (CIV), 31.4, 31.3, 31.2 (CIX), 29.5, 29.5, 29.4, 29.4 (CV), 26.3 (C4), 24.6, 24.5, 24.3 (CVI), 23.7, 23.7 (CVII), 22.1 (C2,2'), 20.4 (C5), 14.5, 14.0 (C6,VIII), 11.3, 11.2, 11.1, 11.0 (CX).

IR: ṽ / cm 1 = 2957 (m), 2926 (m), 2872 (m), 1608 (s), 1459 (m), 1439 (m), 1376 (m), 1352 (m), 1269 (w), 1222 (m), 1202 (m), 1126 (w), 1065 (w), 1007 (w), 934 (w), 834 (w), 765 (w), 727 (w), 708 (m).
ESI-MS (MeCN): pos.: m/z (%) = 124.1 (100) [C9H20N]+,
neg.: m/z (%) = 312.3 (100) [C18H34NO3]-.
HR-MS (MeCN): pos.: m/z = gef. 124.1590 , ber. 124.1590 [C9H20N]+,
neg.: m/z = gef. 312.2546 , ber. 312.2544 [C18H34NO3]-.

C) Dissolution and separation of rare earth element compounds

Extraction in dichlor methane ($CH_2Cl_2$)

Example 22

Liquid-liquid-extraction of $Eu^{3+}$ with *N*-Butyl-*N'*-methyl imidazolium-[di-2-ethyl-hexylamino]-(oxo)acetate

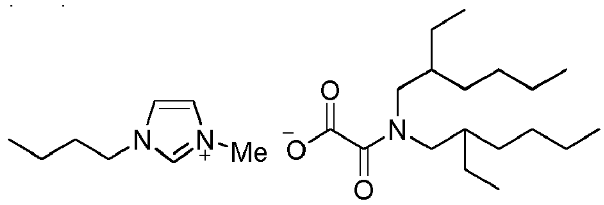

A solution of *N*-Butyl-*N'*-methylimidazolium-[di-2-ethyl-hexylamino]-(oxo)acetate in dichloromethane was mixed with an aqueous solution of $EuCl_3$ and vigorously stirred at room temperature for 20 min. After this time, the phases were separated and analyzed by ICP-MS. Depending on the ratio of ionic liquid to $EuCl_3$, 16.5% to 99.7% of the Eu were extracted into the organic phase.

| Organic phase (g) | Amount IL (mmol) | Aqueous phase (g) | Amount EuCl3 (mmol) | Ratio IL/ Eu3+ | Extraction efficiency (%) |
|---|---|---|---|---|---|
| 6,4 | 0,5 | 4,1 | 0,5 | 1,0 | 16,5 |
| 6,6 | 1,0 | 4,2 | 0,5 | 2,0 | 41,3 |
| 6,4 | 1,5 | 4,1 | 0,5 | 3,0 | 65,7 |
| 6,5 | 2,0 | 4,1 | 0,5 | 4,0 | 87,4 |
| 6,4 | 2,5 | 4,1 | 0,5 | 5,0 | 99,7 |

Extraction in toluene

Example 23

Liquid-liquid-extraction of $Ce^{3+}$ with *N,N'*-Dimethyl imidazolium-[di-2-ethyl-hexylamino]-(oxo)acetate

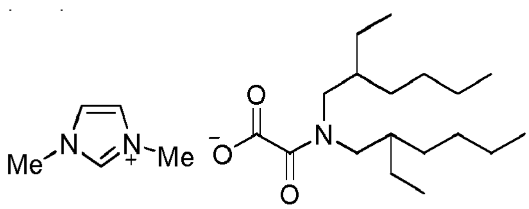

A solution of *N,N'*-Dimethyl imidazolium-[di-2-ethyl-hexylamino]-(oxo)acetate in toluene was mixed with an

aqueous solution of $CeCl_3$ and vigorously stirred at room temperature for 20 min. After this time, the phases were separated and analyzed by ICP-MS. Depending on the ratio of ionic liquid to $CeCl_3$, 35% to >99% of the Ce were extracted into the organic phase.

| Organic phase (g) | Amount IL (mmol) | Aqueous phase (g) | Amount $CeCl_3$ (mmol) | Ratio IL/ Ce3+ | Extraction efficiency (%) |
|---|---|---|---|---|---|
| 3,2 | 0,3 | 3,1 | 0,26 | 1,15 | 35,1 |
| 3,2 | 0,6 | 3,1 | 0,26 | 2,29 | 69,1 |
| 3,2 | 0,9 | 3,1 | 0,26 | 3,44 | quant. |
| 3,2 | 1,2 | 3,1 | 0,26 | 4,57 | quant. |

Example 24

Liquid-liquid-extraction of $Ce^{3+}$ with *N*-Butyl-*N'*-methyl imidazolium-[di-2-ethyl-hexylamino]-(oxo)acetate

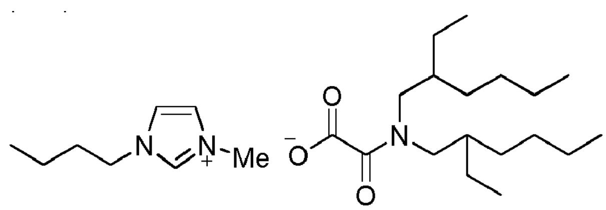

A solution of *N*-Butyl-*N'*-methylimidazolium-[di-2-ethyl-hexylamino]-(oxo)acetate in toluene was mixed with an aqueous solution of $CeCl_3$ and vigorously stirred at room temperature for 20 min. After this time, the phases were separated and analyzed by ICP-MS. Depending on the ratio of ionic liquid to $CeCl_3$, 32% to 97% of the Ce were extracted into the organic phase.

| Organic phase (g) | Amount IL (mmol) | Aqueous phase (g) | Amount $CeCl_3$ (mmol) | Ratio IL/ Ce3+ | Extraction efficiency (%) |
|---|---|---|---|---|---|
| 2,5 | 0,3 | 2,7 | 0,26 | 1,00 | 31,7 |
| 2,5 | 0,5 | 2,6 | 0,25 | 2,00 | 62,9 |
| 2,4 | 0,7 | 2,5 | 0,24 | 3,00 | 95,0 |
| 2,3 | 1,0 | 2,5 | 0,24 | 4,00 | quant. |

Example 25

Liquid-liquid-extraction of $Er^{3+}$ with Tributyl methyl phosphonium di-2-ethyl-hexylamino-(oxo)acetate

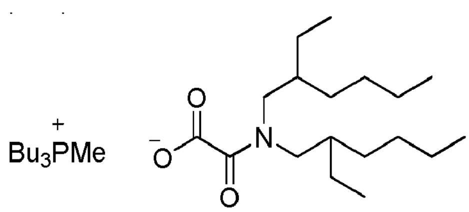

A solution of tributyl methyl phosphonium di-2-ethyl-hexylamino-(oxo)acetate in toluene was mixed with an

aqueous solution of $ErCl_3$ and vigorously stirred at room temperature for 20 min. After this time, the phases were separated and analyzed by ICP-MS. Depending on the ratio of ionic liquid to $ErCl_3$, 31% to >99% of the Er were extracted into the organic phase.

| Organic phase (g) | Amount IL (mmol) | Aqueous phase (g) | Amount ErCl3 (mmol) | Ratio IL/ Er3+ | Extraction efficiency (%) |
|---|---|---|---|---|---|
| 2,0 | 0,2 | 2,0 | 0,19 | 1,14 | 31,6 |
| 2,0 | 0,4 | 2,0 | 0,19 | 2,29 | 67,3 |
| 2,0 | 0,7 | 2,0 | 0,19 | 3,42 | 91,3 |
| 2,0 | 0,9 | 2,0 | 0,19 | 4,53 | 98,7 |
| 2,0 | 1,1 | 2,0 | 0,19 | 5,73 | 81,9 |

Influence of pH-value of the aqueous phase on extraction selectivity

Example 26

Separation of various $REE^{3+}$ by liquid-liquid-extraction with *N*-2-ethyl-hexyl-*N'*-methylimidazolium-[di-2-ethyl-hexylamino]-(oxo)acetate in toluene

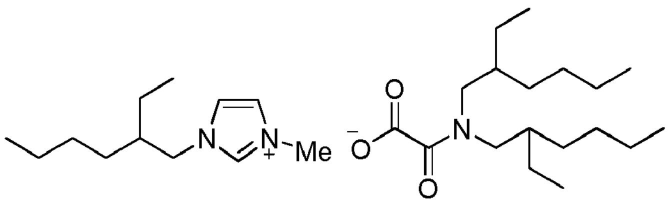

An aqueous stock solution containing various rare earth metal cations was mixed with a solution of *N*-2-ethyl-hexyl-*N'*-methylimidazolium-[di-2-ethyl-hexylamino]-(oxo)acetate in toluene and vigorously stirred at room temperature for 20 min. After this time the phases were separated and analyzed by ICP-MS. Depending on the pH-value of the aqueous phase , various separation factors were achieved.

| Organic phase (g) | Amount IL (mmol) | Aqueous phase (g) | pH-value | Amount REE3+ (mmol) | Ratio IL/ REE3+ |
|---|---|---|---|---|---|
| 2,00 | 0,09 | 2,02 | 1,29 | 0,02 | 0,32 |
| 2,01 | 0,09 | 1,98 | 1,2 | 0,02 | 0,33 |
| 2,00 | 0,09 | 1,96 | 1,09 | 0,02 | 0,32 |

Separation Factors for Example 26

Table I

| pH 1,29 | | | | | | | | | | | | | |
|---|---|---|---|---|---|---|---|---|---|---|---|---|---|
| RE | Ce | Pr | Nd | Sm | Eu | Gd | Tb | Dy | Ho | Er | Tm | Yb | Lu |
| La | 1,45 | 1,99 | 2,62 | 5,72 | 7,07 | 5,84 | 11,14 | 15,62 | 19,10 | 26,34 | 40,28 | 62,62 | 68,90 |
| Ce | | 1,37 | 1,81 | 3,95 | 4,88 | 4,04 | 7,70 | 10,80 | 13,20 | 18,21 | 27,84 | 43,28 | 47,62 |
| Pr | | | 1,32 | 2,87 | 3,55 | 2,94 | 5,60 | 7,85 | 9,60 | 13,24 | 20,25 | 31,48 | 34,63 |
| Nd | | | | 2,18 | 2,69 | 2,23 | 4,25 | 5,95 | 7,28 | 10,04 | 15,35 | 23,86 | 26,25 |

(continued)

| pH 1,29 | | | | | | | | | | | | | |
|---|---|---|---|---|---|---|---|---|---|---|---|---|---|
| RE | Ce | Pr | Nd | Sm | Eu | Gd | Tb | Dy | Ho | Er | Tm | Yb | Lu |
| Sm | | | | | 1,24 | 1,02 | 1,95 | 2,73 | 3,34 | 4,61 | 7,05 | 10,95 | 12,05 |
| Eu | | | | | | 0,83 | 1,58 | 2,21 | 2,70 | 3,73 | 5,70 | 8,86 | 9,75 |
| Gd | | | | | | | 1,91 | 2,67 | 3,27 | 4,51 | 6,89 | 10,72 | 11,79 |
| Tb | | | | | | | | 1,40 | 1,71 | 2,36 | 3,62 | 5,62 | 6,18 |
| Dy | | | | | | | | | 1,22 | 1,69 | 2,58 | 4,01 | 4,41 |
| Ho | | | | | | | | | | 1,38 | 2,11 | 3,28 | 3,61 |
| Er | | | | | | | | | | | 1,53 | 2,38 | 2,62 |
| Tm | | | | | | | | | | | | 1,55 | 1,71 |
| Yb | | | | | | | | | | | | | 1,10 |

Table II

| pH 1,20 | | | | | | | | | | | | | |
|---|---|---|---|---|---|---|---|---|---|---|---|---|---|
| RE | Ce | Pr | Nd | Sm | Eu | Gd | Tb | Dy | Ho | Er | Tm | Yb | Lu |
| La | 2,23 | 3,81 | 5,48 | 14,66 | 18,59 | 15,79 | 32,81 | 48,18 | 60,94 | 86,36 | 132,3 | 206,2 | 228,3 |
| Ce | | 1,71 | 2,46 | 6,59 | 8,35 | 7,10 | 14,75 | 21,65 | 27,39 | 38,81 | 59,49 | 92,71 | 102,6 |
| Pr | | | 1,44 | 3,84 | 4,87 | 4,14 | 8,60 | 12,63 | 15,98 | 22,64 | 34,70 | 54,08 | 59,86 |
| Nd | | | | 2,67 | 3,39 | 2,88 | 5,98 | 8,79 | 11,11 | 15,75 | 24,14 | 37,62 | 41,64 |
| Sm | | | | | 1,27 | 1,08 | 2,24 | 3,29 | 4,16 | 5,89 | 9,03 | 14,07 | 15,57 |
| Eu | | | | | | 0,85 | 1,76 | 2,59 | 3,28 | 4,65 | 7,12 | 11,10 | 12,28 |
| Gd | | | | | | | 2,08 | 3,05 | 3,86 | 5,47 | 8,38 | 13,06 | 14,46 |
| Tb | | | | | | | | 1,47 | 1,86 | 2,63 | 4,03 | 6,29 | 6,96 |
| Dy | | | | | | | | | 1,26 | 1,79 | 2,75 | 4,28 | 4,74 |
| Ho | | | | | | | | | | 1,42 | 2,17 | 3,39 | 3,75 |
| Er | | | | | | | | | | | 1,53 | 2,39 | 2,64 |
| Tm | | | | | | | | | | | | 1,56 | 1,73 |
| Yb | | | | | | | | | | | | | 1,11 |

Table III

| pH 1,09 | | | | | | | | | | | | | |
|---|---|---|---|---|---|---|---|---|---|---|---|---|---|
| RE | Ce | Pr | Nd | Sm | Eu | Gd | Tb | Dy | Ho | Er | Tm | Yb | Lu |
| La | 3,03 | 5,59 | 8,36 | 24,13 | 31,76 | 26,81 | 59,19 | 87,19 | 112,3 | 161,2 | 248,0 | 389,4 | 438,2 |
| Ce | | 1,85 | 2,76 | 7,97 | 10,49 | 8,86 | 19,56 | 28,81 | 37,13 | 53,26 | 81,95 | 128,6 | 144,7 |
| Pr | | | 1,50 | 4,32 | 5,68 | 4,80 | 10,59 | 15,60 | 20,11 | 28,84 | 44,37 | 69,67 | 78,39 |
| Nd | | | | 2,89 | 3,80 | 3,21 | 7,08 | 10,43 | 13,44 | 19,28 | 29,66 | 46,57 | 52,40 |
| Sm | | | | | 1,32 | 1,11 | 2,45 | 3,61 | 4,66 | 6,68 | 10,28 | 16,14 | 18,16 |
| Eu | | | | | | 0,84 | 1,86 | 2,75 | 3,54 | 5,08 | 7,81 | 12,26 | 13,80 |

(continued)

| pH 1,09 | | | | | | | | | | | | | |
|---|---|---|---|---|---|---|---|---|---|---|---|---|---|
| RE | Ce | Pr | Nd | Sm | Eu | Gd | Tb | Dy | Ho | Er | Tm | Yb | Lu |
| Gd | | | | | | | 2,21 | 3,25 | 4,19 | 6,01 | 9,25 | 14,53 | 16,34 |
| Tb | | | | | | | | 1,47 | 1,90 | 2,72 | 4,19 | 6,58 | 7,40 |
| Dy | | | | | | | | | 1,29 | 1,85 | 2,84 | 4,47 | 5,03 |
| Ho | | | | | | | | | | 1,43 | 2,21 | 3,47 | 3,90 |
| Er | | | | | | | | | | | 1,54 | 2,42 | 2,72 |
| Tm | | | | | | | | | | | | 1,57 | 1,77 |
| Yb | | | | | | | | | | | | | 1,13 |

## Claims

**1.** Monosubstituted oxalic acid derivatives of the general formula (I)

$[A]^+$ [O-C(O)-C(O)-X]- (I)

wherein the meaning is for

$[A]^+$ a cation made from an organic moiety A having a formally positively charged heteroatom selected from the group consisting of nitrogen, phosphorus and sulfur and
X is the group $-NR^aR^b$, wherein $R^a$ and $R^b$ are the same or different and are independently from each other hydrogen or a $C_1$ to $C_{30}$ organic residue with the proviso that at least one of $R^a$ and $R^b$ is a $C_1$ to $C_{30}$ organic residue.

**2.** Monosubstituted oxalic acid derivatives of the general formula (I) as claimed in claim 1, wherein $[A]^+$ is selected from amongst the compounds of the formulae (IV.e), (IVs), (IVv) and (IVy)

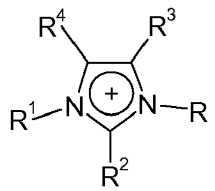

(IV.e)

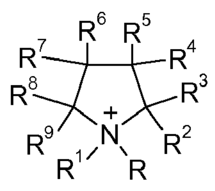

(IV.s)

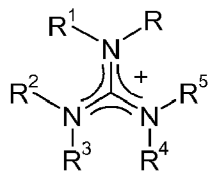

(IV.v)

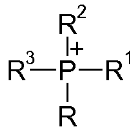

(IV.y)

wherein the substituents have the following meaning for the formulae:

R in any case is hydrogen or a a $C_1$ to $C_{30}$ organic residue with the proviso that at least one of R and $R^1$ is not hydrogen.
for imidazolium ions (IVe)
$R^1$ is hydrogen, methyl, ethyl, 1-propyl, 1-butyl, 1-pentyl, 1-hexyl, 2-ethylhexyl, 1-octyl, 1-nonyl, 1-decyl, 1-dodecyl, 2-hydroxyethyl or 2-cyanoethyl and $R^2$ to $R^4$ are each, independently of one another, hydrogen, methyl or ethyl;
for pyrrolidinium ions (IVs)
$R^1$ is hydrogen, methyl, ethyl, propyl, butyl or phenyl and $R^2$ to $R^9$ are each, independently of one another, hydrogen or methyl;
for guanidinium ions (IVv)
$R^1$ to $R^5$ are methyl, ethyl or butyl;
for phosphonium ions (IVy)
$R^1$ to $R^3$ are each, independently of one another aryl, $C_1$-$C_{18}$-alkyl.

3. Monosubstituted oxalic acid derivatives of the general formula (I) as claimed in claim 2, wherein $R^a$ and $R^b$ are independently from each other hydrogen, methyl, ethyl, 1-propyl, iso-propyl, 1-butyl, sec-butyl, tert-butyl, 1-pentyl, 1-hexyl, 2-ethylhexyl, 1-octyl, 1-nonyl, 1-decyl, 1-dodecyl, with the proviso that at most one of $R^a$ and $R^b$ is hydrogen.

4. Monosubstituted oxalic acid derivatives of the general formula (I) as claimed in claims 1 to 3, wherein each $R^a$ and $R^b$ in the group -$NR^aR^b$ of X in general formula (I) independently from each other are a $C_4$ to $C_{16}$ organic residue.

5. Monosubstituted oxalic acid derivatives of the general formula (I) as claimed in claim 2 to 4, wherein each $R^a$ and $R^b$ in the group -$NR^aR^b$ of X in general formula (I) is the residue 2-ethylhexyl.

6. Monosubstituted oxalic acid derivatives as defined in claim 1 to 5, wherein they are in the state of an ionic liquid.

7. Process for the preparation of monosubstituted oxalic acid derivatives as defined in claims 1 to 5 wherein the non-quaternized precursor of the organic moiety A as defined in claims 1 to 5 is reacted with an oxalic acid compound of the general formula (II)

$$R^1O\text{-}C(O)\text{-}C(O)\text{-}X \qquad (II)$$

wherein $R^1$ is a $C_1$ to $C_{30}$ organic residue and X is the group -$NR^aR^b$, wherein $R^a$ and $R^b$ are independently from each other hydrogen or a $C_1$ to $C_{30}$ organic residue with the proviso that at least one of $R^a$ and $R^b$ is a $C_1$ to $C_{30}$ organic residue.

8. Use of the monosubstituted oxalic acid derivatives as defined in claims 1 to 5 for at least partially dissolving an inorganic or an organic material.

**9.** Use as claimed in claim 8, wherein the inorganic or organic material is a metal compound.

**10.** Use as claimed in claim 9, wherein the metal is a rare earth element or an actinide element.

**11.** Use of the monosubstituted oxalic acid derivatives as defined in claims 1 to 5 in a separation process for metals.

**12.** Use as claimed in claim 11, wherein the metals are rare earth elements or actinide elements.

**13.** Use as claimed in claims 11 to 12 wherein the separation process is a liquid-liquid extraction.

Europäisches Patentamt
European Patent Office
Office européen des brevets

# EUROPEAN SEARCH REPORT

Application Number
EP 14 16 6399

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KO NAKAMURA ET AL: "Creatones A and B. revision of the structure for the product of oxidation of creatinine and creatine", BULLETIN OF THE CHEMICAL SOCIETY OF JAPAN, CHEMICAL SOCIETY OF JAPAN, TOKYO, JP, vol. 63, no. 5, 1 January 1990 (1990-01-01), pages 1540-1542, XP002715723, ISSN: 0009-2673<br>* page 1540; compound 4 *<br>* N-(N'-Methylamidino)oxamic Acid (4: Creatone B) *<br>----- | 1 | INV.<br>C07D233/58<br>C07D207/06<br>C07F9/54<br>C07C211/63 |
| X,D | ISABELLE BILLARD ET AL: "Liquidâ liquid extraction of actinides, lanthanides, and fission products by use of ionic liquids: from discovery to understanding", ANALYTICAL AND BIOANALYTICAL CHEMISTRY, SPRINGER, BERLIN, DE, vol. 400, no. 6, 4 January 2011 (2011-01-04), pages 1555-1566, XP019902260, ISSN: 1618-2650, DOI: 10.1007/S00216-010-4478-X | 1,2,6,13 | |
| A | * abstract *<br>* page 1556, left-hand column - page 1558, right-hand column, paragraph 1 *<br>----- | 3 | **TECHNICAL FIELDS SEARCHED (IPC)**<br>C07D<br>C07F<br>C07C |
| X | PETYUNIN G P ET AL: "PREPARATION OF AMINOACRIDINE SALTS", PHARMACEUTICAL CHEMISTRY JOURNAL, SPRINGER NEW YORK LLC, US, vol. 9, no. 7, 1 July 1975 (1975-07-01), pages 444-447, XP009037755, ISSN: 0091-150X, DOI: 10.1007/BF00759350<br>* page 446; table 2 *<br>* page 445, lines 1-9 *<br>-----<br>-/-- | 1,7 | |

The present search report has been drawn up for all claims

1

| Place of search | Date of completion of the search | Examiner |
|---|---|---|
| Munich | 30 September 2014 | Goss, Ilaria |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 14 16 6399

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2014/056844 A1 (BASF SE [DE]) 17 April 2014 (2014-04-17) * the whole document * ----- | 1-13 | |
| X | US 2014/099249 A1 (MASSONNE KLEMENS [DE] ET AL) 10 April 2014 (2014-04-10) * the whole document * ----- | 1-13 | |

TECHNICAL FIELDS SEARCHED (IPC)

The present search report has been drawn up for all claims

1

| Place of search | Date of completion of the search | Examiner |
|---|---|---|
| Munich | 30 September 2014 | Goss, Ilaria |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 14 16 6399

This annex lists the patent family members relating to the patent documents cited in the above-mentioned European search report. The members are as contained in the European Patent Office EDP file on
The European Patent Office is in no way liable for these particulars which are merely given for the purpose of information.

30-09-2014

| Patent document cited in search report | Publication date | Patent family member(s) | Publication date |
|---|---|---|---|
| WO 2014056844 A1 | 17-04-2014 | NONE | |
| US 2014099249 A1 | 10-04-2014 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **I BILLARD et al.** *Radiochim Acta,* 2009, 355-359
- **I. BILLARD et al.** *Anal Bioanal Chem.,* 2011, 1555-1566
- **X. SUN et al.** *Talanta,* 2012, 132-137